# EUROPEAN PATENT APPLICATION

(11) **EP 3 842 449 A1**
(43) Date of publication of application: **30.06.2021**
(21) Application number: 19425097.3
(22) Date of filing: 23.12.2019
(51) Int. Cl.: C07K 14/605, A61P 3/10, A61K 38/00

(54) **STAPLED OLEFIN CO-AGONISTS OF THE GLUCAGON AND GLP-1 RECEPTORS**

(71) Applicant: MERCK SHARP & DOHME CORP., Rahway, NJ 07065-0907 (US)
(72) Inventor: Bianchi, Elisabetta, 00195 Roma (IT); Deng, Qiaolin, Kenilworth, NJ 07033 (US); Lin, Songnian, Kenilworth, NJ 07033 (US); Orvieto, Federica, 00128 Roma (IT); Palani, Anandan, Boston, MA 02115-5727 (US); Pessi, Antonello, 00141 Roma (IT); Sawyer, Tomi K, Southborough, MA 01722 (US)
(74) Representative: Di Giovine, Paolo

(57) **Abstract**

The stapled peptides of the present invention, and pharmaceutically acceptable salts thereof, are co-agonists of the glucagon and GLP-1 receptors, and may be useful in the treatment, prevention and suppression of diseases mediated by the glucagon receptor and the GLP-1 receptor, including but not limited to, metabolic disorders such as diabetes, non-alcoholic fatty liver disease (NAFLD), non-alcoholic steatohepatitis (NASH), and obesity.

SEQUENCE LISTING

<110>  Bianchi, Elisabetta
       Deng, Qiaolin
       Lin, Songnian
       Orvieto, Frederica
       Palani, Anandan
       Pessi, Antonello
       Sawyer, Tomi

<120>  STAPLED OLEFIN CO-AGONISTS OF THE GLUCAGON AND GLP-1 RECEPTORS

<130>  24934-IT-NP

<160>  20

<170>  PatentIn version 3.5

<210>  1
<211>  29
<212>  PRT
<213>  Homo sapiens

<400>  1

His Ser Gln Gly Thr Phe Thr Ser Asp Tyr Ser Lys Tyr Leu Asp Ser
1               5                   10                  15

Arg Arg Ala Gln Asp Phe Val Gln Trp Leu Met Asn Thr
            20                  25

<210>  2
<211>  30
<212>  PRT
<213>  Homo sapiens

<400>  2

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg
            20                  25                  30

<210>  3
<211>  37
<212>  PRT
<213>  Homo sapiens

<400>  3

His Ser Gln Gly Thr Phe Thr Ser Asp Tyr Ser Lys Tyr Leu Asp Ser
1               5                   10                  15

Arg Arg Ala Gln Asp Phe Val Gln Trp Leu Met Asn Thr Lys Arg Asn
            20                  25                  30

Arg Asn Asn Ile Ala
        35

<210>  4
<211>  39
<212>  PRT
<213>  Heloderma suspectum

<400>  4

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35

<210>  5
<211>  33
<212>  PRT
<213>  Homo sapiens

<400>  5

His Ala Asp Gly Ser Phe Ser Asp Glu Met Asn Thr Ile Leu Asp Asn
1               5                   10                  15

Leu Ala Ala Arg Asp Phe Ile Asn Trp Leu Ile Gln Thr Lys Ile Thr
            20                  25                  30

Asp

<210>  6
<211>  31
<212>  PRT
<213>  Artificial Sequence

<220>
<223>  Peptide

<220>
<221>  MISC_FEATURE
<222>  (1)..(20)
<223>  Miscellaneous feature: the alkene chain of the amino acid at
       position 12 forms a double bond containing ring with the alkene
       chain of the amino acid at position 19

<220>
<221>  MISC_FEATURE
<222>  (2)..(2)
<223>  Xaa is D-Serine

<220>
<221>  MISC_FEATURE
<222>  (12)..(12)
<223>  Xaa is (R)-2-amino-2-methyloct-7-enoic acid

<220>
<221>  MISC_FEATURE
<222>  (19)..(19)
<223>  Xaa is (S)-2-amino-2-methylnon-8-enoic acid

<220>
<221>  MISC_FEATURE
<222>  (29)..(29)
<223>  Xaa is C terminal amide

<400>  6

His Xaa Gln Gly Thr Phe Thr Ser Asp Lys Ser Xaa Tyr Leu Asp Glu
1               5                   10                  15

Arg Ala Xaa Gln Asp Phe Val Gln Trp Leu Leu Asp Thr Asn His
            20                  25                  30

<210>  7
<211>  31
<212>  PRT
<213>  Artificial Sequence

<220>
<223>  Peptide

<220>
<221>  MISC_FEATURE
<222>  (1)..(20)
<223>  Miscellaneous feature: the alkene chain of the amino acid at
       position 12 forms a double bond containing ring with the alkene
       chain of the amino acid at position 19

<220>
<221>  MISC_FEATURE
<222>  (2)..(2)
<223>  Xaa is D-Serine

<220>
<221>  MISC_FEATURE
<222>  (10)..(10)
<223>  Xaa is Lysine conjugated to gamma-Glu-gamma-Glu- C16 fatty acid

<220>
<221>  MISC_FEATURE
<222>  (12)..(12)
<223>  Xaa is (R)-2-amino-2-methyloct-7-enoic acid

<220>
<221>  MISC_FEATURE
<222>  (19)..(19)
<223>  Xaa is (S)-2-amino-2-methylnon-8-enoic acid

<220>
<221>  MISC_FEATURE

<222>  (29)..(29)
<223>  Xaa is C terminal amide

<400>  7

His Xaa Gln Gly Thr Phe Thr Ser Asp Xaa Ser Xaa Tyr Leu Asp Glu
1               5                   10                  15

Arg Ala Xaa Gln Asp Phe Val Gln Trp Leu Leu Asp Thr Asn His
            20                  25                  30

<210>  8
<211>  31
<212>  PRT
<213>  Artificial Sequence

<220>
<223>  Peptide

<220>
<221>  MISC_FEATURE
<222>  (1)..(20)
<223>  Miscellaneous feature: the alkene chain of the amino acid at
       position 17 forms a double bond containing ring with the alkene
       chain of the amino acid at position 24

<220>
<221>  MISC_FEATURE
<222>  (2)..(2)
<223>  Xaa is D-Serine

<220>
<221>  MISC_FEATURE
<222>  (10)..(10)
<223>  Xaa is Lys conjugated to gamma-Glu-gamma-Glu- C16 fatty acid

<220>
<221>  MISC_FEATURE
<222>  (17)..(17)
<223>  Xaa is (R)-2-amino-2-methyloct-7-enoic acid

<220>
<221>  MISC_FEATURE
<222>  (24)..(24)
<223>  Xaa is (S)-2-amino-2-methylnon-8-enoic acid

<220>
<221>  MISC_FEATURE
<222>  (29)..(29)
<223>  Xaa is C terminal amide

<400>  8

His Xaa Gln Gly Thr Phe Thr Ser Asp Xaa Ser Lys Tyr Leu Asp Glu
1               5                   10                  15

Xaa Ala Ala Gln Asp Phe Val Xaa Trp Leu Leu Asp Thr Asn His
            20                  25                  30

<210>  9
<211>  31
<212>  PRT
<213>  Artificial Sequence

<220>
<223>  Peptide



<220>
<221>  MISC_FEATURE
<222>  (1)..(20)
<223>  Miscellaneous feature: the alkene chain of the amino acid at
       position 17 forms a double bond containing ring with the alkene
       chain of the amino acid at position 24

<220>
<221>  MISC_FEATURE
<222>  (2)..(2)
<223>  Xaa is D-Serine

<220>
<221>  MISC_FEATURE
<222>  (17)..(17)
<223>  Xaa is (R)-2-amino-2-methyloct-7-enoic acid

<220>
<221>  MISC_FEATURE
<222>  (24)..(24)
<223>  Xaa is (S)-2-amino-2-methylnon-8-enoic acid

<220>
<221>  MISC_FEATURE
<222>  (29)..(29)
<223>  Xaa is C terminal amide

<400>  9

His Xaa Gln Gly Thr Phe Thr Ser Asp Lys Ser Lys Tyr Leu Asp Glu
1               5                   10                  15


Xaa Ala Ala Gln Asp Phe Val Xaa Trp Leu Leu Asp Thr Asn His
            20                  25                  30


<210>  10
<211>  31
<212>  PRT
<213>  Artificial Sequence

<220>
<223>  Peptide


<220>
<221>  MISC_FEATURE
<222>  (1)..(20)
<223>  Miscellaneous feature: the alkene chain of the amino acid at
       position 20 forms a double bond containing ring with the alkene
       chain of the amino acid at position 27

<220>
<221>  MISC_FEATURE
<222>  (2)..(2)
<223>  Xaa is D-Serine

<220>
<221>  MISC_FEATURE
<222>  (10)..(10)
<223>  Xaa is Lysine conjugated to gamma-Glu-gamma-Glu- C16 fatty acid

<220>
<221>  MISC_FEATURE
<222>  (20)..(20)
<223>  Xaa is (R)-2-amino-2-methyloct-7-enoic acid

<220>
<221>  MISC_FEATURE
<222>  (27)..(27)
<223>  Xaa is (S)-2-amino-2-methylnon-8-enoic acid

<220>
<221>  MISC_FEATURE
<222>  (29)..(29)
<223>  Xaa is C terminal amide

<400>  10

His Xaa Gln Gly Thr Phe Thr Ser Asp Xaa Ser Lys Tyr Leu Asp Glu
1               5                   10                  15

Arg Ala Ala Xaa Asp Phe Val Gln Trp Leu Xaa Asp Thr Asn His
            20                  25                  30

<210>  11
<211>  31
<212>  PRT
<213>  Artificial Sequence

<220>
<223>  Peptide

<220>
<221>  MISC_FEATURE
<222>  (1)..(20)
<223>  Miscellaneous feature :the alkene chain of the amino acid at
       position 20 forms a double bond containing ring with the alkene
       chain of the amino acid at position 27

<220>
<221>  MISC_FEATURE
<222>  (2)..(2)
<223>  Xaa is D-Serine

<220>
<221>  MISC_FEATURE
<222>  (20)..(20)
<223>  Xaa is (R)-2-amino-2-methyloct-7-enoic acid

<220>
<221>  MISC_FEATURE

<222>  (27)..(27)
<223>  Xaa is (S)-2-amino-2-methylnon-8-enoic acid

<220>
<221>  MISC_FEATURE
<222>  (29)..(29)
<223>  Xaa is C terminal amide

<400>  11

His Xaa Gln Gly Thr Phe Thr Ser Asp Lys Ser Lys Tyr Leu Asp Glu
1               5                   10                  15

Arg Ala Ala Xaa Asp Phe Val Gln Trp Leu Xaa Asp Thr Asn His
            20                  25                  30

<210>  12
<211>  31
<212>  PRT
<213>  Artificial Sequence

<220>
<223>  Peptide

<220>
<221>  MISC_FEATURE
<222>  (1)..(20)
<223>  Miscellaneous feature: the alkene chain of the amino acid at
       position 20 forms a double bond containing ring with the alkene
       chain of the amino acid at position 24

<220>
<221>  MISC_FEATURE
<222>  (2)..(2)
<223>  Xaa is D-Serine

<220>
<221>  MISC_FEATURE
<222>  (10)..(10)
<223>  Xaa is Lysine conjugated to gamma-Glu-gamma-Glu- C16 fatty acid

<220>
<221>  MISC_FEATURE
<222>  (20)..(20)
<223>  Xaa is (S)-2-amino-2-methylhept-6-enoic acid

<220>
<221>  MISC_FEATURE
<222>  (24)..(24)
<223>  Xaa is (S)-2-amino-2-methylhept-6-enoic acid

<220>
<221>  MISC_FEATURE
<222>  (29)..(29)
<223>  Xaa is C terminal amide

<400>  12

His Xaa Gln Gly Thr Phe Thr Ser Asp Xaa Ser Lys Tyr Leu Asp Glu
1               5                   10                  15

Arg Ala Ala Xaa Asp Phe Val Xaa Trp Leu Leu Asp Thr Asn His
            20                  25                  30

<210>  13
<211>  32
<212>  PRT
<213>  Artificial Sequence

<220>
<223>  Peptide

<220>
<221>  MISC_FEATURE
<222>  (1)..(29)
<223>  Miscellaneous feature: the alkene chain of the amino acid at
       position 21 forms a double bond containing ring with the alkene
       chain of the amino acid at position 28

<220>
<221>  MISC_FEATURE
<222>  (2)..(2)
<223>  Xaa is alpha aminoisobutyric acid

<220>
<221>  MISC_FEATURE
<222>  (10)..(10)
<223>  Xaa is Lysine conjugated to gamma-Glu-gamma-Glu- C16 fatty acid

<220>
<221>  MISC_FEATURE
<222>  (16)..(16)
<223>  Xaa is alpha aminoisobutyric acid

<220>
<221>  MISC_FEATURE
<222>  (21)..(21)
<223>  Xaa is (R)-2-amino-2-methyloct-7-enoic acid

<220>
<221>  MISC_FEATURE
<222>  (28)..(28)
<223>  Xaa is (S)-2-amino-2-methylnon-8-enoic acid

<220>
<221>  MISC_FEATURE
<222>  (29)..(29)
<223>  Xaa is C terminal amide

<220>
<221>  MISC_FEATURE
<222>  (30)..(30)
<223>  Xaa is Lysine conjugated to gamma-Glutamic acid

<400>  13

His Xaa Gln Gly Thr Phe Thr Ser Asp Xaa Ser Lys Tyr Leu Asp Xaa
1               5                   10                  15

Arg Ala Ala Gln Xaa Phe Val Gln Trp Leu Met Xaa Thr Xaa Asn His
            20                  25                  30

<210>  14
<211>  32
<212>  PRT
<213>  Artificial Sequence

<220>
<223>  Peptide

<220>
<221>  MISC_FEATURE
<222>  (1)..(20)
<223>  Miscellaneous feature: the alkene chain of the amino acid at
       position 24 forms a double bond containing ring with the alkene
       chain of the amino acid at position 28

<220>
<221>  MISC_FEATURE
<222>  (2)..(2)
<223>  Xaa is alpha aminoisobutyric acid

<220>
<221>  MISC_FEATURE
<222>  (10)..(10)
<223>  Xaa is Lysine conjugated to gamma-Glu-gamma-Glu- C16 fatty acid

<220>
<221>  MISC_FEATURE
<222>  (16)..(16)
<223>  Xaa is alpha aminoisobutyric acid

<220>
<221>  MISC_FEATURE
<222>  (24)..(24)
<223>  Xaa is (S)-2-amino-2-methylhept-6-enoic acid

<220>
<221>  MISC_FEATURE
<222>  (28)..(28)
<223>  Xaa is (S)-2-amino-2-methylhept-6-enoic acid

<220>
<221>  MISC_FEATURE
<222>  (29)..(29)
<223>  Xaa is C terminal amide

<220>
<221>  MISC_FEATURE
<222>  (30)..(30)
<223>  Xaa is Lysine conjugated to gamma-Glutamic acid

<400>  14

His Xaa Gln Gly Thr Phe Thr Ser Asp Xaa Ser Lys Tyr Leu Asp Xaa
1               5                   10                  15

Arg Ala Ala Gln Asp Phe Val Xaa Trp Leu Met Xaa Thr Xaa Asn His
            20                  25                  30

<210>  15
<211>  31
<212>  PRT
<213>  Artificial Sequence

<220>
<223>  Peptide



<220>
<221>  MISC_FEATURE
<222>  (1)..(20)
<223>  Miscellaneous feature: the alkene chain of the amino acid at
       position 20 forms a double bond containing ring with the alkene
       chain of the amino acid at position 24

<220>
<221>  MISC_FEATURE
<222>  (2)..(2)
<223>  Xaa is D-Serine

<220>
<221>  MISC_FEATURE
<222>  (10)..(10)
<223>  Xaa is Lys conjugated to gamma-Glu-gamma-Glu- C16 fatty acid

<220>
<221>  MISC_FEATURE
<222>  (20)..(20)
<223>  Xaa is (S)-2-aminohept-6-enoic acid

<220>
<221>  MISC_FEATURE
<222>  (24)..(24)
<223>  Xaa is (S)-2-aminohept-6-enoic acid

<220>
<221>  MISC_FEATURE
<222>  (29)..(29)
<223>  Xaa is C terminal amide

<400>  15

His Xaa Gln Gly Thr Phe Thr Ser Asp Xaa Ser Lys Tyr Leu Asp Glu
1               5                   10                  15


Arg Ala Ala Xaa Asp Phe Val Xaa Trp Leu Leu Asp Thr Asn His
            20                  25                  30


<210>  16
<211>  32
<212>  PRT
<213>  Artificial Sequence

<220>
<223>  Peptide


<220>

<221>  MISC_FEATURE
<222>  (1)..(20)
<223>  Miscellaneous feature :the alkene chain of the amino acid at
       position 24 forms a double bond containing ring with the alkene
       chain of the amino acid at position 28

<220>
<221>  MISC_FEATURE
<222>  (2)..(2)
<223>  Xaa is alpha aminoisobutyric acid

<220>
<221>  MISC_FEATURE
<222>  (10)..(10)
<223>  Xaa is Lysine conjugated to gamma-Glu-gamma-Glu- C16 fatty acid

<220>
<221>  MISC_FEATURE
<222>  (16)..(16)
<223>  Xaa is alpha aminoisobutyric acid

<220>
<221>  MISC_FEATURE
<222>  (24)..(24)
<223>  Xaa is (S)-2-aminohept-6-enoic acid

<220>
<221>  MISC_FEATURE
<222>  (28)..(28)
<223>  Xaa is (S)-2-aminohept-6-enoic acid

<220>
<221>  MISC_FEATURE
<222>  (29)..(29)
<223>  Xaa is C terminal amide

<220>
<221>  MISC_FEATURE
<222>  (30)..(30)
<223>  Xaa is Lysine conjugated to gamma-Glutamic acid

<400>  16

His Xaa Gln Gly Thr Phe Thr Ser Asp Xaa Ser Lys Tyr Leu Asp Xaa
1               5                   10                  15


Arg Ala Ala Gln Asp Phe Val Xaa Trp Leu Leu Xaa Thr Xaa Asn His
            20                  25                  30


<210>  17
<211>  31
<212>  PRT
<213>  Artificial Sequence

<220>
<223>  Peptide


<220>
<221>  MISC_FEATURE
<222>  (1)..(20)

<223>  Miscellaneous feature: the alkene chain of the amino acid at
       position 20 forms a double bond containing ring with the alkene
       chain of the amino acid at position 24

<220>
<221>  MISC_FEATURE
<222>  (2)..(2)
<223>  Xaa is D-Serine

<220>
<221>  MISC_FEATURE
<222>  (20)..(20)
<223>  Xaa is (S)-2-aminohept-6-enoic acid

<220>
<221>  MISC_FEATURE
<222>  (24)..(24)
<223>  Xaa is (S)-2-aminohept-6-enoic acid

<220>
<221>  MISC_FEATURE
<222>  (29)..(29)
<223>  Xaa is C terminal amide

<400>  17

His Xaa Gln Gly Thr Phe Thr Ser Asp Lys Ser Lys Tyr Leu Asp Glu
1               5                   10                  15


Arg Ala Ala Xaa Asp Phe Val Xaa Trp Leu Leu Asp Thr Asn His
            20                  25                  30


<210>  18
<211>  32
<212>  PRT
<213>  Artificial Sequence

<220>
<223>  Peptide


<220>
<221>  MISC_FEATURE
<222>  (1)..(20)
<223>  Miscellaneous feature: the alkene chain of the amino acid at
       position 24 forms a double bond containing ring with the alkene
       chain of the amino acid at position 28

<220>
<221>  MISC_FEATURE
<222>  (2)..(2)
<223>  Xaa is alpha aminoisobutyric acid

<220>
<221>  MISC_FEATURE
<222>  (16)..(16)
<223>  Xaa is alpha aminoisobutyric acid

<220>
<221>  MISC_FEATURE
<222>  (24)..(24)

<223>  Xaa is (S)-2-aminohept-6-enoic acid

<220>
<221>  MISC_FEATURE
<222>  (28)..(28)
<223>  Xaa is (S)-2-aminohept-6-enoic acid

<220>
<221>  MISC_FEATURE
<222>  (29)..(29)
<223>  Xaa is C terminal amide

<220>
<221>  MISC_FEATURE
<222>  (30)..(30)
<223>  Xaa is Lysine conjugated to gamma-Glutamic acid

<400>  18

His Xaa Gln Gly Thr Phe Thr Ser Asp Lys Ser Lys Tyr Leu Asp Xaa
1               5                   10                  15

Arg Ala Ala Gln Asp Phe Val Xaa Trp Leu Leu Xaa Thr Xaa Asn His
            20                  25                  30

<210>  19
<211>  31
<212>  PRT
<213>  Artificial Sequence

<220>
<223>  Peptide

<220>
<221>  MISC_FEATURE
<222>  (1)..(20)
<223>  Miscellaneous feature: the alkene chain of the amino acid at
       position 20 forms a double bond containing ring with the alkene
       chain of the amino acid at position 24

<220>
<221>  MISC_FEATURE
<222>  (2)..(2)
<223>  Xaa is D-Serine

<220>
<221>  MISC_FEATURE
<222>  (20)..(20)
<223>  Xaa is (S)-2-amino-2-methylhept-6-enoic acid

<220>
<221>  MISC_FEATURE
<222>  (24)..(24)
<223>  Xaa is (S)-2-amino-2-methylhept-6-enoic acid

<220>
<221>  MISC_FEATURE
<222>  (29)..(29)
<223>  Xaa is  C terminal amide

<400>  19

His Xaa Gln Gly Thr Phe Thr Ser Asp Lys Ser Lys Tyr Leu Asp Glu
1               5                   10                  15

Arg Ala Ala Xaa Asp Phe Val Xaa Trp Leu Leu Asp Thr Asn His
            20                  25                  30

<210>  20
<211>  32
<212>  PRT
<213>  Artificial Sequence

<220>
<223>  Peptide

<220>
<221>  MISC_FEATURE
<222>  (1)..(20)
<223>  Miscellaneous feature:  wherein the alkene chain of
       (R)-2-amino-2-methyloct-7-enoic acid forms a double bond
       containing ring with the alkene chain of
       (S)-2-amino-2-methylnon-8-enoic acid; or the alkene chains of

<220>
<221>  MISC_FEATURE
<222>  (2)..(2)
<223>  Xaa is alpha-aminoisobutyric acid or D-Serine

<220>
<221>  MISC_FEATURE
<222>  (10)..(10)
<223>  Xaa is Lysine, Lysine conjugated to a fatty acid, or Lysine
       conjugated to a fatty diacid

<220>
<221>  MISC_FEATURE
<222>  (12)..(12)
<223>  Xaa is (R)-2-amino-2-methyloct-7-enoic acid, or Lysine

<220>
<221>  MISC_FEATURE
<222>  (16)..(16)
<223>  Xaa is  alpha-aminoisobutyric acid, or Glutamic acid

<220>
<221>  MISC_FEATURE
<222>  (17)..(17)
<223>  Xaa is (R)-2-amino-2-methyloct-7-enoic acid, or Arginine

<220>
<221>  MISC_FEATURE
<222>  (19)..(19)
<223>  Xaa is (S)-2-amino-2-methylnon-8-enoic acid, or Alanine

<220>
<221>  MISC_FEATURE
<222>  (20)..(20)
<223>  Xaa is Glutamine, (R)-2-amino-2-methyloct-7-enoic acid,

(S)-2-amino-2-methylhept-6-enoic acid, or (S)-2-aminohept-6-enoic
acid

<220>
<221>  MISC_FEATURE
<222>  (21)..(21)
<223>  Xaa is Aspartic acid or (R)-2-amino-2-methyloct-7-enoic acid

<220>
<221>  MISC_FEATURE
<222>  (24)..(24)
<223>  Xaa is  Glutamine, (S)-2-amino-2-methylnon-8-enoic acid,
(S)-2-amino-2-methylhept-6-enoic acid, or (S)-2-aminohept-6-enoic
acid

<220>
<221>  MISC_FEATURE
<222>  (27)..(27)
<223>  Xaa is Leucine, (S)-2-amino-2-methylnon-8-enoic acid, or
Methionine

<220>
<221>  MISC_FEATURE
<222>  (28)..(28)
<223>  Xaa is Aspartic acid, (S)-2-amino-2-methylnon-8-enoic acid,
(S)-2-amino-2-methylhept-6-enoic acid, or (S)-2-aminohept-6-enoic
acid

<220>
<221>  MISC_FEATURE
<222>  (29)..(29)
<223>  Xaa is C terminal amide

<220>
<221>  MISC_FEATURE
<222>  (30)..(30)
<223>  Xaa is absent, or Lysine conjugated by a gamma-glutamic acid
spacer

<400>  20

His Xaa Gln Gly Thr Phe Thr Ser Asp Xaa Ser Xaa Tyr Leu Asp Xaa
1               5                   10                  15

Xaa Ala Xaa Xaa Xaa Phe Val Xaa Trp Leu Xaa Xaa Thr Xaa Asn His
            20                  25                  30

SEQUENCE LISTING

<110>  Bianchi, Elisabetta
       Deng, Qiaolin
       Lin, Songnian
       Orvieto, Frederica
       Palani, Anandan
       Pessi, Antonello
       Sawyer, Tomi

<120>  STAPLED OLEFIN CO-AGONISTS OF THE GLUCAGON AND GLP-1 RECEPTORS

<130>  24934-IT-NP

<160>  20

<170>  PatentIn version 3.5

<210>  1
<211>  29
<212>  PRT
<213>  Homo sapiens

<400>  1

His Ser Gln Gly Thr Phe Thr Ser Asp Tyr Ser Lys Tyr Leu Asp Ser
1               5                   10                  15

Arg Arg Ala Gln Asp Phe Val Gln Trp Leu Met Asn Thr
            20                  25

<210>  2
<211>  30
<212>  PRT
<213>  Homo sapiens

<400>  2

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg
            20                  25                  30

<210>  3
<211>  37
<212>  PRT
<213>  Homo sapiens

<400>  3

His Ser Gln Gly Thr Phe Thr Ser Asp Tyr Ser Lys Tyr Leu Asp Ser
1               5                   10                  15

Arg Arg Ala Gln Asp Phe Val Gln Trp Leu Met Asn Thr Lys Arg Asn
            20                  25                  30

Arg Asn Asn Ile Ala
        35

<210>  4
<211>  39
<212>  PRT
<213>  Heloderma suspectum

<400>  4

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Pro Ser
        35

<210>  5
<211>  33
<212>  PRT
<213>  Homo sapiens

<400>  5

His Ala Asp Gly Ser Phe Ser Asp Glu Met Asn Thr Ile Leu Asp Asn
1               5                   10                  15

Leu Ala Ala Arg Asp Phe Ile Asn Trp Leu Ile Gln Thr Lys Ile Thr
            20                  25                  30

Asp

<210>  6
<211>  31
<212>  PRT
<213>  Artificial Sequence

<220>
<223>  Peptide

<220>
<221>  MISC_FEATURE
<222>  (1)..(20)
<223>  Miscellaneous feature: the alkene chain of the amino acid at
       position 12 forms a double bond containing ring with the alkene
       chain of the amino acid at position 19

<220>
<221>  MISC_FEATURE
<222>  (2)..(2)
<223>  Xaa is D-Serine

<220>
<221>  MISC_FEATURE
<222>  (12)..(12)
<223>  Xaa is (R)-2-amino-2-methyloct-7-enoic acid

<220>
<221>  MISC_FEATURE
<222>  (19)..(19)
<223>  Xaa is (S)-2-amino-2-methylnon-8-enoic acid

<220>
<221>  MISC_FEATURE
<222>  (29)..(29)
<223>  Xaa is C terminal amide

<400>  6

His Xaa Gln Gly Thr Phe Thr Ser Asp Lys Ser Xaa Tyr Leu Asp Glu
1               5                   10                  15

Arg Ala Xaa Gln Asp Phe Val Gln Trp Leu Leu Asp Thr Asn His
            20                  25                  30

<210>  7
<211>  31
<212>  PRT
<213>  Artificial Sequence

<220>
<223>  Peptide

<220>
<221>  MISC_FEATURE
<222>  (1)..(20)
<223>  Miscellaneous feature: the alkene chain of the amino acid at
       position 12 forms a double bond containing ring with the alkene
       chain of the amino acid at position 19

<220>
<221>  MISC_FEATURE
<222>  (2)..(2)
<223>  Xaa is D-Serine

<220>
<221>  MISC_FEATURE
<222>  (10)..(10)
<223>  Xaa is Lysine conjugated to gamma-Glu-gamma-Glu- C16 fatty acid

<220>
<221>  MISC_FEATURE
<222>  (12)..(12)
<223>  Xaa is (R)-2-amino-2-methyloct-7-enoic acid

<220>
<221>  MISC_FEATURE
<222>  (19)..(19)
<223>  Xaa is (S)-2-amino-2-methylnon-8-enoic acid

<220>
<221>  MISC_FEATURE

<222>  (29)..(29)
<223>  Xaa is C terminal amide

<400>  7

His Xaa Gln Gly Thr Phe Thr Ser Asp Xaa Ser Xaa Tyr Leu Asp Glu
1               5                   10                  15

Arg Ala Xaa Gln Asp Phe Val Gln Trp Leu Leu Asp Thr Asn His
            20                  25                  30

<210>  8
<211>  31
<212>  PRT
<213>  Artificial Sequence

<220>
<223>  Peptide

<220>
<221>  MISC_FEATURE
<222>  (1)..(20)
<223>  Miscellaneous feature: the alkene chain of the amino acid at
       position 17 forms a double bond containing ring with the alkene
       chain of the amino acid at position 24

<220>
<221>  MISC_FEATURE
<222>  (2)..(2)
<223>  Xaa is D-Serine

<220>
<221>  MISC_FEATURE
<222>  (10)..(10)
<223>  Xaa is Lys conjugated to gamma-Glu-gamma-Glu- C16 fatty acid

<220>
<221>  MISC_FEATURE
<222>  (17)..(17)
<223>  Xaa is (R)-2-amino-2-methyloct-7-enoic acid

<220>
<221>  MISC_FEATURE
<222>  (24)..(24)
<223>  Xaa is (S)-2-amino-2-methylnon-8-enoic acid

<220>
<221>  MISC_FEATURE
<222>  (29)..(29)
<223>  Xaa is C terminal amide

<400>  8

His Xaa Gln Gly Thr Phe Thr Ser Asp Xaa Ser Lys Tyr Leu Asp Glu
1               5                   10                  15

Xaa Ala Ala Gln Asp Phe Val Xaa Trp Leu Leu Asp Thr Asn His
            20                  25                  30

<210>  9
<211>  31
<212>  PRT
<213>  Artificial Sequence

<220>
<223>  Peptide



<220>
<221>  MISC_FEATURE
<222>  (1)..(20)
<223>  Miscellaneous feature: the alkene chain of the amino acid at
       position 17 forms a double bond containing ring with the alkene
       chain of the amino acid at position 24

<220>
<221>  MISC_FEATURE
<222>  (2)..(2)
<223>  Xaa is D-Serine

<220>
<221>  MISC_FEATURE
<222>  (17)..(17)
<223>  Xaa is (R)-2-amino-2-methyloct-7-enoic acid

<220>
<221>  MISC_FEATURE
<222>  (24)..(24)
<223>  Xaa is (S)-2-amino-2-methylnon-8-enoic acid

<220>
<221>  MISC_FEATURE
<222>  (29)..(29)
<223>  Xaa is C terminal amide

<400>  9

His Xaa Gln Gly Thr Phe Thr Ser Asp Lys Ser Lys Tyr Leu Asp Glu
1               5                   10                  15


Xaa Ala Ala Gln Asp Phe Val Xaa Trp Leu Leu Asp Thr Asn His
            20                  25                  30


<210>  10
<211>  31
<212>  PRT
<213>  Artificial Sequence

<220>
<223>  Peptide


<220>
<221>  MISC_FEATURE
<222>  (1)..(20)
<223>  Miscellaneous feature: the alkene chain of the amino acid at
       position 20 forms a double bond containing ring with the alkene
       chain of the amino acid at position 27

<220>
<221>  MISC_FEATURE
<222>  (2)..(2)
<223>  Xaa is D-Serine

<220>
<221>  MISC_FEATURE
<222>  (10)..(10)
<223>  Xaa is Lysine conjugated to gamma-Glu-gamma-Glu- C16 fatty acid

<220>
<221>  MISC_FEATURE
<222>  (20)..(20)
<223>  Xaa is (R)-2-amino-2-methyloct-7-enoic acid

<220>
<221>  MISC_FEATURE
<222>  (27)..(27)
<223>  Xaa is (S)-2-amino-2-methylnon-8-enoic acid

<220>
<221>  MISC_FEATURE
<222>  (29)..(29)
<223>  Xaa is C terminal amide

<400>  10

His Xaa Gln Gly Thr Phe Thr Ser Asp Xaa Ser Lys Tyr Leu Asp Glu
1               5                   10                  15

Arg Ala Ala Xaa Asp Phe Val Gln Trp Leu Xaa Asp Thr Asn His
            20                  25                  30

<210>  11
<211>  31
<212>  PRT
<213>  Artificial Sequence

<220>
<223>  Peptide

<220>
<221>  MISC_FEATURE
<222>  (1)..(20)
<223>  Miscellaneous feature :the alkene chain of the amino acid at
       position 20 forms a double bond containing ring with the alkene
       chain of the amino acid at position 27

<220>
<221>  MISC_FEATURE
<222>  (2)..(2)
<223>  Xaa is D-Serine

<220>
<221>  MISC_FEATURE
<222>  (20)..(20)
<223>  Xaa is (R)-2-amino-2-methyloct-7-enoic acid

<220>
<221>  MISC_FEATURE

<222>  (27)..(27)
<223>  Xaa is (S)-2-amino-2-methylnon-8-enoic acid

<220>
<221>  MISC_FEATURE
<222>  (29)..(29)
<223>  Xaa is C terminal amide

<400>  11

His Xaa Gln Gly Thr Phe Thr Ser Asp Lys Ser Lys Tyr Leu Asp Glu
1               5                   10                  15

Arg Ala Ala Xaa Asp Phe Val Gln Trp Leu Xaa Asp Thr Asn His
            20                  25                  30

<210>  12
<211>  31
<212>  PRT
<213>  Artificial Sequence

<220>
<223>  Peptide

<220>
<221>  MISC_FEATURE
<222>  (1)..(20)
<223>  Miscellaneous feature: the alkene chain of the amino acid at
       position 20 forms a double bond containing ring with the alkene
       chain of the amino acid at position 24

<220>
<221>  MISC_FEATURE
<222>  (2)..(2)
<223>  Xaa is D-Serine

<220>
<221>  MISC_FEATURE
<222>  (10)..(10)
<223>  Xaa is Lysine conjugated to gamma-Glu-gamma-Glu- C16 fatty acid

<220>
<221>  MISC_FEATURE
<222>  (20)..(20)
<223>  Xaa is (S)-2-amino-2-methylhept-6-enoic acid

<220>
<221>  MISC_FEATURE
<222>  (24)..(24)
<223>  Xaa is (S)-2-amino-2-methylhept-6-enoic acid

<220>
<221>  MISC_FEATURE
<222>  (29)..(29)
<223>  Xaa is C terminal amide

<400>  12

His Xaa Gln Gly Thr Phe Thr Ser Asp Xaa Ser Lys Tyr Leu Asp Glu
1               5                   10                  15

Arg Ala Ala Xaa Asp Phe Val Xaa Trp Leu Leu Asp Thr Asn His
            20                  25                  30

<210>  13
<211>  32
<212>  PRT
<213>  Artificial Sequence

<220>
<223>  Peptide

<220>
<221>  MISC_FEATURE
<222>  (1)..(29)
<223>  Miscellaneous feature: the alkene chain of the amino acid at
       position 21 forms a double bond containing ring with the alkene
       chain of the amino acid at position 28

<220>
<221>  MISC_FEATURE
<222>  (2)..(2)
<223>  Xaa is alpha aminoisobutyric acid

<220>
<221>  MISC_FEATURE
<222>  (10)..(10)
<223>  Xaa is Lysine conjugated to gamma-Glu-gamma-Glu- C16 fatty acid

<220>
<221>  MISC_FEATURE
<222>  (16)..(16)
<223>  Xaa is alpha aminoisobutyric acid

<220>
<221>  MISC_FEATURE
<222>  (21)..(21)
<223>  Xaa is (R)-2-amino-2-methyloct-7-enoic acid

<220>
<221>  MISC_FEATURE
<222>  (28)..(28)
<223>  Xaa is (S)-2-amino-2-methylnon-8-enoic acid

<220>
<221>  MISC_FEATURE
<222>  (29)..(29)
<223>  Xaa is C terminal amide

<220>
<221>  MISC_FEATURE
<222>  (30)..(30)
<223>  Xaa is Lysine conjugated to gamma-Glutamic acid

<400>  13

His Xaa Gln Gly Thr Phe Thr Ser Asp Xaa Ser Lys Tyr Leu Asp Xaa
1               5                   10                  15

Arg Ala Ala Gln Xaa Phe Val Gln Trp Leu Met Xaa Thr Xaa Asn His
            20                  25                  30

<210>  14
<211>  32
<212>  PRT
<213>  Artificial Sequence

<220>
<223>  Peptide

<220>
<221>  MISC_FEATURE
<222>  (1)..(20)
<223>  Miscellaneous feature: the alkene chain of the amino acid at
       position 24 forms a double bond containing ring with the alkene
       chain of the amino acid at position 28

<220>
<221>  MISC_FEATURE
<222>  (2)..(2)
<223>  Xaa is alpha aminoisobutyric acid

<220>
<221>  MISC_FEATURE
<222>  (10)..(10)
<223>  Xaa is Lysine conjugated to gamma-Glu-gamma-Glu- C16 fatty acid

<220>
<221>  MISC_FEATURE
<222>  (16)..(16)
<223>  Xaa is alpha aminoisobutyric acid

<220>
<221>  MISC_FEATURE
<222>  (24)..(24)
<223>  Xaa is (S)-2-amino-2-methylhept-6-enoic acid

<220>
<221>  MISC_FEATURE
<222>  (28)..(28)
<223>  Xaa is (S)-2-amino-2-methylhept-6-enoic acid

<220>
<221>  MISC_FEATURE
<222>  (29)..(29)
<223>  Xaa is C terminal amide

<220>
<221>  MISC_FEATURE
<222>  (30)..(30)
<223>  Xaa is Lysine conjugated to gamma-Glutamic acid

<400>  14

His Xaa Gln Gly Thr Phe Thr Ser Asp Xaa Ser Lys Tyr Leu Asp Xaa
1               5                   10                  15

Arg Ala Ala Gln Asp Phe Val Xaa Trp Leu Met Xaa Thr Xaa Asn His
            20                  25                  30

<210>  15
<211>  31
<212>  PRT
<213>  Artificial Sequence

<220>
<223>  Peptide



<220>
<221>  MISC_FEATURE
<222>  (1)..(20)
<223>  Miscellaneous feature: the alkene chain of the amino acid at
       position 20 forms a double bond containing ring with the alkene
       chain of the amino acid at position 24

<220>
<221>  MISC_FEATURE
<222>  (2)..(2)
<223>  Xaa is D-Serine

<220>
<221>  MISC_FEATURE
<222>  (10)..(10)
<223>  Xaa is Lys conjugated to gamma-Glu-gamma-Glu- C16 fatty acid

<220>
<221>  MISC_FEATURE
<222>  (20)..(20)
<223>  Xaa is (S)-2-aminohept-6-enoic acid

<220>
<221>  MISC_FEATURE
<222>  (24)..(24)
<223>  Xaa is (S)-2-aminohept-6-enoic acid

<220>
<221>  MISC_FEATURE
<222>  (29)..(29)
<223>  Xaa is C terminal amide

<400>  15

His Xaa Gln Gly Thr Phe Thr Ser Asp Xaa Ser Lys Tyr Leu Asp Glu
1               5                   10                  15


Arg Ala Ala Xaa Asp Phe Val Xaa Trp Leu Leu Asp Thr Asn His
            20                  25                  30


<210>  16
<211>  32
<212>  PRT
<213>  Artificial Sequence

<220>
<223>  Peptide


<220>

<221>  MISC_FEATURE
<222>  (1)..(20)
<223>  Miscellaneous feature :the alkene chain of the amino acid at
       position 24 forms a double bond containing ring with the alkene
       chain of the amino acid at position 28

<220>
<221>  MISC_FEATURE
<222>  (2)..(2)
<223>  Xaa is alpha aminoisobutyric acid

<220>
<221>  MISC_FEATURE
<222>  (10)..(10)
<223>  Xaa is Lysine conjugated to gamma-Glu-gamma-Glu- C16 fatty acid

<220>
<221>  MISC_FEATURE
<222>  (16)..(16)
<223>  Xaa is alpha aminoisobutyric acid

<220>
<221>  MISC_FEATURE
<222>  (24)..(24)
<223>  Xaa is (S)-2-aminohept-6-enoic acid

<220>
<221>  MISC_FEATURE
<222>  (28)..(28)
<223>  Xaa is (S)-2-aminohept-6-enoic acid

<220>
<221>  MISC_FEATURE
<222>  (29)..(29)
<223>  Xaa is C terminal amide

<220>
<221>  MISC_FEATURE
<222>  (30)..(30)
<223>  Xaa is Lysine conjugated to gamma-Glutamic acid

<400>  16

His Xaa Gln Gly Thr Phe Thr Ser Asp Xaa Ser Lys Tyr Leu Asp Xaa
1               5                   10                  15


Arg Ala Ala Gln Asp Phe Val Xaa Trp Leu Leu Xaa Thr Xaa Asn His
            20                  25                  30


<210>  17
<211>  31
<212>  PRT
<213>  Artificial Sequence

<220>
<223>  Peptide


<220>
<221>  MISC_FEATURE
<222>  (1)..(20)

<223>  Miscellaneous feature: the alkene chain of the amino acid at
       position 20 forms a double bond containing ring with the alkene
       chain of the amino acid at position 24

<220>
<221>  MISC_FEATURE
<222>  (2)..(2)
<223>  Xaa is D-Serine

<220>
<221>  MISC_FEATURE
<222>  (20)..(20)
<223>  Xaa is (S)-2-aminohept-6-enoic acid

<220>
<221>  MISC_FEATURE
<222>  (24)..(24)
<223>  Xaa is (S)-2-aminohept-6-enoic acid

<220>
<221>  MISC_FEATURE
<222>  (29)..(29)
<223>  Xaa is C terminal amide

<400>  17

His Xaa Gln Gly Thr Phe Thr Ser Asp Lys Ser Lys Tyr Leu Asp Glu
1               5                   10                  15


Arg Ala Ala Xaa Asp Phe Val Xaa Trp Leu Leu Asp Thr Asn His
            20                  25                  30


<210>  18
<211>  32
<212>  PRT
<213>  Artificial Sequence

<220>
<223>  Peptide


<220>
<221>  MISC_FEATURE
<222>  (1)..(20)
<223>  Miscellaneous feature: the alkene chain of the amino acid at
       position 24 forms a double bond containing ring with the alkene
       chain of the amino acid at position 28

<220>
<221>  MISC_FEATURE
<222>  (2)..(2)
<223>  Xaa is alpha aminoisobutyric acid

<220>
<221>  MISC_FEATURE
<222>  (16)..(16)
<223>  Xaa is alpha aminoisobutyric acid

<220>
<221>  MISC_FEATURE
<222>  (24)..(24)

<223>  Xaa is (S)-2-aminohept-6-enoic acid

<220>
<221>  MISC_FEATURE
<222>  (28)..(28)
<223>  Xaa is (S)-2-aminohept-6-enoic acid

<220>
<221>  MISC_FEATURE
<222>  (29)..(29)
<223>  Xaa is C terminal amide

<220>
<221>  MISC_FEATURE
<222>  (30)..(30)
<223>  Xaa is Lysine conjugated to gamma-Glutamic acid

<400>  18

His Xaa Gln Gly Thr Phe Thr Ser Asp Lys Ser Lys Tyr Leu Asp Xaa
1               5                   10                  15

Arg Ala Ala Gln Asp Phe Val Xaa Trp Leu Leu Xaa Thr Xaa Asn His
            20                  25                  30

<210>  19
<211>  31
<212>  PRT
<213>  Artificial Sequence

<220>
<223>  Peptide

<220>
<221>  MISC_FEATURE
<222>  (1)..(20)
<223>  Miscellaneous feature: the alkene chain of the amino acid at
       position 20 forms a double bond containing ring with the alkene
       chain of the amino acid at position 24

<220>
<221>  MISC_FEATURE
<222>  (2)..(2)
<223>  Xaa is D-Serine

<220>
<221>  MISC_FEATURE
<222>  (20)..(20)
<223>  Xaa is (S)-2-amino-2-methylhept-6-enoic acid

<220>
<221>  MISC_FEATURE
<222>  (24)..(24)
<223>  Xaa is (S)-2-amino-2-methylhept-6-enoic acid

<220>
<221>  MISC_FEATURE
<222>  (29)..(29)
<223>  Xaa is  C terminal amide

<400>  19

His Xaa Gln Gly Thr Phe Thr Ser Asp Lys Ser Lys Tyr Leu Asp Glu
1               5                   10                  15

Arg Ala Ala Xaa Asp Phe Val Xaa Trp Leu Leu Asp Thr Asn His
            20                  25                  30

<210>  20
<211>  32
<212>  PRT
<213>  Artificial Sequence

<220>
<223>  Peptide

<220>
<221>  MISC_FEATURE
<222>  (1)..(20)
<223>  Miscellaneous feature:  wherein the alkene chain of
       (R)-2-amino-2-methyloct-7-enoic acid forms a double bond
       containing ring with the alkene chain of
       (S)-2-amino-2-methylnon-8-enoic acid; or the alkene chains of

<220>
<221>  MISC_FEATURE
<222>  (2)..(2)
<223>  Xaa is alpha-aminoisobutyric acid or D-Serine

<220>
<221>  MISC_FEATURE
<222>  (10)..(10)
<223>  Xaa is Lysine, Lysine conjugated to a fatty acid, or Lysine
       conjugated to a fatty diacid

<220>
<221>  MISC_FEATURE
<222>  (12)..(12)
<223>  Xaa is (R)-2-amino-2-methyloct-7-enoic acid, or Lysine

<220>
<221>  MISC_FEATURE
<222>  (16)..(16)
<223>  Xaa is  alpha-aminoisobutyric acid, or Glutamic acid

<220>
<221>  MISC_FEATURE
<222>  (17)..(17)
<223>  Xaa is (R)-2-amino-2-methyloct-7-enoic acid, or Arginine

<220>
<221>  MISC_FEATURE
<222>  (19)..(19)
<223>  Xaa is (S)-2-amino-2-methylnon-8-enoic acid, or Alanine

<220>
<221>  MISC_FEATURE
<222>  (20)..(20)
<223>  Xaa is Glutamine, (R)-2-amino-2-methyloct-7-enoic acid,

(S)-2-amino-2-methylhept-6-enoic acid, or (S)-2-aminohept-6-enoic
      acid

<220>
<221>  MISC_FEATURE
<222>  (21)..(21)
<223>  Xaa is Aspartic acid or (R)-2-amino-2-methyloct-7-enoic acid

<220>
<221>  MISC_FEATURE
<222>  (24)..(24)
<223>  Xaa is  Glutamine, (S)-2-amino-2-methylnon-8-enoic acid,
      (S)-2-amino-2-methylhept-6-enoic acid, or (S)-2-aminohept-6-enoic
      acid

<220>
<221>  MISC_FEATURE
<222>  (27)..(27)
<223>  Xaa is Leucine, (S)-2-amino-2-methylnon-8-enoic acid, or
      Methionine

<220>
<221>  MISC_FEATURE
<222>  (28)..(28)
<223>  Xaa is Aspartic acid, (S)-2-amino-2-methylnon-8-enoic acid,
      (S)-2-amino-2-methylhept-6-enoic acid, or (S)-2-aminohept-6-enoic
      acid

<220>
<221>  MISC_FEATURE
<222>  (30)..(30)
<223>  Xaa is absent, or Lysine conjugated by a gamma-glutamic acid
      spacer

<400>  20

His Xaa Gln Gly Thr Phe Thr Ser Asp Xaa Ser Xaa Tyr Leu Asp Xaa
1               5                   10                  15

Xaa Ala Xaa Xaa Xaa Phe Val Xaa Trp Leu Xaa Xaa Thr Xaa Asn His
            20                  25                  30

## Description

### BACKGROUND OF THE INVENTION

The present invention is related to stapled co-agonist peptides of the glucagon and GLP-1 receptors, and the use of these stapled GLP-1 receptor/GCG receptor co-agonists for treatment of metabolic disorders.

Pre-proglucagon is a 158 amino acid precursor polypeptide that is processed in different tissues to form a number of different proglucagon-derived peptides, including glucagon, glucagon-like peptide- 1 (GLP-1), glucagon-like peptide-2 (GLP-2) and oxyntomodulin (OXM), that are involved in a wide variety of physiological functions, including glucose homeostasis, insulin secretion, gastric emptying, and intestinal growth, as well as the regulation of food intake. Glucagon is a 29-amino acid peptide that corresponds to amino acids 33 through 61 of pre-proglucagon, while GLP-1 is produced as a 37-amino acid peptide that corresponds to amino acids 72 through 108 of pre-proglucagon. GLP-1 (7-36) amide or GLP-1 (7-37) acid are biologically potent forms of GLP-1, that demonstrate essentially equivalent activity at the GLP-1 receptor.

During hypoglycemia, when blood glucose levels drop below normal, glucagon signals the liver to break down glycogen and release glucose, causing blood glucose levels to rise toward a normal level. Hypoglycemia is a common side effect of insulin therapy in patients with hyperglycemia (elevated blood glucose levels) due to diabetes. Thus, glucagon's most recognized role in glucose regulation is to counteract the action of insulin and maintain blood glucose levels.

GLP-1 has different biological activities compared to glucagon. Its actions include stimulation of insulin synthesis and secretion, inhibition of glucagon secretion, and inhibition of food intake. GLP-1 has been shown to reduce hyperglycemia in diabetics. Exendin-4, a peptide from lizard venom that shares about 50% amino acid identity with GLP-1, activates the GLP-1 receptor and likewise has been shown to reduce hyperglycemia in diabetics.

There is also evidence that GLP-1 and exendin-4 may reduce food intake and promote weight loss, an effect that would be beneficial not only for diabetics but also for patients suffering from obesity. Patients with obesity have a higher risk of diabetes, hypertension, hyperlipidemia, cardiovascular disease, and musculoskeletal diseases.

Glucagon is a peptide hormone structurally related to GLP-1 that is well recognized for its acute ability to increase blood glucose through stimulation of glycogenolysis and gluconeogenesis (Jiang & Zhang, Am. J. Physio.1 Endocrinol. Metab. 284: E671-E678 (2003)). Of lesser appreciation are the chronic effects of glucagon pharmacology characterized by increases in thermogenesis, satiety, lipolysis, fatty acid oxidation, and ketogenesis (Habegger et al., Nat. Rev. Endocrinol. 6: 689-697 (2010)). Repeated administration of glucagon was first reported decades ago to yield improvements in rodent metabolism, accompanied with lower body weight (Salter, Am. J. Clin. Nutr. 8: 535-539 (1960)). Nonetheless, the inherent risk of hyperglycemia, especially in insulinresistant states such T2DM, has complicated the translation of these observations to human study.

The hormone oxyntomodulin (OXM, glucagon-37) is a posttranslational product of preproglucagon processing in the intestine and central nervous system (CNS) and is secreted from L-cells in the gut in response to food intake. Discovered in 1983, OXM has been implicated in the regulation of food intake and energy expenditure (Jarrouse et al., Endocrinol. 115: 102-105 (1984); Schjoldager et al., Eur. J. Clin. Invest., 18: 499-503 (1988)). Central or peripheral administration of OXM in rats causes a decrease in short term food intake with minimal effects on gastric emptying (Dakin et al. Endocrinology, 142: 4244-4250 (2001), Dakin et al. Endocrinology, 145: 2687-2695 (2004)). Repeated intracerebroventricular administration of OXM in rats results in elevated core temperatures and reduced weight gain compared to pair-fed animals, suggesting effects on both caloric intake and energy expenditure (Dakin et al. Am. J.Physiol. Endocrinol. Metab., 283: E1173-E1177 (2002)).

In related studies, peripheral administration of OXM dose-dependently inhibited both fast-induced and dark phase food intake, but unlike GLP-1, had no effect on gastric emptying.

OXM also reduced levels of fasting ghrelin and increased c-fos immunoreactivity, in the arcuate nucleus (ARC). Repeated seven-day IP administration of OXM caused a reduction in the rate of body weight gain and adiposity in rats (See Dakin et al. Endocrinology, 145: 2687-2695 (2004)).

Studies of OXM action in mice have demonstrated that although OXM can activate both the glucagon (GCG) and the GLP-1 receptors, the anorectic actions of OXM require only the GLP-1 receptor, as icv OXM inhibits food intake in glucagon receptor knockout mice. However, the anorectic effects of OXM are completely absent in GLP-1 receptor knockout mice. Furthermore, exendin-4, but not OXM, regulates energy expenditure in mice. Hence, OXM appears to be a weak agonist at the GLP-1 receptor, when used in pharmacological concentrations (See Baggio et al., Gastroenterol. 127: 546-58 (2004)). OXM was also found to ameliorate glucose intolerance in mice fed a high fat diet (Dakin et al., Am. J. Physiol. Endocrinol. Metab. 294: E142-E147 (2008) and increase the intrinsic heart rate in mice independent of the GLP-1 receptor (Sowden et al., Am. J. Physiol. Regul. Integr. Comp. Physiol. 292: R962-R970 (2007). OXM has also been shown to differentially affect GLP-1 receptor beta-arrestin recruitment and signaling through Galpha (Jorgensen et al., J. Pharma. Exp. Therapeut. 322: 148-154 (2007)) and to differentially affect hypothalamic neuronal activation following peripheral injection of OXM (Choudhri et al., Biochem. Biophys. Res. Commun. 350: 298-306 (2006)).

In humans, a single 90 minute intravenous infusion of OXM in normal weight healthy subjects reduced hunger scores and food intake at a buffet meal by about 19%. Cumulative twelve-hour caloric intake was reduced by about 11% with no reports of nausea or changes in food palatability (Cohen et al., J. Clin. Endocrinol. Metab., 88: 4696-4701 (2003); Lykkegaard et al., ADA Scientific Sessions, Abstract #1506-P (2003)). More recently, pre-prandial injections of OXM over a four-week period in obese healthy volunteers (BMI about 33) led to a significant reduction of caloric intake on the first day of treatment (about 25%) that was maintained over the course of the study (35% reduction after four weeks) (Wynne et al., Diabetes 54: 2390-2395 (2005)). Robust weight loss was observed at the end of the study in treated subjects (1.9%, placebo-corrected). Plasma levels of OXM were similar to that observed in the infusion study (peak concentration about 950 pM). The absence of any tachyphylaxis and a low incidence of mild and transient nausea (about 3%) despite the relatively high doses necessitated by the poor in vivo stability of OXM (plasma tl/2 < 12 minutes) renders this hormone one of the few obesity targets with both human validation and an attractive tolerability profile.

OXM has a very short half-life and is rapidly inactivated by the cell surface dipeptidyl peptidase IV (DPP-IV) (Zhu et al., J. Biol. Chem. 278: 22418-22423 (2002). However, DPP-IV inhibitors are weight-neutral in the clinic, suggesting that supraphysiological levels of OXM (900-1000 pM) may be required to achieve weight loss in humans. OXM peptide analogs for inducing weight loss in humans have been the object of Published International Application Nos. WO03/022304, WO2004/062685, WO2006/134340, and WO2010/096052.

Recently, two independent and simultaneous papers reported the use of relatively balanced GLP-1 receptor/GCG receptor co-agonists as being of enhanced efficacy and safety relative to pure GLP1R agonists in the treatment of rodent obesity, with simultaneous improvement in glycemic control (Day et al., Nat. Chem. Biol. 5: 749-757 (2009); Pocai eta al., Diabetes 58: 2258-2266 (2009)). Of related significance is work with oxyntomodulin (OXM), an endogenous precursor to glucagon, which is secreted postprandially by L-cells of the jejunoileum together with GLP-1 (Holst, Regul. Pept. 93: 45-51 (2000); Drucker, Nat. Clin. Pract. Endocrinol. Metab. 1: 22-31 (2005).

Glucagon peptide analogs and derivatives modified to have various degrees of activity at the GLP-1 receptor and GCG receptor have been disclosed in Published International Application Nos. WO2008/1010017, WO2009/155258, WO2011/075393, WO2012/177444, WO2012/177443; WO2016/065090; and WO2019/060660. Some of the disclosed glucagon peptide analogs were reported therein to have activity at both the GLP-1 receptor and GCG receptor; however, there remains a need for co-agonist peptides that have relatively balanced activity or potency at the GLP-1 receptor and GCG receptor.

### BRIEF SUMMARY OF THE INVENTION

The present invention provides stapled co-agonist peptides of the glucagon (GCG) receptor and the glucagon-like protein 1 (GLP-1) receptor, which have an α-helical conformation due to intramolecular ring formation between two peptide amino acids. The alpha helical structure of the stapled peptides of the present invention results in an increase in physical stability due to decreased proteolytic degradation, and in a lower propensity to self-associate to form β-sheets via hydrogen bonding resulting in a decrease in fibril formation and interpeptide aggregation. Reduced fibril formation and peptide aggregation may result in an increase in peptide efficacy and allows for the use of lower doses of peptide for the same physiological or pharmacological effect. Additionally, the reduction in fibril formation and peptide aggregation leads to a reduction in undesirable amounts of precipitate formation in sterile peptide solutions and peptide formulations. The precipitate formation can lead to cloudy and gel-like suspensions which may adversely affect the dosing accuracy and may cause immunogenic reactions on injection.

The peptides of the present invention are useful for the treatment of metabolic diseases or disorders, such as but not limited to, diabetes (e.g., type 1 diabetes, Type 2 diabetes, or gestational diabetes), non-alcoholic fatty liver disease (NAFLD), non-alcoholic steatohepatitis (NASH), and/or obesity.

### DETAILED DESCRIPTION OF THE INVENTION

The present invention provides stapled peptide co-agonists of the glucagon (GCG) receptor and the glucagon-like protein 1 (GLP-1) receptor that have the amino acid sequences provided below.

In one embodiment, the GCG receptor/GLP-1 receptor co-agonist peptides of the present invention comprise the amino acid sequence of native human glucagon (SEQ ID NO:1) wherein
1) L-Serine at X² is replaced with α-aminoisobutyric acid (Aib), or D-Serine;
2) Tyrosine at X¹⁰ is replaced with Lysine, Lysine conjugated to a fatty acid, or Lysine conjugated to a fatty diacid;
3) L-Serine at X¹⁶ is replaced with α-aminoisobutyric acid (Aib), or Glutamic acid; and
4) X³⁰ is absent, or Lysine conjugated by a gamma-glutamic acid (γE) spacer; and may include up to eight additional amino acid substitutions; wherein the eight additional amino acid substitutions are selected from:
   1) Lysine at X¹² is optionally replaced with (R)-2-amino-2-methyloct-7-enoic acid;
   2) Arginine at X¹⁷ is optionally replaced with (R)-2-amino-2-methyloct-7-enoic acid;
   3) Alanine at X¹⁹ is optionally replaced with (S)-2-amino-2-methylnon-8-enoic acid;
   4) Glutamine at X²⁰ is optionally replaced with (R)-2-amino-2-methyloct-7-enoic acid, (S)-2-amino-2-methylhept-6-enoic acid, or (S)-2-aminohept-6-enoic acid;
   5) Aspartic acid at X²¹ is optionally replaced with (R)-2-amino-2-methyloct-7-enoic acid;
   6) Glutamine at X²⁴ is optionally replaced with (S)-2-amino-2-methylnon-8-enoic acid, (S)-2-amino-2-methylhept-6-enoic acid, or (S)-2-aminohept-6-enoic acid;
   7) Methionine at X²⁷ is optionally replaced with Leucine, or (S)-2-amino-2-methylnon-8-enoic acid; and
   8) Asparagine at X²⁸ is optionally replaced with Aspartic acid, (S)-2-amino-2-methylnon-8-enoic acid, (S)-2-amino-2-methylhept-6-enoic acid, or (S)-2-aminohept-6-enoic acid;
   provided that the peptide contains at least two amino acids selected from (R)-2-amino-2-methyloct-7-enoic acid, (S)-2-amino-2-methylnon-8-enoic acid, (S)-2-amino-2-methylhept-6-enoic acid, and (S)-2-aminohept-6-enoic acid, which cyclize to form a double bond containing ring; or a pharmaceutically acceptable salt thereof.

In a class of this embodiment, the two amino acids acids which cyclize to form a double bond containing ring are selected from: 1) one (R)-2-amino-2-methyloct-7-enoic acid and one (S)-2-amino-2-methylnon-8-enoic acid; 2) two (S)-2-amino-2-methylhept-6-enoic acids; and 3) two (S)-2-aminohept-6-enoic acids.

In another class of this embodiment, the two amino acids acids which cyclize to form a double bond containing ring are one (R)-2-amino-2-methyloct-7-enoic acid and one (S)-2-amino-2-methylnon-8-enoic acid.

In another class of this embodiment, the two amino acids acids which cyclize to form a double bond containing ring are two (S)-2-amino-2-methylhept-6-enoic acids.

In another class of this embodiment, the two amino acids acids which cyclize to form a double bond containing ring are two (S)-2-aminohept-6-enoic acids.

In another class of this embodiment, (R)-2-amino-2-methyloct-7-enoic acid and (S)-2-amino-2-methylnon-8-enoic acid cyclize to form a double bond containing ring; or two (S)-2-amino-2-methylhept-6-enoic acids cyclize to form a double bond containing ring; or two (S)-2-aminohept-6-enoic acids cyclize to form a double bond containing ring.

In another class of this embodiment, (R)-2-amino-2-methyloct-7-enoic acid and (S)-2-amino-2-methylnon-8-enoic acid cyclize to form a double bond containing ring.

In another class of this embodiment, two (S)-2-amino-2-methylhept-6-enoic acids cyclize to form a double bond containing ring.

In another class of this embodiment, two (S)-2-aminohept-6-enoic acids cyclize to form a double bond containing ring.

In another class of this embodiment, the alkene of one (R)-2-amino-2-methyloct-7-enoic acid and the alkene of one (S)-2-amino-2-methylnon-8-enoic acid cyclize to form a double bond containing ring; or the alkenes of two (S)-2-amino-2-methylhept-6-enoic acids cyclize to form a double bond containing ring; or the alkenes of two (S)-2-aminohept-6-enoic acids cyclize to form a double bond containing ring.

In another class of this embodiment, the alkene of one (R)-2-amino-2-methyloct-7-enoic acid and the alkene of one (S)-2-amino-2-methylnon-8-enoic acid cyclize to form a double bond containing ring.

In another class of this embodiment, the alkenes of two (S)-2-amino-2-methylhept-6-enoic acids cyclize to form a double bond containing ring.

In another class of this embodiment, the alkenes of two (S)-2-aminohept-6-enoic acids cyclize to form a double bond containing ring.

In another class of this embodiment, the GCG receptor/GLP-1 receptor co-agonist peptides of the present invention comprise the amino acid sequence of native human glucagon (SEQ ID NO:1) wherein Tyrosine at X¹⁰ is replaced with is Lysine, or Lysine conjugated to a fatty acid; or a pharmaceutically acceptable salt thereof.

In another class of this embodiment, the GCG receptor/GLP-1 receptor co-agonist peptides of the present invention comprise the amino acid sequence of native human glucagon (SEQ ID NO:1) with up to six additional amino acid substitutions are selected from:
1) Lysine at X¹² is optionally replaced with (R)-2-amino-2-methyloct-7-enoic acid;
2) Arginine at X¹⁷ is optionally replaced with (R)-2-amino-2-methyloct-7-enoic acid;
3) Alanine at X¹⁹ is optionally replaced with (S)-2-amino-2-methylnon-8-enoic acid;
4) Glutamine at X²⁰ is optionally replaced with (R)-2-amino-2-methyloct-7-enoic acid, (S)-2-amino-2-methylhept-6-enoic acid, or (S)-2-aminohept-6-enoic acid;
5) Aspartic acid at X²¹ is optionally replaced with (R)-2-amino-2-methyloct-7-enoic acid;
6) Glutamine at X²⁴ is optionally replaced with (S)-2-amino-2-methylnon-8-enoic acid, (S)-2-amino-2-methylhept-6-enoic acid, or (S)-2-aminohept-6-enoic acid;
7) Methionine at X²⁷ is optionally replaced with Leucine, or (S)-2-amino-2-methylnon-8-enoic acid; and
8) Asparagine at X²⁸ is optionally replaced with Aspartic acid, (S)-2-amino-2-methylnon-8-enoic acid, (S)-2-amino-2-methylhept-6-enoic acid, or (S)-2-aminohept-6-enoic acid; or a pharmaceutically acceptable salt thereof.

In another class of this embodiment, the GCG receptor/GLP-1 receptor co-agonist peptides of the present invention comprise the amino acid sequence of native human glucagon (SEQ ID NO:1) with up to four additional amino acid substitutions are selected from:
1) Lysine at X¹² is optionally replaced with (R)-2-amino-2-methyloct-7-enoic acid;
2) Arginine at X¹⁷ is optionally replaced with (R)-2-amino-2-methyloct-7-enoic acid;
3) Alanine at X¹⁹ is optionally replaced with (S)-2-amino-2-methylnon-8-enoic acid;
4) Glutamine at X²⁰ is optionally replaced with (R)-2-amino-2-methyloct-7-enoic acid, (S)-2-amino-2-methylhept-6-enoic acid, or (S)-2-aminohept-6-enoic acid;
5) Aspartic acid at X²¹ is optionally replaced with (R)-2-amino-2-methyloct-7-enoic acid;
6) Glutamine at X²⁴ is optionally replaced with (S)-2-amino-2-methylnon-8-enoic acid, (S)-2-amino-2-methylhept-6-enoic acid, or (S)-2-aminohept-6-enoic acid;
7) Methionine at X²⁷ is optionally replaced with Leucine, or (S)-2-amino-2-methylnon-8-enoic acid; and
8) Asparagine at X²⁸ is optionally replaced with Aspartic acid, (S)-2-amino-2-methylnon-8-enoic acid, (S)-2-amino-2-methylhept-6-enoic acid, or (S)-2-aminohept-6-enoic acid;
or a pharmaceutically acceptable salt thereof.

In another embodiment, the present invention provides a peptide comprising the amino acid sequence
HX²QGTFTSDX¹⁰SX¹²YLDX¹⁶X¹⁷AX¹⁹X²⁰X²¹FVX²⁴WLX²⁷X²⁸TX³⁰-NH₂ (SEQ ID NO:20) wherein
X² is α-aminoisobutyric acid (Aib), or D-Serine;
X¹⁰ is Lysine, Lysine conjugated to a fatty acid, or Lysine conjugated to a fatty diacid;
X¹² is (R)-2-amino-2-methyloct-7-enoic acid, or Lysine;
X¹⁶ is α-aminoisobutyric acid (Aib), or Glutamic acid;
X¹⁷ is (R)-2-amino-2-methyloct-7-enoic acid, or Arginine;
X¹⁹ is (S)-2-amino-2-methylnon-8-enoic acid, or Alanine;
X²⁰ is Glutamine, (R)-2-amino-2-methyloct-7-enoic acid, (S)-2-amino-2-methylhept-6-enoic acid, or (S)-2-aminohept-6-enoic acid;
X²¹ is Aspartic acid or (R)-2-amino-2-methyloct-7-enoic acid;
X²⁴ is Glutamine, (S)-2-amino-2-methylnon-8-enoic acid, (S)-2-amino-2-methylhept-6-enoic acid, or (S)-2-aminohept-6-enoic acid;
X²⁷ is Leucine, (S)-2-amino-2-methylnon-8-enoic acid, or Methionine;
X²⁸ is Aspartic acid, (S)-2-amino-2-methylnon-8-enoic acid, (S)-2-amino-2-methylhept-6-enoic acid, or (S)-2-aminohept-6-enoic acid;
X³⁰ is absent, or Lysine conjugated by a gamma-glutamic acid (γE) spacer;
provided that the peptide contains at least two amino acids selected from (R)-2-amino-2-methyloct-7-enoic acid, (S)-2-amino-2-methylnon-8-enoic acid, (S)-2-amino-2-methylhept-6-enoic acid, and (S)-2-aminohept-6-enoic acid, which cyclize to form a double bond containing ring; and wherein the peptide optionally includes a protecting group that, if present, is joined to the C-terminal carboxy group of the peptide; or a pharmaceutically acceptable salt thereof.

In a class of this embodiment, the two amino acids acids which cyclize to form a double bond containing ring are selected from: 1) one (R)-2-amino-2-methyloct-7-enoic acid and one (S)-2-amino-2-methylnon-8-enoic acid; 2) two (S)-2-amino-2-methylhept-6-enoic acids; and 3) two (S)-2-aminohept-6-enoic acids.

In another class of this embodiment, the two amino acids acids which cyclize to form a double bond containing ring are one (R)-2-amino-2-methyloct-7-enoic acid and one (S)-2-amino-2-methylnon-8-enoic acid.

In another class of this embodiment, the two amino acids acids which cyclize to form a double bond containing ring are two (S)-2-amino-2-methylhept-6-enoic acids.

In another class of this embodiment, the two amino acids acids which cyclize to form a double bond containing ring are two (S)-2-aminohept-6-enoic acids.

In another class of this embodiment, (R)-2-amino-2-methyloct-7-enoic acid and (S)-2-amino-2-methylnon-8-enoic acid cyclize to form a double bond containing ring; or two (S)-2-amino-2-methylhept-6-enoic acids cyclize to form a double bond containing ring; or two (S)-2-aminohept-6-enoic acids cyclize to form a double bond containing ring.

In another class of this embodiment, (R)-2-amino-2-methyloct-7-enoic acid and (S)-2-amino-2-methylnon-8-enoic acid cyclize to form a double bond containing ring.

In another class of this embodiment, two (S)-2-amino-2-methylhept-6-enoic acids cyclize to form a double bond containing ring.

In another class of this embodiment, two (S)-2-aminohept-6-enoic acids cyclize to form a double bond containing ring.

In another class of this embodiment, the alkene of one (R)-2-amino-2-methyloct-7-enoic acid and the alkene of one (S)-2-amino-2-methylnon-8-enoic acid cyclize to form a double bond containing ring; or the alkenes of two (S)-2-amino-2-methylhept-6-enoic acids cyclize to form a double bond containing ring; or the alkenes of two (S)-2-aminohept-6-enoic acids cyclize to form a double bond containing ring.

In another class of this embodiment, the alkene of one (R)-2-amino-2-methyloct-7-enoic acid and the alkene of one (S)-2-amino-2-methylnon-8-enoic acid cyclize to form a double bond containing ring.

In another class of this embodiment, the alkenes of two (S)-2-amino-2-methylhept-6-enoic acids cyclize to form a double bond containing ring.

In another class of this embodiment, the alkenes of two (S)-2-aminohept-6-enoic acids cyclize to form a double bond containing ring.

In another class of this embodiment, X¹⁰ is Lysine, Lysine conjugated to a fatty acid by a gamma-glutamic acid - gamma-glutamic acid linker, or Lysine conjugated to a fatty diacid via a gamma-glutamic acid - gamma-glutamic acid linker.

In another class of this embodiment, the fatty diacid at position 10 comprises a C14, C15, C16, C17, C18, C19, or C20 fatty diacid, and the fatty acid at position 10 comprises a C14, C16, C17, C18, C19, or C20 fatty acid.

In another class of this embodiment, the fatty diacid at position 10 comprises a C14, C15, C16, C17, C18, C19, or C20 fatty diacid. In another class of this embodiment, the fatty diacid comprises a C16 or C18 fatty diacid. In another class of this embodiment, the fatty diacid comprises a C18 fatty diacid. In another class of this embodiment, the fatty diacid comprises a C16 fatty diacid.

In another class of this embodiment, the fatty acid at position 10 comprises a C14, C16, C17, C18, C19, or C20 fatty acid. In another class of this embodiment, the fatty acid comprises a C16 or C18 fatty acid. In another class of this embodiment, the fatty acid comprises a C18 fatty acid. In another class of this embodiment, the fatty acid comprises a C16 fatty acid.

In another class of this embodiment, X¹⁰ is Lysine, or Lysine conjugated to a fatty acid.

In another class of this embodiment, X¹⁰ is Lysine, or Lysine conjugated to a fatty acid via a gamma-glutamic acid - gamma-glutamic acid linker.

In another class of this embodiment, X¹⁰ is Lysine or Lysine conjugated to a C16 fatty acid via a gamma-glutamic acid - gamma-glutamic acid linker.

In another class of this embodiment, X¹⁰ is Lysine.

In another class of this embodiment, X¹⁰ is Lysine conjugated to a fatty acid via a gamma-glutamic acid - gamma-glutamic acid linker.

In another class of this embodiment, X¹⁰ is Lysine conjugated to a C16 or C18 fatty acid via a gamma-glutamic acid - gamma-glutamic acid linker.

In another class of this embodiment, X¹⁰ is Lysine conjugated to a C18 fatty acid via a gamma-glutamic acid - gamma-glutamic acid linker.

In another class of this embodiment, X¹⁰ is Lysine conjugated to a C16 fatty acid via a gamma-glutamic acid - gamma-glutamic acid linker.

In another class of this embodiment, the peptide comprises at X¹⁰ a Lysine or a Lysine conjugated to a fatty diacid via a gamma-glutamic acid - gamma-glutamic acid linker.

In another class of this embodiment, the peptide comprises at X¹⁰ a Lysine, or a Lysine conjugated to a C16 or C18 fatty diacid via a gamma-glutamic acid - gamma-glutamic acid linker.

In another class of this embodiment, the peptide comprises at X¹⁰ a Lys conjugated to a C18 fatty diacid via a gamma-glutamic acid - gamma-glutamic acid linker.

In another class of this embodiment, the peptide comprises at X¹⁰ a Lys conjugated to a C16 fatty diacid via a gamma-glutamic acid - gamma-glutamic acid linker.

In another class of this embodiment, X³⁰ is absent. In another class of this embodiment, X³⁰ is Lysine conjugated by a gamma-glutamic acid (γE) spacer.

In another class of this embodiment, X² is α-aminoisobutyric acid, or D-Serine. In another class of this embodiment, X² is α-aminoisobutyric acid. In another class of this embodiment, X² is D-Serine.

In another class of this embodiment, X¹² is (R)-2-amino-2-methyloct-7-enoic acid. In another class of this embodiment, X¹² is Lysine.

In another class of this embodiment, X¹⁶ is α-aminoisobutyric acid, or Glutamic acid. In another class of this embodiment, X¹⁶ is α-aminoisobutyric acid. In another class of this embodiment, X¹⁶ is Glutamic acid.

In another class of this embodiment, X¹⁷ is (R)-2-amino-2-methyloct-7-enoic acid. In another class of this embodiment, X¹⁷ is Arginine.

In another class of this embodiment, X¹⁹ is (S)-2-amino-2-methylnon-8-enoic acid. In another class of this embodiment, X¹⁹ is Alanine.

In another class of this embodiment, X²⁰ is Glutamine. In another class of this embodiment, X²⁰ is (R)-2-amino-2-methyloct-7-enoic acid. In another class of this embodiment, X²⁰ is (S)-2-amino-2-methylhept-6-enoic acid. In another class of this embodiment, X²⁰ is (S)-2-aminohept-6-enoic acid.

In another class of this embodiment, X²¹ is Aspartic acid. In another class of this embodiment, X²¹ is (R)-2-amino-2-methyloct-7-enoic acid.

In another class of this embodiment, X²⁴ is Glutamine. In another class of this embodiment, X²⁴ is (S)-2-amino-2-methylnon-8-enoic acid. In another class of this embodiment, X²⁴ is (S)-2-amino-2-methylhept-6-enoic acid. In another class of this embodiment, X²⁴ is (S)-2-aminohept-6-enoic acid.

In another class of this embodiment, X²⁷ is Leucine. In another class of this embodiment, X²⁷ is (S)-2-amino-2-methylnon-8-enoic acid. In another class of this embodiment, X²⁷ is Methionine.

In another class of this embodiment, X²⁸ is Aspartic acid. In another class of this embodiment, X²⁸ is (S)-2-amino-2-methylnon-8-enoic acid. In another class of this embodiment, X²⁸ is (S)-2-amino-2-methylhept-6-enoic acid. In another class of this embodiment, X²⁸ is (S)-2-aminohept-6-enoic acid.

In another class of this embodiment, the peptide includes a protecting group that is joined to the C-terminal carboxy group of the peptide.

In another class of this embodiment, the peptide does not include a protecting group that is joined to the C-terminal carboxy group.

In another class of this embodiment, the peptide has the amino acid sequence of SEQ ID NO: 6, 7, 8, 9, 10, 11, 12, 13, 14, 15, 16, 17, 18, or 19.

The aforementioned GCG/GLP-1 receptor co-agonist peptides have the structure as shown in Table 1.

In another embodiment, the present invention provides a peptide comprising the amino acid sequence
HX²QGTFTSDX¹⁰SX¹²YLDX¹⁶X¹⁷AX¹⁹X²⁰X²¹FVX²⁴WLX²⁷X²⁸TX³⁰-NH₂ (SEQ ID NO:20) wherein
X² is α-aminoisobutyric acid (Aib), or D-Serine;
X¹⁰ is Lysine, or Lysine conjugated to a fatty acid;
X¹² is (R)-2-amino-2-methyloct-7-enoic acid, or Lysine;
X¹⁶ is α-aminoisobutyric acid (Aib), or Glutamic acid;
X¹⁷ is (R)-2-amino-2-methyloct-7-enoic acid, or Arginine;
X¹⁹ is (S)-2-amino-2-methylnon-8-enoic acid, or Alanine;
X²⁰ is Glutamine, (R)-2-amino-2-methyloct-7-enoic acid, (S)-2-amino-2-methylhept-6-enoic acid, or (S)-2-aminohept-6-enoic acid;
X²¹ is Aspartic acid or (R)-2-amino-2-methyloct-7-enoic acid;
X²⁴ is Glutamine, (S)-2-amino-2-methylnon-8-enoic acid, (S)-2-amino-2-methylhept-6-enoic acid, or (S)-2-aminohept-6-enoic acid;
X²⁷ is Leucine, (S)-2-amino-2-methylnon-8-enoic acid, or Methionine;
X²⁸ is Aspartic acid, (S)-2-amino-2-methylnon-8-enoic acid, (S)-2-amino-2-methylhept-6-enoic acid, or (S)-2-aminohept-6-enoic acid;
X³⁰ is absent, or Lysine conjugated by a gamma-glutamic acid (γE) spacer;
provided that the peptide contains at least two amino acids selected from (R)-2-amino-2-methyloct-7-enoic acid, (S)-2-amino-2-methylnon-8-enoic acid, (S)-2-amino-2-methylhept-6-enoic acid, and (S)-2-aminohept-6-enoic acid, which cyclize to form a double bond containing ring; and wherein the peptide optionally includes a protecting group that, if present, is joined to the C-terminal carboxy group of the peptide; or a pharmaceutically acceptable salt thereof.

In a class of this embodiment, the two amino acids acids which cyclize to form a double bond containing ring are selected from: 1) one (R)-2-amino-2-methyloct-7-enoic acid and one (S)-2-amino-2-methylnon-8-enoic acid; 2) two (S)-2-amino-2-methylhept-6-enoic acids; and 3) two (S)-2-aminohept-6-enoic acids.

In another class of this embodiment, the two amino acids acids which cyclize to form a double bond containing ring are one (R)-2-amino-2-methyloct-7-enoic acid and one (S)-2-amino-2-methylnon-8-enoic acid.

In another class of this embodiment, the two amino acids acids which cyclize to form a double bond containing ring are two (S)-2-amino-2-methylhept-6-enoic acids.

In another class of this embodiment, the two amino acids acids which cyclize to form a double bond containing ring are two (S)-2-aminohept-6-enoic acids.

In another class of this embodiment, (R)-2-amino-2-methyloct-7-enoic acid and (S)-2-amino-2-methylnon-8-enoic acid cyclize to form a double bond containing ring; or two (S)-2-amino-2-methylhept-6-enoic acids cyclize to form a double bond containing ring; or two (S)-2-aminohept-6-enoic acids cyclize to form a double bond containing ring.

In another class of this embodiment, (R)-2-amino-2-methyloct-7-enoic acid and (S)-2-amino-2-methylnon-8-enoic acid cyclize to form a double bond containing ring.

In another class of this embodiment, two (S)-2-amino-2-methylhept-6-enoic acids cyclize to form a double bond containing ring.

In another class of this embodiment, two (S)-2-aminohept-6-enoic acids cyclize to form a double bond containing ring.

In another class of this embodiment, the alkene of one (R)-2-amino-2-methyloct-7-enoic acid and the alkene of one (S)-2-amino-2-methylnon-8-enoic acid cyclize to form a double bond containing ring; or the alkenes of two (S)-2-amino-2-methylhept-6-enoic acids cyclize to form a double bond containing ring; or the alkenes of two (S)-2-aminohept-6-enoic acids cyclize to form a double bond containing ring.

In another class of this embodiment, the alkene of one (R)-2-amino-2-methyloct-7-enoic acid and the alkene of one (S)-2-amino-2-methylnon-8-enoic acid cyclize to form a double bond containing ring.

In another class of this embodiment, the alkenes of two (S)-2-amino-2-methylhept-6-enoic acids cyclize to form a double bond containing ring.

In another class of this embodiment, the alkenes of two (S)-2-aminohept-6-enoic acids cyclize to form a double bond containing ring.

In another class of this embodiment, X¹⁰ is Lysine, or Lysine conjugated to a fatty acid via a gamma-glutamic acid - gamma-glutamic acid linker.

In another class of this embodiment, X¹⁰ is Lysine or Lysine conjugated to a C16 fatty acid via a gamma-glutamic acid - gamma-glutamic acid linker.

In another class of this embodiment, the fatty acid at position 10 comprises a C14, C16, C17, C18, C19, or C20 fatty acid. In another class of this embodiment, the fatty acid comprises a C16 or C18 fatty acid. In another class of this embodiment, the fatty acid comprises a C18 fatty acid. In another class of this embodiment, the fatty acid comprises a C16 fatty acid.

In another class of this embodiment, X¹⁰ is Lysine.

In another class of this embodiment, X¹⁰ is Lysine conjugated to a fatty acid via a gamma-glutamic acid - gamma-glutamic acid linker.

In another class of this embodiment, X¹⁰ is Lysine conjugated to a C16 or C18 fatty acid via a gamma-glutamic acid - gamma-glutamic acid linker.

In another class of this embodiment, X¹⁰ is Lysine conjugated to a C18 fatty acid via a gamma-glutamic acid - gamma-glutamic acid linker.

In another class of this embodiment, X¹⁰ is Lysine conjugated to a C16 fatty acid via a gamma-glutamic acid - gamma-glutamic acid linker.

In another class of this embodiment, X³⁰ is absent. In another class of this embodiment, X³⁰ is Lysine conjugated by a gamma-glutamic acid (γE) spacer.

In another class of this embodiment, X² is α-aminoisobutyric acid, or D-Serine. In another class of this embodiment, X² is α-aminoisobutyric acid. In another class of this embodiment, X² is D-Serine.

In another class of this embodiment, X¹² is (R)-2-amino-2-methyloct-7-enoic acid. In another class of this embodiment, X¹² is Lysine.

In another class of this embodiment, X¹⁶ is α-aminoisobutyric acid, or Glutamic acid. In another class of this embodiment, X¹⁶ is α-aminoisobutyric acid. In another class of this embodiment, X¹⁶ is Glutamic acid.

In another class of this embodiment, X¹⁷ is (R)-2-amino-2-methyloct-7-enoic acid. In another class of this embodiment, X¹⁷ is Arginine.

In another class of this embodiment, X¹⁹ is (S)-2-amino-2-methylnon-8-enoic acid. In another class of this embodiment, X¹⁹ is Alanine.

In another class of this embodiment, X²⁰ is Glutamine. In another class of this embodiment, X²⁰ is (R)-2-amino-2-methyloct-7-enoic acid. In another class of this embodiment, X²⁰ is (S)-2-amino-2-methylhept-6-enoic acid. In another class of this embodiment, X²⁰ is (S)-2-aminohept-6-enoic acid.

In another class of this embodiment, X²¹ is Aspartic acid. In another class of this embodiment, X²¹ is (R)-2-amino-2-methyloct-7-enoic acid.

In another class of this embodiment, X²⁴ is Glutamine. In another class of this embodiment, X²⁴ is (S)-2-amino-2-methylnon-8-enoic acid. In another class of this embodiment, X²⁴ is (S)-2-amino-2-methylhept-6-enoic acid. In another class of this embodiment, X²⁴ is (S)-2-aminohept-6-enoic acid.

In another class of this embodiment, X²⁷ is Leucine. In another class of this embodiment, X²⁷ is (S)-2-amino-2-methylnon-8-enoic acid. In another class of this embodiment, X²⁷ is Methionine.

In another class of this embodiment, X²⁸ is Aspartic acid. In another class of this embodiment, X²⁸ is (S)-2-amino-2-methylnon-8-enoic acid. In another class of this embodiment, X²⁸ is (S)-2-amino-2-methylhept-6-enoic acid. In another class of this embodiment, X²⁸ is (S)-2-aminohept-6-enoic acid.

In another class of this embodiment, the peptide includes a protecting group that is joined to the C-terminal carboxy group of the peptide.

In another class of this embodiment, the peptide does not include a protecting group that is joined to the C-terminal carboxy group.

In another class of this embodiment, the peptide has the amino acid sequence of SEQ ID NO: 6, 7, 8, 9, 10, 11, 12, 13, 14, 15, 16, 17, 18, or 19.

In another embodiment, the present invention provides a peptide comprising the amino acid sequence
HX²QGTFTSDX¹⁰SX¹²YLDX¹⁶X¹⁷AX¹⁹X²⁰X²¹FVX²⁴WLX²⁷X²⁸TX³⁰-NH₂ (SEQ ID NO:20) wherein
X² is α-aminoisobutyric acid (Aib), or D-Serine;
X¹⁰ is Lysine, or Lysine conjugated to a fatty acid via a gamma-glutamic acid - gamma-glutamic acid linker;
X¹² is (R)-2-amino-2-methyloct-7-enoic acid, or Lysine;
X¹⁶ is α-aminoisobutyric acid (Aib), or Glutamic acid;
X¹⁷ is (R)-2-amino-2-methyloct-7-enoic acid, or Arginine;
X¹⁹ is (S)-2-amino-2-methylnon-8-enoic acid, or Alanine;
X²⁰ is Glutamine, (R)-2-amino-2-methyloct-7-enoic acid, (S)-2-amino-2-methylhept-6-enoic acid, or (S)-2-aminohept-6-enoic acid;
X²¹ is Aspartic acid or (R)-2-amino-2-methyloct-7-enoic acid;
X²⁴ is Glutamine, (S)-2-amino-2-methylnon-8-enoic acid, (S)-2-amino-2-methylhept-6-enoic acid, or (S)-2-aminohept-6-enoic acid;
X²⁷ is Leucine, (S)-2-amino-2-methylnon-8-enoic acid, or Methionine;
X²⁸ is Aspartic acid, (S)-2-amino-2-methylnon-8-enoic acid, (S)-2-amino-2-methylhept-6-enoic acid, or (S)-2-aminohept-6-enoic acid;
X³⁰ is absent, or Lysine conjugated by a gamma-glutamic acid (γE) spacer; provided that the peptide contains at least two amino acids selected from (R)-2-amino-2-methyloct-7-enoic acid, (S)-2-amino-2-methylnon-8-enoic acid, (S)-2-amino-2-methylhept-6-enoic acid, and (S)-2-aminohept-6-enoic acid, which cyclize to form a double bond containing ring; and wherein the peptide optionally includes a protecting group that, if present, is joined to the C-terminal carboxy group of the peptide; or a pharmaceutically acceptable salt thereof.

In a class of this embodiment, the two amino acids acids which cyclize to form a double bond containing ring are selected from: 1) one (R)-2-amino-2-methyloct-7-enoic acid and one (S)-2-amino-2-methylnon-8-enoic acid; 2) two (S)-2-amino-2-methylhept-6-enoic acids; and 3) two (S)-2-aminohept-6-enoic acids.

In another class of this embodiment, the two amino acids acids which cyclize to form a double bond containing ring are one (R)-2-amino-2-methyloct-7-enoic acid and one (S)-2-amino-2-methylnon-8-enoic acid.

In another class of this embodiment, the two amino acids acids which cyclize to form a double bond containing ring are two (S)-2-amino-2-methylhept-6-enoic acids.

In another class of this embodiment, the two amino acids acids which cyclize to form a double bond containing ring are two (S)-2-aminohept-6-enoic acids.

In another class of this embodiment, (R)-2-amino-2-methyloct-7-enoic acid and (S)-2-amino-2-methylnon-8-enoic acid cyclize to form a double bond containing ring; or two (S)-2-amino-2-methylhept-6-enoic acids cyclize to form a double bond containing ring; or two (S)-2-aminohept-6-enoic acids cyclize to form a double bond containing ring.

In another class of this embodiment, (R)-2-amino-2-methyloct-7-enoic acid and (S)-2-amino-2-methylnon-8-enoic acid cyclize to form a double bond containing ring.

In another class of this embodiment, two (S)-2-amino-2-methylhept-6-enoic acids cyclize to form a double bond containing ring.

In another class of this embodiment, two (S)-2-aminohept-6-enoic acids cyclize to form a double bond containing ring.

In another class of this embodiment, the alkene of one (R)-2-amino-2-methyloct-7-enoic acid and the alkene of one (S)-2-amino-2-methylnon-8-enoic acid cyclize to form a double bond containing ring; or the alkenes of two (S)-2-amino-2-methylhept-6-enoic acids cyclize to form a double bond containing ring; or the alkenes of two (S)-2-aminohept-6-enoic acids cyclize to form a double bond containing ring.

In another class of this embodiment, the alkene of one (R)-2-amino-2-methyloct-7-enoic acid and the alkene of one (S)-2-amino-2-methylnon-8-enoic acid cyclize to form a double bond containing ring.

In another class of this embodiment, the alkenes of two (S)-2-amino-2-methylhept-6-enoic acids cyclize to form a double bond containing ring.

In another class of this embodiment, the alkenes of two (S)-2-aminohept-6-enoic acids cyclize to form a double bond containing ring.

In a class of this embodiment, the fatty acid at position 10 comprises a C14, C16, C17, C18, C19, or C20 fatty acid. In another class of this embodiment, the fatty acid comprises a C16 or C18 fatty acid. In another class of this embodiment, the fatty acid comprises a C18 fatty acid. In another class of this embodiment, the fatty acid comprises a C16 fatty acid.

In another class of this embodiment, X¹⁰ is Lysine or Lysine conjugated to a C16 fatty acid via a gamma-glutamic acid - gamma-glutamic acid linker.

In another class of this embodiment, X¹⁰ is Lysine.

In another class of this embodiment, X¹⁰ is Lysine conjugated to a fatty acid via a gamma-glutamic acid - gamma-glutamic acid linker.

In another class of this embodiment, X¹⁰ is Lysine conjugated to a C16 or C18 fatty acid via a gamma-glutamic acid - gamma-glutamic acid linker.

In another class of this embodiment, X¹⁰ is Lysine conjugated to a C18 fatty acid via a gamma-glutamic acid - gamma-glutamic acid linker.

In another class of this embodiment, X¹⁰ is Lysine conjugated to a C16 fatty acid via a gamma-glutamic acid - gamma-glutamic acid linker.

In another class of this embodiment, X³⁰ is absent. In another class of this embodiment, X³⁰ is Lysine conjugated by a gamma-glutamic acid (γE) spacer.

In another class of this embodiment, X² is α-aminoisobutyric acid, or D-Serine. In another class of this embodiment, X² is α-aminoisobutyric acid. In another class of this embodiment, X² is D-Serine.

In another class of this embodiment, X¹² is (R)-2-amino-2-methyloct-7-enoic acid. In another class of this embodiment, X¹² is Lysine.

In another class of this embodiment, X¹⁶ is α-aminoisobutyric acid, or Glutamic acid. In another class of this embodiment, X¹⁶ is α-aminoisobutyric acid. In another class of this embodiment, X¹⁶ is Glutamic acid.

In another class of this embodiment, X¹⁷ is (R)-2-amino-2-methyloct-7-enoic acid. In another class of this embodiment, X¹⁷ is Arginine.

In another class of this embodiment, X¹⁹ is (S)-2-amino-2-methylnon-8-enoic acid. In another class of this embodiment, X¹⁹ is Alanine.

In another class of this embodiment, X²⁰ is Glutamine. In another class of this embodiment, X²⁰ is (R)-2-amino-2-methyloct-7-enoic acid. In another class of this embodiment, X²⁰ is (S)-2-amino-2-methylhept-6-enoic acid. In another class of this embodiment, X²⁰ is (S)-2-aminohept-6-enoic acid.

In another class of this embodiment, X²¹ is Aspartic acid. In another class of this embodiment, X²¹ is (R)-2-amino-2-methyloct-7-enoic acid.

In another class of this embodiment, X²⁴ is Glutamine. In another class of this embodiment, X²⁴ is (S)-2-amino-2-methylnon-8-enoic acid. In another class of this embodiment, X²⁴ is (S)-2-amino-2-methylhept-6-enoic acid. In another class of this embodiment, X²⁴ is (S)-2-aminohept-6-enoic acid.

In another class of this embodiment, X²⁷ is Leucine. In another class of this embodiment, X²⁷ is (S)-2-amino-2-methylnon-8-enoic acid. In another class of this embodiment, X²⁷ is Methionine.

In another class of this embodiment, X²⁸ is Aspartic acid. In another class of this embodiment, X²⁸ is (S)-2-amino-2-methylnon-8-enoic acid. In another class of this embodiment, X²⁸ is (S)-2-amino-2-methylhept-6-enoic acid. In another class of this embodiment, X²⁸ is (S)-2-aminohept-6-enoic acid.

In another class of this embodiment, the peptide includes a protecting group that is joined to the C-terminal carboxy group of the peptide.

In another class of this embodiment, the peptide does not include a protecting group that is joined to the C-terminal carboxy group.

In particular embodiments, the present invention provides a peptide consisting of the amino acid sequence
HX²QGTFTSDX¹⁰SX¹²YLDX¹⁶X¹⁷AX¹⁹X²⁰X²¹FVX²⁴WLX²⁷X²⁸TX³⁰-NH₂ (SEQ ID NO:20) wherein
X² is α-aminoisobutyric acid (Aib), or D-Serine;
X¹⁰ is Lysine, Lysine conjugated to a fatty acid, or Lysine conjugated to a fatty diacid;
X¹² is (R)-2-amino-2-methyloct-7-enoic acid, or Lysine;
X¹⁶ is α-aminoisobutyric acid (Aib), or Glutamic acid;
X¹⁷ is (R)-2-amino-2-methyloct-7-enoic acid, or Arginine;
X¹⁹ is (S)-2-amino-2-methylnon-8-enoic acid, or Alanine;
X²⁰ is Glutamine, (R)-2-amino-2-methyloct-7-enoic acid, (S)-2-amino-2-methylhept-6-enoic acid, or (S)-2-aminohept-6-enoic acid;
X²¹ is Aspartic acid or (R)-2-amino-2-methyloct-7-enoic acid;
X²⁴ is Glutamine, (S)-2-amino-2-methylnon-8-enoic acid, (S)-2-amino-2-methylhept-6-enoic acid, or (S)-2-aminohept-6-enoic acid;
X²⁷ is Leucine, (S)-2-amino-2-methylnon-8-enoic acid, or Methionine;
X²⁸ is Aspartic acid, (S)-2-amino-2-methylnon-8-enoic acid, (S)-2-amino-2-methylhept-6-enoic acid, or (S)-2-aminohept-6-enoic acid;
X³⁰ is absent, or Lysine conjugated by a gamma-glutamic acid (γE) spacer; provided that the peptide contains at least two amino acids selected from (R)-2-amino-2-methyloct-7-enoic acid, (S)-2-amino-2-methylnon-8-enoic acid, (S)-2-amino-2-methylhept-6-enoic acid, and (S)-2-aminohept-6-enoic acid, which cyclize to form a double bond containing ring; and wherein the peptide optionally includes a protecting group that, if present, is joined to the C-terminal carboxy group of the peptide; or a pharmaceutically acceptable salt thereof.

In a class of this embodiment, the two amino acids acids which cyclize to form a double bond containing ring are selected from: 1) one (R)-2-amino-2-methyloct-7-enoic acid and one (S)-2-amino-2-methylnon-8-enoic acid; 2) two (S)-2-amino-2-methylhept-6-enoic acids; and 3) two (S)-2-aminohept-6-enoic acids.

In another class of this embodiment, the two amino acids acids which cyclize to form a double bond containing ring are one (R)-2-amino-2-methyloct-7-enoic acid and one (S)-2-amino-2-methylnon-8-enoic acid

In another class of this embodiment, the two amino acids acids which cyclize to form a double bond containing ring are two (S)-2-amino-2-methylhept-6-enoic acids.

In another class of this embodiment, the two amino acids acids which cyclize to form a double bond containing ring are two (S)-2-aminohept-6-enoic acids.

In another class of this embodiment, (R)-2-amino-2-methyloct-7-enoic acid and (S)-2-amino-2-methylnon-8-enoic acid cyclize to form a double bond containing ring; or two (S)-2-amino-2-methylhept-6-enoic acids cyclize to form a double bond containing ring; or two (S)-2-aminohept-6-enoic acids cyclize to form a double bond containing ring.

In another class of this embodiment, (R)-2-amino-2-methyloct-7-enoic acid and (S)-2-amino-2-methylnon-8-enoic acid cyclize to form a double bond containing ring.

In another class of this embodiment, two (S)-2-amino-2-methylhept-6-enoic acids cyclize to form a double bond containing ring.

In another class of this embodiment, two (S)-2-aminohept-6-enoic acids cyclize to form a double bond containing ring.

In another class of this embodiment, the alkene of one (R)-2-amino-2-methyloct-7-enoic acid and the alkene of one (S)-2-amino-2-methylnon-8-enoic acid cyclize to form a double bond containing ring; or the alkenes of two (S)-2-amino-2-methylhept-6-enoic acids cyclize to form a double bond containing ring; or the alkenes of two (S)-2-aminohept-6-enoic acids cyclize to form a double bond containing ring.

In another class of this embodiment, the alkene of one (R)-2-amino-2-methyloct-7-enoic acid and the alkene of one (S)-2-amino-2-methylnon-8-enoic acid cyclize to form a double bond containing ring.

In another class of this embodiment, the alkenes of two (S)-2-amino-2-methylhept-6-enoic acids cyclize to form a double bond containing ring.

In another class of this embodiment, the alkenes of (S)-2-aminohept-6-enoic acids cyclize to form a double bond containing ring.

In another class of this embodiment, X¹⁰ is Lysine, Lysine conjugated to a fatty acid by a gamma-glutamic acid - gamma-glutamic acid linker, or Lysine conjugated to a fatty diacid via a gamma-glutamic acid - gamma-glutamic acid linker.

In another class of this embodiment, the fatty diacid at position 10 comprises a C14, C15, C16, C17, C18, C19, or C20 fatty diacid, and the fatty acid at position 10 comprises a C14, C16, C17, C18, C19, or C20 fatty acid.

In another class of this embodiment, the fatty diacid at position 10 comprises a C14, C15, C16, C17, C18, C19, or C20 fatty diacid. In another class of this embodiment, the fatty diacid comprises a C16 or C18 fatty diacid. In another class of this embodiment, the fatty diacid comprises a C18 fatty diacid. In another class of this embodiment, the fatty diacid comprises a C16 fatty diacid.

In another class of this embodiment, the fatty acid at position 10 comprises a C14, C16, C17, C18, C19, or C20 fatty acid. In another class of this embodiment, the fatty acid comprises a C16 or C18 fatty acid. In another class of this embodiment, the fatty acid comprises a C18 fatty acid. In another class of this embodiment, the fatty acid comprises a C16 fatty acid.

In another class of this embodiment, X¹⁰ is Lysine, or Lysine conjugated to a fatty acid.

In another class of this embodiment, X¹⁰ is Lysine, or Lysine conjugated to a fatty acid via a gamma-glutamic acid - gamma-glutamic acid linker.

In another class of this embodiment, X¹⁰ is Lysine or Lysine conjugated to a C16 fatty acid via a gamma-glutamic acid - gamma-glutamic acid linker.

In another class of this embodiment, X¹⁰ is Lysine.

In another class of this embodiment, X¹⁰ is Lysine conjugated to a fatty acid via a gamma-glutamic acid - gamma-glutamic acid linker.

In another class of this embodiment, X¹⁰ is Lysine conjugated to a C16 or C18 fatty acid via a gamma-glutamic acid - gamma-glutamic acid linker.

In another class of this embodiment, X¹⁰ is Lysine conjugated to a C18 fatty acid via a gamma-glutamic acid - gamma-glutamic acid linker.

In another class of this embodiment, X¹⁰ is Lysine conjugated to a C16 fatty acid via a gamma-glutamic acid - gamma-glutamic acid linker.

In another class of this embodiment, the peptide comprises at X¹⁰ a Lysine or a Lysine conjugated to a fatty diacid via a gamma-glutamic acid - gamma-glutamic acid linker.

In another class of this embodiment, the peptide comprises at X¹⁰ a Lysine, or a Lysine conjugated to a C16 or C18 fatty diacid via a gamma-glutamic acid - gamma-glutamic acid linker.

In another class of this embodiment, the peptide comprises at X¹⁰ a Lys conjugated to a C18 fatty diacid via a gamma-glutamic acid - gamma-glutamic acid linker.

In another class of this embodiment, the peptide comprises at X¹⁰ a Lys conjugated to a C16 fatty diacid via a gamma-glutamic acid - gamma-glutamic acid linker.

In another class of this embodiment, X³⁰ is absent. In another class of this embodiment, X³⁰ is Lysine conjugated by a gamma-glutamic acid (γE) spacer.

In another class of this embodiment, X² is α-aminoisobutyric acid, or D-Serine. In another class of this embodiment, X² is α-aminoisobutyric acid. In another class of this embodiment, X² is D-Serine.

In another class of this embodiment, X¹² is (R)-2-amino-2-methyloct-7-enoic acid. In another class of this embodiment, X¹² is Lysine.

In another class of this embodiment, X¹⁶ is α-aminoisobutyric acid, or Glutamic acid. In another class of this embodiment, X¹⁶ is a-aminoisobutyric acid. In another class of this embodiment, X¹⁶ is Glutamic acid.

In another class of this embodiment, X¹⁷ is (R)-2-amino-2-methyloct-7-enoic acid. In another class of this embodiment, X¹⁷ is Arginine.

In another class of this embodiment, X¹⁹ is (S)-2-amino-2-methylnon-8-enoic acid. In another class of this embodiment, X¹⁹ is Alanine.

In another class of this embodiment, X²⁰ is Glutamine. In another class of this embodiment, X²⁰ is (R)-2-amino-2-methyloct-7-enoic acid. In another class of this embodiment, X²⁰ is (S)-2-amino-2-methylhept-6-enoic acid. In another class of this embodiment, X²⁰ is (S)-2-aminohept-6-enoic acid.

In another class of this embodiment, X²¹ is Aspartic acid. In another class of this embodiment, X²¹ is (R)-2-amino-2-methyloct-7-enoic acid.

In another class of this embodiment, X²⁴ is Glutamine. In another class of this embodiment, X²⁴ is (S)-2-amino-2-methylnon-8-enoic acid. In another class of this embodiment, X²⁴ is (S)-2-amino-2-methylhept-6-enoic acid. In another class of this embodiment, X²⁴ is (S)-2-aminohept-6-enoic acid.

In another class of this embodiment, X²⁷ is Leucine. In another class of this embodiment, X²⁷ is (S)-2-amino-2-methylnon-8-enoic acid. In another class of this embodiment, X²⁷ is Methionine.

In another class of this embodiment, X²⁸ is Aspartic acid. In another class of this embodiment, X²⁸ is (S)-2-amino-2-methylnon-8-enoic acid. In another class of this embodiment, X²⁸ is (S)-2-amino-2-methylhept-6-enoic acid. In another class of this embodiment, X²⁸ is (S)-2-aminohept-6-enoic acid.

In another class of this embodiment, the peptide includes a protecting group that is joined to the C-terminal carboxy group of the peptide.

In another class of this embodiment, the peptide does not include a protecting group that is joined to the C-terminal carboxy group.

In another class of this embodiment, the peptide has the amino acid sequence of SEQ ID NO: 6, 7, 8, 9, 10, 11, 12, 13, 14, 15, 16, 17, 18, or 19.

The aforementioned GCG/GLP-1 receptor co-agonist peptides have the structure as shown in Table 1.

In particular embodiments, the present invention provides a peptide consisting of the amino acid sequence
HX²QGTFTSDX¹⁰SX¹²YLDX¹⁶X¹⁷AX¹⁹X²⁰X²¹FVX²⁴WLX²⁷X²⁸TX³⁰-NH₂ (SEQ ID NO:20) wherein
X² is a-aminoisobutyric acid (Aib), or D-Serine;
X¹⁰ is Lysine, or Lysine conjugated to a fatty acid;
X¹² is (R)-2-amino-2-methyloct-7-enoic acid, or Lysine;
X¹⁶ is a-aminoisobutyric acid (Aib), or Glutamic acid;
X¹⁷ is (R)-2-amino-2-methyloct-7-enoic acid, or Arginine;
X¹⁹ is (S)-2-amino-2-methylnon-8-enoic acid, or Alanine;
X²⁰ is Glutamine, (R)-2-amino-2-methyloct-7-enoic acid, (S)-2-amino-2-methylhept-6-enoic acid, or (S)-2-aminohept-6-enoic acid;
X²¹ is Aspartic acid or (R)-2-amino-2-methyloct-7-enoic acid;
X²⁴ is Glutamine, (S)-2-amino-2-methylnon-8-enoic acid, (S)-2-amino-2-methylhept-6-enoic acid, or (S)-2-aminohept-6-enoic acid;
X²⁷ is Leucine, (S)-2-amino-2-methylnon-8-enoic acid, or Methionine;
X²⁸ is Aspartic acid, (S)-2-amino-2-methylnon-8-enoic acid, (S)-2-amino-2-methylhept-6-enoic acid, or (S)-2-aminohept-6-enoic acid ;
X³⁰ is absent, or Lysine conjugated by a gamma-glutamic acid (γE) spacer; provided that the peptide contains at least two amino acids selected from (R)-2-amino-2-methyloct-7-enoic acid, (S)-2-amino-2-methylnon-8-enoic acid, (S)-2-amino-2-methylhept-6-enoic acid, and (S)-2-aminohept-6-enoic acid, which cyclize to form a double bond containing ring; and wherein the peptide optionally includes a protecting group that, if present, is joined to the C-terminal carboxy group of the peptide; or a pharmaceutically acceptable salt thereof.

In a class of this embodiment, the two amino acids acids which cyclize to form a double bond containing ring are selected from: 1) one (R)-2-amino-2-methyloct-7-enoic acid and one (S)-2-amino-2-methylnon-8-enoic acid; 2) two (S)-2-amino-2-methylhept-6-enoic acids; and 3) two (S)-2-aminohept-6-enoic acids.

In another class of this embodiment, the two amino acids acids which cyclize to form a double bond containing ring are one (R)-2-amino-2-methyloct-7-enoic acid and one (S)-2-amino-2-methylnon-8-enoic acid.

In another class of this embodiment, the two amino acids acids which cyclize to form a double bond containing ring are two (S)-2-amino-2-methylhept-6-enoic acids.

In another class of this embodiment, the two amino acids acids which cyclize to form a double bond containing ring are two (S)-2-aminohept-6-enoic acids.

In another class of this embodiment, (R)-2-amino-2-methyloct-7-enoic acid and (S)-2-amino-2-methylnon-8-enoic acid cyclize to form a double bond containing ring; or two (S)-2-amino-2-methylhept-6-enoic acids cyclize to form a double bond containing ring; or two (S)-2-aminohept-6-enoic acids cyclize to form a double bond containing ring.

In another class of this embodiment, (R)-2-amino-2-methyloct-7-enoic acid and (S)-2-amino-2-methylnon-8-enoic acid cyclize to form a double bond containing ring.

In another class of this embodiment, two (S)-2-amino-2-methylhept-6-enoic acids cyclize to form a double bond containing ring.

In another class of this embodiment, two (S)-2-aminohept-6-enoic acids cyclize to form a double bond containing ring.

In another class of this embodiment, the alkene of one (R)-2-amino-2-methyloct-7-enoic acid and the alkene of one (S)-2-amino-2-methylnon-8-enoic acid cyclize to form a double bond containing ring; or the alkenes of two (S)-2-amino-2-methylhept-6-enoic acids cyclize to form a double bond containing ring; or the alkenes of two (S)-2-aminohept-6-enoic acids cyclize to form a double bond containing ring.

In another class of this embodiment, the alkene of one (R)-2-amino-2-methyloct-7-enoic acid and the alkene of one (S)-2-amino-2-methylnon-8-enoic acid cyclize to form a double bond containing ring.

In another class of this embodiment, the alkenes of two (S)-2-amino-2-methylhept-6-enoic acids cyclize to form a double bond containing ring.

In another class of this embodiment, the alkenes of two (S)-2-aminohept-6-enoic acids cyclize to form a double bond containing ring.

In another class of this embodiment, X¹⁰ is Lysine, or Lysine conjugated to a fatty acid via a gamma-glutamic acid - gamma-glutamic acid linker.

In another class of this embodiment, X¹⁰ is Lysine or Lysine conjugated to a C16 fatty acid via a gamma-glutamic acid - gamma-glutamic acid linker.

In another class of this embodiment, the fatty acid at position 10 comprises a C14, C16, C17, C18, C19, or C20 fatty acid. In another class of this embodiment, the fatty acid comprises a C16 or C18 fatty acid. In another class of this embodiment, the fatty acid comprises a C18 fatty acid. In another class of this embodiment, the fatty acid comprises a C16 fatty acid.

In another class of this embodiment, X¹⁰ is Lysine.

In another class of this embodiment, X¹⁰ is Lysine conjugated to a fatty acid via a gamma-glutamic acid - gamma-glutamic acid linker.

In another class of this embodiment, X¹⁰ is Lysine conjugated to a C16 or C18 fatty acid via a gamma-glutamic acid - gamma-glutamic acid linker.

In another class of this embodiment, X¹⁰ is Lysine conjugated to a C18 fatty acid via a gamma-glutamic acid - gamma-glutamic acid linker.

In another class of this embodiment, X¹⁰ is Lysine conjugated to a C16 fatty acid via a gamma-glutamic acid - gamma-glutamic acid linker.

In another class of this embodiment, X³⁰ is absent. In another class of this embodiment, X³⁰ is Lysine conjugated by a gamma-glutamic acid (γE) spacer.

In another class of this embodiment, X² is a-aminoisobutyric acid, or D-Serine. In another class of this embodiment, X² is α-aminoisobutyric acid. In another class of this embodiment, X² is D-Serine.

In another class of this embodiment, X¹² is (R)-2-amino-2-methyloct-7-enoic acid. In another class of this embodiment, X¹² is Lysine.

In another class of this embodiment, X¹⁶ is a-aminoisobutyric acid, or Glutamic acid. In another class of this embodiment, X¹⁶ is a-aminoisobutyric acid. In another class of this embodiment, X¹⁶ is Glutamic acid.

In another class of this embodiment, X¹⁷ is (R)-2-amino-2-methyloct-7-enoic acid. In another class of this embodiment, X¹⁷ is Arginine.

In another class of this embodiment, X¹⁹ is (S)-2-amino-2-methylnon-8-enoic acid. In another class of this embodiment, X¹⁹ is Alanine.

In another class of this embodiment, X²⁰ is Glutamine. In another class of this embodiment, X²⁰ is (R)-2-amino-2-methyloct-7-enoic acid. In another class of this embodiment, X²⁰ is (S)-2-amino-2-methylhept-6-enoic acid. In another class of this embodiment, X²⁰ is (S)-2-aminohept-6-enoic acid.

In another class of this embodiment, X²¹ is Aspartic acid. In another class of this embodiment, X²¹ is (R)-2-amino-2-methyloct-7-enoic acid.

In another class of this embodiment, X²⁴ is Glutamine. In another class of this embodiment, X²⁴ is (S)-2-amino-2-methylnon-8-enoic acid. In another class of this embodiment, X²⁴ is (S)-2-amino-2-methylhept-6-enoic acid. In another class of this embodiment, X²⁴ is (S)-2-aminohept-6-enoic acid.

In another class of this embodiment, X²⁷ is Leucine. In another class of this embodiment, X²⁷ is (S)-2-amino-2-methylnon-8-enoic acid. In another class of this embodiment, X²⁷ is Methionine.

In another class of this embodiment, X²⁸ is Aspartic acid. In another class of this embodiment, X²⁸ is (S)-2-amino-2-methylnon-8-enoic acid. In another class of this embodiment, X²⁸ is (S)-2-amino-2-methylhept-6-enoic acid. In another class of this embodiment, X²⁸ is (S)-2-aminohept-6-enoic acid.

In another class of this embodiment, the peptide includes a protecting group that is joined to the C-terminal carboxy group of the peptide.

In another class of this embodiment, the peptide does not include a protecting group that is joined to the C-terminal carboxy group.

In another class of this embodiment, the peptide has the amino acid sequence of SEQ ID NO: 6, 7, 8, 9, 10, 11, 12, 13, 14, 15, 16, 17, 18, or 19.

In particular embodiments, the present invention provides a peptide consisting of the amino acid sequence
HX²QGTFTSDX¹⁰SX¹²YLDX¹⁶X¹⁷AX¹⁹X²⁰X²¹FVX²⁴WLX²⁷X²⁸TX³⁰-NH₂ (SEQ ID NO:20) wherein
X² is a-aminoisobutyric acid (Aib), or D-Serine;
X¹⁰ is Lysine, or Lysine conjugated to a fatty acid via a gamma-glutamic acid - gamma-glutamic acid linker;
X¹² is (R)-2-amino-2-methyloct-7-enoic acid, or Lysine;
X¹⁶ is a-aminoisobutyric acid (Aib), or Glutamic acid;
X¹⁷ is (R)-2-amino-2-methyloct-7-enoic acid, or Arginine;
X¹⁹ is (S)-2-amino-2-methylnon-8-enoic acid, or Alanine;
X²⁰ is Glutamine, (R)-2-amino-2-methyloct-7-enoic acid, (S)-2-amino-2-methylhept-6-enoic acid, or (S)-2-aminohept-6-enoic acid;
X²¹ is Aspartic acid or (R)-2-amino-2-methyloct-7-enoic acid;
X²⁴ is Glutamine, (S)-2-amino-2-methylnon-8-enoic acid, (S)-2-amino-2-methylhept-6-enoic acid, or (S)-2-aminohept-6-enoic acid;
X²⁷ is Leucine, (S)-2-amino-2-methylnon-8-enoic acid, or Methionine;
X²⁸ is Aspartic acid, (S)-2-amino-2-methylnon-8-enoic acid, (S)-2-amino-2-methylhept-6-enoic acid, or (S)-2-aminohept-6-enoic acid;
X³⁰ is absent, or Lysine conjugated by a gamma-glutamic acid (γE) spacer; provided that the peptide contains at least two amino acids selected from (R)-2-amino-2-methyloct-7-enoic acid, (S)-2-amino-2-methylnon-8-enoic acid, (S)-2-amino-2-methylhept-6-enoic acid, and (S)-2-aminohept-6-enoic acid, which cyclize to form a double bond containing ring; and wherein the peptide optionally includes a protecting group that, if present, is joined to the C-terminal carboxy group of the peptide; or a pharmaceutically acceptable salt thereof.

In a class of this embodiment, the two amino acids acids which cyclize to form a double bond containing ring are selected from: 1) one (R)-2-amino-2-methyloct-7-enoic acid and one (S)-2-amino-2-methylnon-8-enoic acid; 2) two (S)-2-amino-2-methylhept-6-enoic acids; and 3) two (S)-2-aminohept-6-enoic acids.

In another class of this embodiment, the two amino acids acids which cyclize to form a double bond containing ring are one (R)-2-amino-2-methyloct-7-enoic acid and one (S)-2-amino-2-methylnon-8-enoic acid.

In another class of this embodiment, the two amino acids acids which cyclize to form a double bond containing ring are two (S)-2-amino-2-methylhept-6-enoic acids.

In another class of this embodiment, the two amino acids acids which cyclize to form a double bond containing ring are two (S)-2-aminohept-6-enoic acids.

In another class of this embodiment, (R)-2-amino-2-methyloct-7-enoic acid and (S)-2-amino-2-methylnon-8-enoic acid cyclize to form a double bond containing ring; or two (S)-2-amino-2-methylhept-6-enoic acids cyclize to form a double bond containing ring; or two (S)-2-aminohept-6-enoic acids cyclize to form a double bond containing ring.

In another class of this embodiment, (R)-2-amino-2-methyloct-7-enoic acid and (S)-2-amino-2-methylnon-8-enoic acid cyclize to form a double bond containing ring.

In another class of this embodiment, two (S)-2-amino-2-methylhept-6-enoic acids cyclize to form a double bond containing ring.

In another class of this embodiment, two (S)-2-aminohept-6-enoic acids cyclize to form a double bond containing ring.

In another class of this embodiment, the alkene of one (R)-2-amino-2-methyloct-7-enoic acid and the alkene of one (S)-2-amino-2-methylnon-8-enoic acid cyclize to form a double bond containing ring; or the alkenes of two (S)-2-amino-2-methylhept-6-enoic acids cyclize to form a double bond containing ring; or the alkenes of two (S)-2-aminohept-6-enoic acids cyclize to form a double bond containing ring.

In another class of this embodiment, the alkene of one (R)-2-amino-2-methyloct-7-enoic acid and the alkene of one (S)-2-amino-2-methylnon-8-enoic acid cyclize to form a double bond containing ring.

In another class of this embodiment, the alkenes of two (S)-2-amino-2-methylhept-6-enoic acids cyclize to form a double bond containing ring.

In another class of this embodiment, the alkenes of two (S)-2-aminohept-6-enoic acids cyclize to form a double bond containing ring.

In a class of this embodiment, the fatty acid at position 10 comprises a C14, C16, C17, C18, C19, or C20 fatty acid. In another class of this embodiment, the fatty acid comprises a C16 or C18 fatty acid. In another class of this embodiment, the fatty acid comprises a C18 fatty acid. In another class of this embodiment, the fatty acid comprises a C16 fatty acid.

In another class of this embodiment, X¹⁰ is Lysine or Lysine conjugated to a C16 fatty acid via a gamma-glutamic acid - gamma-glutamic acid linker.

In another class of this embodiment, X¹⁰ is Lysine.

In another class of this embodiment, X¹⁰ is Lysine conjugated to a fatty acid via a gamma-glutamic acid - gamma-glutamic acid linker.

In another class of this embodiment, X¹⁰ is Lysine conjugated to a C16 or C18 fatty acid via a gamma-glutamic acid - gamma-glutamic acid linker.

In another class of this embodiment, X¹⁰ is Lysine conjugated to a C18 fatty acid via a gamma-glutamic acid - gamma-glutamic acid linker.

In another class of this embodiment, X¹⁰ is Lysine conjugated to a C16 fatty acid via a gamma-glutamic acid - gamma-glutamic acid linker.

In another class of this embodiment, X³⁰ is absent. In another class of this embodiment, X³⁰ is Lysine conjugated by a gamma-glutamic acid (γE) spacer.

In another class of this embodiment, X² is a-aminoisobutyric acid, or D-Serine. In another class of this embodiment, X² is α-aminoisobutyric acid. In another class of this embodiment, X² is D-Serine.

In another class of this embodiment, X¹² is (R)-2-amino-2-methyloct-7-enoic acid. In another class of this embodiment, X¹² is Lysine.

In another class of this embodiment, X¹⁶ is α-aminoisobutyric acid, or Glutamic acid. In another class of this embodiment, X¹⁶ is a-aminoisobutyric acid. In another class of this embodiment, X¹⁶ is Glutamic acid.

In another class of this embodiment, X¹⁷ is (R)-2-amino-2-methyloct-7-enoic acid. In another class of this embodiment, X¹⁷ is Arginine.

In another class of this embodiment, X¹⁹ is (S)-2-amino-2-methylnon-8-enoic acid. In another class of this embodiment, X¹⁹ is Alanine.

In another class of this embodiment, X²⁰ is Glutamine. In another class of this embodiment, X²⁰ is (R)-2-amino-2-methyloct-7-enoic acid. In another class of this embodiment, X²⁰ is (S)-2-amino-2-methylhept-6-enoic acid. In another class of this embodiment, X²⁰ is (S)-2-aminohept-6-enoic acid.

In another class of this embodiment, X²¹ is Aspartic acid. In another class of this embodiment, X²¹ is (R)-2-amino-2-methyloct-7-enoic acid.

In another class of this embodiment, X²⁴ is Glutamine. In another class of this embodiment, X²⁴ is (S)-2-amino-2-methylnon-8-enoic acid. In another class of this embodiment, X²⁴ is (S)-2-amino-2-methylhept-6-enoic acid. In another class of this embodiment, X²⁴ is (S)-2-aminohept-6-enoic acid.

In another class of this embodiment, X²⁷ is Leucine. In another class of this embodiment, X²⁷ is (S)-2-amino-2-methylnon-8-enoic acid. In another class of this embodiment, X²⁷ is Methionine.

In another class of this embodiment, X²⁸ is Aspartic acid. In another class of this embodiment, X²⁸ is (S)-2-amino-2-methylnon-8-enoic acid. In another class of this embodiment, X²⁸ is (S)-2-amino-2-methylhept-6-enoic acid. In another class of this embodiment, X²⁸ is (S)-2-aminohept-6-enoic acid.

In another class of this embodiment, the peptide includes a protecting group that is joined to the C-terminal carboxy group of the peptide.

In another class of this embodiment, the peptide does not include a protecting group that is joined to the C-terminal carboxy group.

The present invention further provides a pharmaceutical composition comprising a peptide having the amino acid sequence
HX²QGTFTSDX¹⁰SX¹²YLDX¹⁶X¹⁷AX¹⁹X²⁰X²¹FVX²⁴WLX²⁷X²⁸TX³⁰-NH₂ (SEQ ID NO:20) wherein
X² is α-aminoisobutyric acid (Aib), or D-Serine;
X¹⁰ is Lysine, Lysine conjugated to a fatty acid, or Lysine conjugated to a fatty diacid; X¹² is (R)-2-amino-2-methyloct-7-enoic acid, or Lysine;
X¹⁶ is a-aminoisobutyric acid (Aib), or Glutamic acid;
X¹⁷ is (R)-2-amino-2-methyloct-7-enoic acid, or Arginine;
X¹⁹ is (S)-2-amino-2-methylnon-8-enoic acid, or Alanine;
X²⁰ is Glutamine, (R)-2-amino-2-methyloct-7-enoic acid, (S)-2-amino-2-methylhept-6-enoic acid, or (S)-2-aminohept-6-enoic acid;
X²¹ is Aspartic acid or (R)-2-amino-2-methyloct-7-enoic acid;
X²⁴ is Glutamine, (S)-2-amino-2-methylnon-8-enoic acid, (S)-2-amino-2-methylhept-6-enoic acid, or (S)-2-aminohept-6-enoic acid;
X²⁷ is Leucine, (S)-2-amino-2-methylnon-8-enoic acid, or Methionine;
X²⁸ is Aspartic acid, (S)-2-amino-2-methylnon-8-enoic acid, (S)-2-amino-2-methylhept-6-enoic acid, or (S)-2-aminohept-6-enoic acid;
X³⁰ is absent, or Lysine conjugated by a gamma-glutamic acid (γE) spacer;
provided that the peptide contains at least two amino acids selected from (R)-2-amino-2-methyloct-7-enoic acid, (S)-2-amino-2-methylnon-8-enoic acid, (S)-2-amino-2-methylhept-6-enoic acid, and (S)-2-aminohept-6-enoic acid, which cyclize to form a double bond containing ring; and wherein the peptide optionally includes a protecting group that, if present, is joined to the C-terminal carboxy group of the peptide; or a pharmaceutically acceptable salt thereof.

In a class of this embodiment, the two amino acids acids which cyclize to form a double bond containing ring are selected from: 1) one (R)-2-amino-2-methyloct-7-enoic acid and one (S)-2-amino-2-methylnon-8-enoic acid; 2) two (S)-2-amino-2-methylhept-6-enoic acids; and 3) two (S)-2-aminohept-6-enoic acids.

In another class of this embodiment, the two amino acids acids which cyclize to form a double bond containing ring are one (R)-2-amino-2-methyloct-7-enoic acid and one (S)-2-amino-2-methylnon-8-enoic acid.

In another class of this embodiment, the two amino acids acids which cyclize to form a double bond containing ring are two (S)-2-amino-2-methylhept-6-enoic acids.

In another class of this embodiment, the two amino acids acids which cyclize to form a double bond containing ring are two (S)-2-aminohept-6-enoic acids.

In another class of this embodiment, (R)-2-amino-2-methyloct-7-enoic acid and (S)-2-amino-2-methylnon-8-enoic acid cyclize to form a double bond containing ring; or two (S)-2-amino-2-methylhept-6-enoic acids cyclize to form a double bond containing ring; or two (S)-2-aminohept-6-enoic acids cyclize to form a double bond containing ring.

In another class of this embodiment, (R)-2-amino-2-methyloct-7-enoic acid and (S)-2-amino-2-methylnon-8-enoic acid cyclize to form a double bond containing ring.

In another class of this embodiment, two (S)-2-amino-2-methylhept-6-enoic acids cyclize to form a double bond containing ring.

In another class of this embodiment, two (S)-2-aminohept-6-enoic acids cyclize to form a double bond containing ring.

In another class of this embodiment, the alkene of one (R)-2-amino-2-methyloct-7-enoic acid and the alkene of one (S)-2-amino-2-methylnon-8-enoic acid cyclize to form a double bond containing ring; or the alkenes of two (S)-2-amino-2-methylhept-6-enoic acids cyclize to form a double bond containing ring; or the alkenes of two (S)-2-aminohept-6-enoic acids cyclize to form a double bond containing ring.

In another class of this embodiment, the alkene of one (R)-2-amino-2-methyloct-7-enoic acid and the alkene of one (S)-2-amino-2-methylnon-8-enoic acid cyclize to form a double bond containing ring.

In another class of this embodiment, the alkenes of two (S)-2-amino-2-methylhept-6-enoic acids cyclize to form a double bond containing ring.

In another class of this embodiment, the alkenes of two (S)-2-aminohept-6-enoic acids cyclize to form a double bond containing ring.

In another class of this embodiment, X¹⁰ is Lysine, Lysine conjugated to a fatty acid by a gamma-glutamic acid - gamma-glutamic acid linker, or Lysine conjugated to a fatty diacid via a gamma-glutamic acid - gamma-glutamic acid linker.

In another class of this embodiment, the fatty diacid at position 10 comprises a C14, C15, C16, C17, C18, C19, or C20 fatty diacid, and the fatty acid at position 10 comprises a C14, C16, C17, C18, C19, or C20 fatty acid.

In another class of this embodiment, the fatty diacid at position 10 comprises a C14, C15, C16, C17, C18, C19, or C20 fatty diacid. In another class of this embodiment, the fatty diacid comprises a C16 or C18 fatty diacid. In another class of this embodiment, the fatty diacid comprises a C18 fatty diacid. In another class of this embodiment, the fatty diacid comprises a C16 fatty diacid.

In another class of this embodiment, the fatty acid at position 10 comprises a C14, C16, C17, C18, C19, or C20 fatty acid. In another class of this embodiment, the fatty acid comprises a C1 6 or C18 fatty acid. In another class of this embodiment, the fatty acid comprises a C18 fatty acid. In another class of this embodiment, the fatty acid comprises a C16 fatty acid.

In another class of this embodiment, X¹⁰ is Lysine, or Lysine conjugated to a fatty acid.

In another class of this embodiment, X¹⁰ is Lysine, or Lysine conjugated to a fatty acid via a gamma-glutamic acid - gamma-glutamic acid linker.

In another class of this embodiment, X¹⁰ is Lysine or Lysine conjugated to a C16 fatty acid via a gamma-glutamic acid - gamma-glutamic acid linker.

In another class of this embodiment, X¹⁰ is Lysine.

In another class of this embodiment, X¹⁰ is Lysine conjugated to a fatty acid via a gamma-glutamic acid - gamma-glutamic acid linker.

In another class of this embodiment, X¹⁰ is Lysine conjugated to a C16 or C18 fatty acid via a gamma-glutamic acid - gamma-glutamic acid linker.

In another class of this embodiment, X¹⁰ is Lysine conjugated to a C18 fatty acid via a gamma-glutamic acid - gamma-glutamic acid linker.

In another class of this embodiment, X¹⁰ is Lysine conjugated to a C16 fatty acid via a gamma-glutamic acid - gamma-glutamic acid linker.

In another class of this embodiment, the peptide comprises at X¹⁰ a Lysine or a Lysine conjugated to a fatty diacid via a gamma-glutamic acid - gamma-glutamic acid linker.

In another class of this embodiment, the peptide comprises at X¹⁰ a Lysine, or a Lysine conjugated to a C16 or C18 fatty diacid via a gamma-glutamic acid - gamma-glutamic acid linker.

In another class of this embodiment, the peptide comprises at X¹⁰ a Lys conjugated to a C18 fatty diacid via a gamma-glutamic acid - gamma-glutamic acid linker.

In another class of this embodiment, the peptide comprises at X¹⁰ a Lys conjugated to a C16 fatty diacid via a gamma-glutamic acid - gamma-glutamic acid linker.

In another class of this embodiment, X³⁰ is absent. In another class of this embodiment, X³⁰ is Lysine conjugated by a gamma-glutamic acid (γE) spacer.

In another class of this embodiment, X² is a-aminoisobutyric acid, or D-Serine. In another class of this embodiment, X² is a-aminoisobutyric acid. In another class of this embodiment, X² is D-Serine.

In another class of this embodiment, X¹² is (R)-2-amino-2-methyloct-7-enoic acid. In another class of this embodiment, X¹² is Lysine.

In another class of this embodiment, X¹⁶ is a-aminoisobutyric acid, or Glutamic acid. In another class of this embodiment, X¹⁶ is a-aminoisobutyric acid. In another class of this embodiment, X¹⁶ is Glutamic acid.

In another class of this embodiment, X¹⁷ is (R)-2-amino-2-methyloct-7-enoic acid. In another class of this embodiment, X¹⁷ is Arginine.

In another class of this embodiment, X¹⁹ is (S)-2-amino-2-methylnon-8-enoic acid. In another class of this embodiment, X¹⁹ is Alanine.

In another class of this embodiment, X²⁰ is Glutamine. In another class of this embodiment, X²⁰ is (R)-2-amino-2-methyloct-7-enoic acid. In another class of this embodiment, X²⁰ is (S)-2-amino-2-methylhept-6-enoic acid. In another class of this embodiment, X²⁰ is (S)-2-aminohept-6-enoic acid.

In another class of this embodiment, X²¹ is Aspartic acid. In another class of this embodiment, X²¹ is (R)-2-amino-2-methyloct-7-enoic acid.

In another class of this embodiment, X²⁴ is Glutamine. In another class of this embodiment, X²⁴ is (S)-2-amino-2-methylnon-8-enoic acid. In another class of this embodiment, X²⁴ is (S)-2-amino-2-methylhept-6-enoic acid. In another class of this embodiment, X²⁴ is (S)-2-aminohept-6-enoic acid.

In another class of this embodiment, X²⁷ is Leucine. In another class of this embodiment, X²⁷ is (S)-2-amino-2-methylnon-8-enoic acid. In another class of this embodiment, X²⁷ is Methionine.

In another class of this embodiment, X²⁸ is Aspartic acid. In another class of this embodiment, X²⁸ is (S)-2-amino-2-methylnon-8-enoic acid. In another class of this embodiment, X²⁸ is (S)-2-amino-2-methylhept-6-enoic acid. In another class of this embodiment, X²⁸ is (S)-2-aminohept-6-enoic acid.

In another class of this embodiment, the peptide includes a protecting group that is joined to the C-terminal carboxy group of the peptide.

In another class of this embodiment, the peptide does not include a protecting group that is joined to the C-terminal carboxy group.

In another class of this embodiment, the peptide has the amino acid sequence of SEQ ID NO: 6, 7, 8, 9, 10, 11, 12, 13, 14, 15, 16, 17, 18, or 19.

The present invention further provides a pharmaceutical composition comprising a peptide having the amino acid sequence
HX²QGTFTSDX¹⁰SX¹²YLDX¹⁶X¹⁷AX¹⁹X²⁰X²¹FVX²⁴WLX²⁷X²⁸TX³⁰-NH₂ (SEQ ID NO:20) wherein
X² is a-aminoisobutyric acid (Aib), or D-Serine;
X¹⁰ is Lysine, or Lysine conjugated to a fatty acid;
X¹² is (R)-2-amino-2-methyloct-7-enoic acid, or Lysine;
X¹⁶ is a-aminoisobutyric acid (Aib), or Glutamic acid;
X¹⁷ is (R)-2-amino-2-methyloct-7-enoic acid, or Arginine;
X¹⁹ is (S)-2-amino-2-methylnon-8-enoic acid, or Alanine;
X²⁰ is Glutamine, (R)-2-amino-2-methyloct-7-enoic acid, (S)-2-amino-2-methylhept-6-enoic acid, or (S)-2-aminohept-6-enoic acid;
X²¹ is Aspartic acid or (R)-2-amino-2-methyloct-7-enoic acid;
X²⁴ is Glutamine, (S)-2-amino-2-methylnon-8-enoic acid, (S)-2-amino-2-methylhept-6-enoic acid, or (S)-2-aminohept-6-enoic acid;
X²⁷ is Leucine, (S)-2-amino-2-methylnon-8-enoic acid, or Methionine;
X²⁸ is Aspartic acid, (S)-2-amino-2-methylnon-8-enoic acid, (S)-2-amino-2-methylhept-6-enoic acid, or (S)-2-aminohept-6-enoic acid;
X³⁰ is absent, or Lysine conjugated by a gamma-glutamic acid (γE) spacer;
provided that the peptide contains at least two amino acids selected from (R)-2-amino-2-methyloct-7-enoic acid, (S)-2-amino-2-methylnon-8-enoic acid, (S)-2-amino-2-methylhept-6-enoic acid, and (S)-2-aminohept-6-enoic acid, which cyclize to form a double bond containing ring; and wherein the peptide optionally includes a protecting group that, if present, is joined to the C-terminal carboxy group of the peptide; or a pharmaceutically acceptable salt thereof.

In a class of this embodiment, the two amino acids acids which cyclize to form a double bond containing ring are selected from: 1) one (R)-2-amino-2-methyloct-7-enoic acid and one (S)-2-amino-2-methylnon-8-enoic acid; 2) two (S)-2-amino-2-methylhept-6-enoic acids; and 3) two (S)-2-aminohept-6-enoic acids.

In another class of this embodiment, the two amino acids acids which cyclize to form a double bond containing ring are one (R)-2-amino-2-methyloct-7-enoic acid and one (S)-2-amino-2-methylnon-8-enoic acid.

In another class of this embodiment, the two amino acids acids which cyclize to form a double bond containing ring are two (S)-2-amino-2-methylhept-6-enoic acids.

In another class of this embodiment, the two amino acids acids which cyclize to form a double bond containing ring are two (S)-2-aminohept-6-enoic acids.

In another class of this embodiment, (R)-2-amino-2-methyloct-7-enoic acid and (S)-2-amino-2-methylnon-8-enoic acid cyclize to form a double bond containing ring; or two (S)-2-amino-2-methylhept-6-enoic acids cyclize to form a double bond containing ring; or two (S)-2-aminohept-6-enoic acids cyclize to form a double bond containing ring.

In another class of this embodiment, (R)-2-amino-2-methyloct-7-enoic acid and (S)-2-amino-2-methylnon-8-enoic acid cyclize to form a double bond containing ring.

In another class of this embodiment, two (S)-2-amino-2-methylhept-6-enoic acids cyclize to form a double bond containing ring.

In another class of this embodiment, two (S)-2-aminohept-6-enoic acids cyclize to form a double bond containing ring.

In another class of this embodiment, the alkene of one (R)-2-amino-2-methyloct-7-enoic acid and the alkene of one (S)-2-amino-2-methylnon-8-enoic acid cyclize to form a double bond containing ring; or the alkenes of two (S)-2-amino-2-methylhept-6-enoic acids cyclize to form a double bond containing ring; or the alkenes of two (S)-2-aminohept-6-enoic acids cyclize to form a double bond containing ring.

In another class of this embodiment, the alkene of one (R)-2-amino-2-methyloct-7-enoic acid and the alkene of one (S)-2-amino-2-methylnon-8-enoic acid cyclize to form a double bond containing ring.

In another class of this embodiment, the alkenes of two (S)-2-amino-2-methylhept-6-enoic acids cyclize to form a double bond containing ring.

In another class of this embodiment, the alkenes of (S)-2-aminohept-6-enoic acids cyclize to form a double bond containing ring.

In another class of this embodiment, X¹⁰ is Lysine, or Lysine conjugated to a fatty acid via a gamma-glutamic acid - gamma-glutamic acid linker.

In another class of this embodiment, X¹⁰ is Lysine or Lysine conjugated to a C16 fatty acid via a gamma-glutamic acid - gamma-glutamic acid linker.

In another class of this embodiment, the fatty acid at position 10 comprises a C14, C16, C17, C18, C19, or C20 fatty acid. In another class of this embodiment, the fatty acid comprises a C16 or C18 fatty acid. In another class of this embodiment, the fatty acid comprises a C18 fatty acid. In another class of this embodiment, the fatty acid comprises a C16 fatty acid.

In another class of this embodiment, X¹⁰ is Lysine.

In another class of this embodiment, X¹⁰ is Lysine conjugated to a fatty acid via a gamma-glutamic acid - gamma-glutamic acid linker.

In another class of this embodiment, X¹⁰ is Lysine conjugated to a C16 or C18 fatty acid via a gamma-glutamic acid - gamma-glutamic acid linker.

In another class of this embodiment, X¹⁰ is Lysine conjugated to a C18 fatty acid via a gamma-glutamic acid - gamma-glutamic acid linker.

In another class of this embodiment, X¹⁰ is Lysine conjugated to a C16 fatty acid via a gamma-glutamic acid - gamma-glutamic acid linker.

In another class of this embodiment, X³⁰ is absent. In another class of this embodiment, X³⁰ is Lysine conjugated by a gamma-glutamic acid (γE) spacer.

In another class of this embodiment, X² is a-aminoisobutyric acid, or D-Serine. In another class of this embodiment, X² is a-aminoisobutyric acid. In another class of this embodiment, X² is D-Serine.

In another class of this embodiment, X¹² is (R)-2-amino-2-methyloct-7-enoic acid. In another class of this embodiment, X¹² is Lysine.

In another class of this embodiment, X¹⁶ is α-aminoisobutyric acid, or Glutamic acid. In another class of this embodiment, X¹⁶ is a-aminoisobutyric acid. In another class of this embodiment, X¹⁶ is Glutamic acid.

In another class of this embodiment, X¹⁷ is (R)-2-amino-2-methyloct-7-enoic acid. In another class of this embodiment, X¹⁷ is Arginine.

In another class of this embodiment, X¹⁹ is (S)-2-amino-2-methylnon-8-enoic acid. In another class of this embodiment, X ¹⁹ is Alanine.

In another class of this embodiment, X²⁰ is Glutamine. In another class of this embodiment, X²⁰ is (R)-2-amino-2-methyloct-7-enoic acid. In another class of this embodiment, X²⁰ is (S)-2-amino-2-methylhept-6-enoic acid. In another class of this embodiment, X²⁰ is (S)-2-aminohept-6-enoic acid.

In another class of this embodiment, X²¹ is Aspartic acid. In another class of this embodiment, X²¹ is (R)-2-amino-2-methyloct-7-enoic acid.

In another class of this embodiment, X²⁴ is Glutamine. In another class of this embodiment, X²⁴ is (S)-2-amino-2-methylnon-8-enoic acid. In another class of this embodiment, X²⁴ is (S)-2-amino-2-methylhept-6-enoic acid. In another class of this embodiment, X²⁴ is (S)-2-aminohept-6-enoic acid.

In another class of this embodiment, X²⁷ is Leucine. In another class of this embodiment, X²⁷ is (S)-2-amino-2-methylnon-8-enoic acid. In another class of this embodiment, X²⁷ is Methionine.

In another class of this embodiment, X²⁸ is Aspartic acid. In another class of this embodiment, X²⁸ is (S)-2-amino-2-methylnon-8-enoic acid. In another class of this embodiment, X²⁸ is (S)-2-amino-2-methylhept-6-enoic acid. In another class of this embodiment, X²⁸ is (S)-2-aminohept-6-enoic acid.

In another class of this embodiment, the peptide includes a protecting group that is joined to the C-terminal carboxy group of the peptide.

In another class of this embodiment, the peptide does not include a protecting group that is joined to the C-terminal carboxy group.

In another class of this embodiment, the peptide has the amino acid sequence of SEQ ID NO: 6, 7, 8, 9, 10, 11, 12, 13, 14, 15, 16, 17, 18, or 19.

The present invention further provides a pharmaceutical composition comprising a peptide having the amino acid sequence
HX²QGTFTSDX¹⁰SX¹²YLDX¹⁶X¹⁷AX¹⁹X²⁰X²¹FVX²⁴WLX²⁷X²⁸TX³⁰-NH₂ (SEQ ID NO:20) wherein
X² is a-aminoisobutyric acid (Aib), or D-Serine;
X¹⁰ is Lysine, or Lysine conjugated to a fatty acid via a gamma-glutamic acid - gamma-glutamic acid linker;
X¹² is (R)-2-amino-2-methyloct-7-enoic acid, or Lysine;
X¹⁶ is α-aminoisobutyric acid (Aib), or Glutamic acid;
X¹⁷ is (R)-2-amino-2-methyloct-7-enoic acid, or Arginine;
X¹⁹ is (S)-2-amino-2-methylnon-8-enoic acid, or Alanine;
X²⁰ is Glutamine, (R)-2-amino-2-methyloct-7-enoic acid, (S)-2-amino-2-methylhept-6-enoic acid, or (S)-2-aminohept-6-enoic acid;
X²¹ is Aspartic acid or (R)-2-amino-2-methyloct-7-enoic acid;
X²⁴ is Glutamine, (S)-2-amino-2-methylnon-8-enoic acid, (S)-2-amino-2-methylhept-6-enoic acid, or (S)-2-aminohept-6-enoic acid;
X²⁷ is Leucine, (S)-2-amino-2-methylnon-8-enoic acid, or Methionine;
X²⁸ is Aspartic acid, (S)-2-amino-2-methylnon-8-enoic acid, (S)-2-amino-2-methylhept-6-enoic acid, or (S)-2-aminohept-6-enoic acid;
X³⁰ is absent, or Lysine conjugated by a gamma-glutamic acid (γE) spacer;
provided that the peptide contains at least two amino acids selected from (R)-2-amino-2-methyloct-7-enoic acid, (S)-2-amino-2-methylnon-8-enoic acid, (S)-2-amino-2-methylhept-6-enoic acid, and (S)-2-aminohept-6-enoic acid, which cyclize to form a double bond containing ring; and wherein the peptide optionally includes a protecting group that, if present, is joined to the C-terminal carboxy group of the peptide; or a pharmaceutically acceptable salt thereof.

In a class of this embodiment, the two amino acids acids which cyclize to form a double bond containing ring are selected from: 1) one (R)-2-amino-2-methyloct-7-enoic acid and one (S)-2-amino-2-methylnon-8-enoic acid; 2) two (S)-2-amino-2-methylhept-6-enoic acids; and 3) two (S)-2-aminohept-6-enoic acids.

In another class of this embodiment, the two amino acids acids which cyclize to form a double bond containing ring are one (R)-2-amino-2-methyloct-7-enoic acid and one (S)-2-amino-2-methylnon-8-enoic acid.

In another class of this embodiment, the two amino acids acids which cyclize to form a double bond containing ring are two (S)-2-amino-2-methylhept-6-enoic acids.

In another class of this embodiment, the two amino acids acids which cyclize to form a double bond containing ring are two (S)-2-aminohept-6-enoic acids.

In another class of this embodiment, (R)-2-amino-2-methyloct-7-enoic acid and (S)-2-amino-2-methylnon-8-enoic acid cyclize to form a double bond containing ring; or two (S)-2-amino-2-methylhept-6-enoic acids cyclize to form a double bond containing ring; or two (S)-2-aminohept-6-enoic acids cyclize to form a double bond containing ring.

In another class of this embodiment, (R)-2-amino-2-methyloct-7-enoic acid and (S)-2-amino-2-methylnon-8-enoic acid cyclize to form a double bond containing ring.

In another class of this embodiment, two (S)-2-amino-2-methylhept-6-enoic acids cyclize to form a double bond containing ring.

In another class of this embodiment, two (S)-2-aminohept-6-enoic acids cyclize to form a double bond containing ring.

In another class of this embodiment, the alkene of one (R)-2-amino-2-methyloct-7-enoic acid and the alkene of one (S)-2-amino-2-methylnon-8-enoic acid cyclize to form a double bond containing ring; or the alkenes of two (S)-2-amino-2-methylhept-6-enoic acids cyclize to form a double bond containing ring; or the alkenes of two (S)-2-aminohept-6-enoic acids cyclize to form a double bond containing ring.

In another class of this embodiment, the alkene of one (R)-2-amino-2-methyloct-7-enoic acid and the alkene of one (S)-2-amino-2-methylnon-8-enoic acid cyclize to form a double bond containing ring.

In another class of this embodiment, the alkenes of two (S)-2-amino-2-methylhept-6-enoic acids cyclize to form a double bond containing ring.

In another class of this embodiment, the alkenes of two (S)-2-aminohept-6-enoic acids cyclize to form a double bond containing ring.

In a class of this embodiment, the fatty acid at position 10 comprises a C14, C16, C17, C18, C19, or C20 fatty acid. In another class of this embodiment, the fatty acid comprises a C16 or C18 fatty acid. In another class of this embodiment, the fatty acid comprises a C18 fatty acid. In another class of this embodiment, the fatty acid comprises a C16 fatty acid.

In another class of this embodiment, X¹⁰ is Lysine or Lysine conjugated to a C16 fatty acid via a gamma-glutamic acid - gamma-glutamic acid linker.

In another class of this embodiment, X¹⁰ is Lysine.

In another class of this embodiment, X¹⁰ is Lysine conjugated to a fatty acid via a gamma-glutamic acid - gamma-glutamic acid linker.

In another class of this embodiment, X¹⁰ is Lysine conjugated to a C16 or C18 fatty acid via a gamma-glutamic acid - gamma-glutamic acid linker.

In another class of this embodiment, X¹⁰ is Lysine conjugated to a C18 fatty acid via a gamma-glutamic acid - gamma-glutamic acid linker.

In another class of this embodiment, X¹⁰ is Lysine conjugated to a C16 fatty acid via a gamma-glutamic acid - gamma-glutamic acid linker.

In another class of this embodiment, X³⁰ is absent. In another class of this embodiment, X³⁰ is Lysine conjugated by a gamma-glutamic acid (γE) spacer.

In another class of this embodiment, X² is α-aminoisobutyric acid, or D-Serine. In another class of this embodiment, X² is α-aminoisobutyric acid. In another class of this embodiment, X² is D-Serine.

In another class of this embodiment, X¹² is (R)-2-amino-2-methyloct-7-enoic acid. In another class of this embodiment, X¹² is Lysine.

In another class of this embodiment, X¹⁶ is a-aminoisobutyric acid, or Glutamic acid. In another class of this embodiment, X¹⁶ is α-aminoisobutyric acid. In another class of this embodiment, X¹⁶ is Glutamic acid.

In another class of this embodiment, X¹⁷ is (R)-2-amino-2-methyloct-7-enoic acid. In another class of this embodiment, X¹⁷ is Arginine.

In another class of this embodiment, X¹⁹ is (S)-2-amino-2-methylnon-8-enoic acid. In another class of this embodiment, X¹⁹ is Alanine.

In another class of this embodiment, X²⁰ is Glutamine. In another class of this embodiment, X²⁰ is (R)-2-amino-2-methyloct-7-enoic acid. In another class of this embodiment, X²⁰ is (S)-2-amino-2-methylhept-6-enoic acid. In another class of this embodiment, X²⁰ is (S)-2-aminohept-6-enoic acid.

In another class of this embodiment, X²¹ is Aspartic acid. In another class of this embodiment, X²¹ is (R)-2-amino-2-methyloct-7-enoic acid.

In another class of this embodiment, X²⁴ is Glutamine. In another class of this embodiment, X²⁴ is (S)-2-amino-2-methylnon-8-enoic acid. In another class of this embodiment, X²⁴ is (S)-2-amino-2-methylhept-6-enoic acid. In another class of this embodiment, X²⁴ is (S)-2-aminohept-6-enoic acid.

In another class of this embodiment, X²⁷ is Leucine. In another class of this embodiment, X²⁷ is (S)-2-amino-2-methylnon-8-enoic acid. In another class of this embodiment, X²⁷ is Methionine.

In another class of this embodiment, X²⁸ is Aspartic acid. In another class of this embodiment, X²⁸ is (S)-2-amino-2-methylnon-8-enoic acid. In another class of this embodiment, X²⁸ is (S)-2-amino-2-methylhept-6-enoic acid. In another class of this embodiment, X²⁸ is (S)-2-aminohept-6-enoic acid.

In another class of this embodiment, the peptide includes a protecting group that is joined to the C-terminal carboxy group of the peptide.

In another class of this embodiment, the peptide does not include a protecting group that is joined to the C-terminal carboxy group.

Conjugates, fusion proteins and multimers of any of the peptide sequences disclosed herein are also contemplated.

Examples of peptides of the present invention are shown in **Table 1.**

| SEQ ID. NO. | Peptide Name | SEQUENCE |
|---|---|---|
| 6 | TP397 | 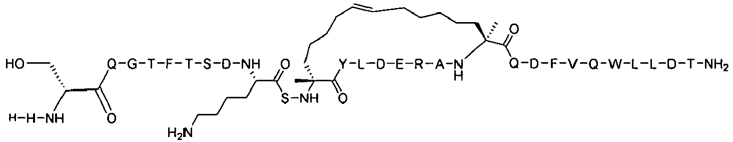 |
| | | HsQGTFTSDKS^{∗}R₆ₘₑYLDERA^{∗}S₇ₘₑQDFVQWLLDT-NH₂ |
| 7 | TP397 | 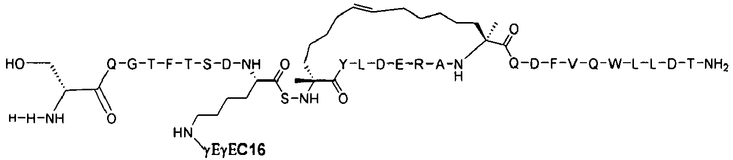 |
| | | HsQGTFTSDK(γEγEC16)S^{∗}R₆ₘₑYLDERA^{∗}S₇ₘₑDFVQWLLDT-NH₂ |
| 8 | TP398 | 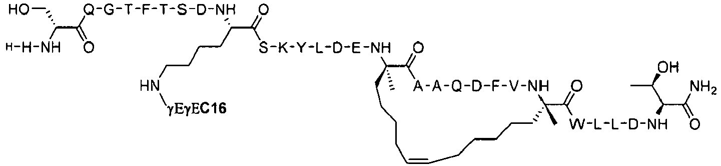 |
| | | HsQGTFTSDK(γEγEC16)SKYLDE^{∗}R₆ₘₑAAQDFV^{∗}S₇ₘₑWLLDT-NH₂ |
| 9 | TP398 | 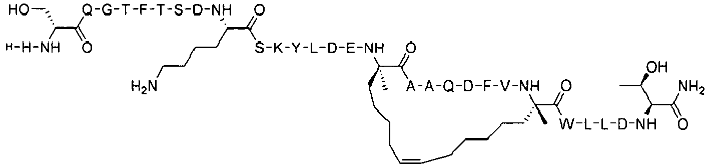 |
| | | HsQGTFTSDKSKYLDE^{∗}R₆ₘₑAAQDFV^{∗}S₇ₘₑWLLDT-NH₂ |
| 10 | TP399 | 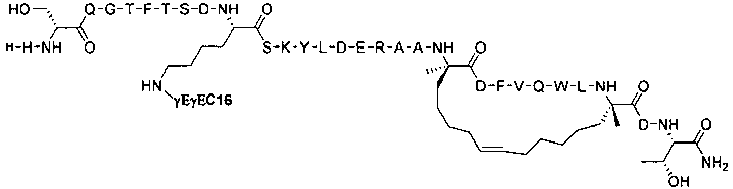 |
| | | HsQGTFTSDK(γEγEC 16)SKYLDERAA^{∗}R₆ₘₑDFVQWL^{∗}S₇ₘₑDT-NH₂ |
| 11 | TP 399 | 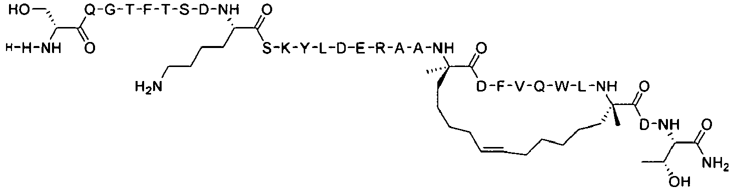 |
| | | HsQGTFTSDKSKYLDERAA^{∗}R₆ₘₑDFVQWL^{∗}S₇ₘₑDT-NH₂ |
| 12 | TP400 | 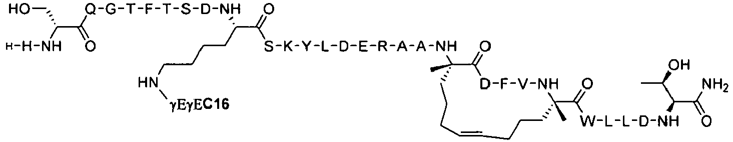 |
| | | HsQGTFTSDK(γEγEC16)SKYLDERAA*S₅ₘₑDFV*S₅ₘₑWLLDT-NH2 |
| 13 | TP401 | 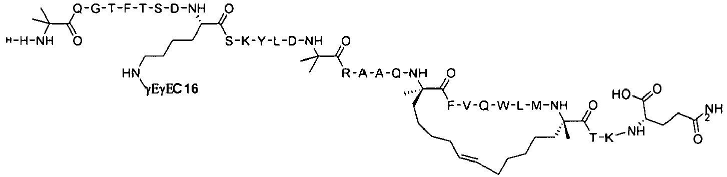 |
| | | HUQGTFTSDK(γEγEC 16)SKYLDURAAQ^{∗}R₆ₘₑFVQWLM^{∗}S₇ₘₑTKγE-NH₂ |
| 14 | TP402 | 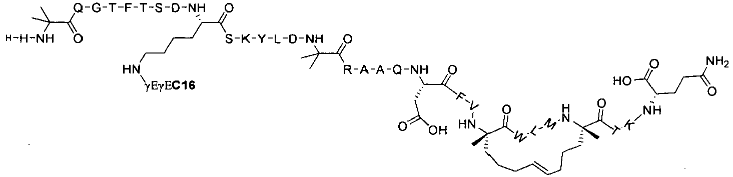 |
| | | HUQGTFTSDK(γEγEC16)SKYLDURAAQDFV^{∗}S₅ₘₑWLM^{∗}₅ₘₑTKγE-NH₂ |
| 15 | TP506 | 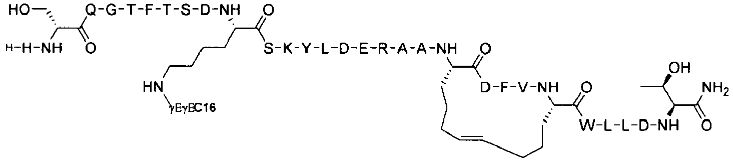 |
| | | HsQGTFTSDK(γEγEC16)SKYLDERAA^{∗}S_{5H}DFV^{∗}S_{5H}WLLDT-NH₂ |
| 16 | TP 551 | 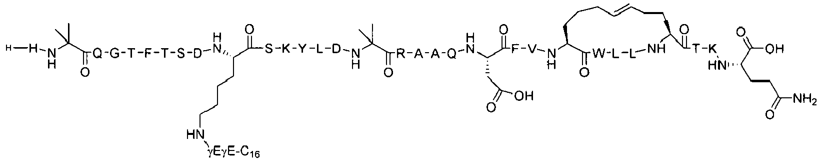 |
| | | HUQGTFTSDK(γEγEC16)SKYLDURAAQDFV^{∗}S_{5H}WLL^{∗}S_{5H}TKγE-NH₂ |
| 17 | TP 556 | 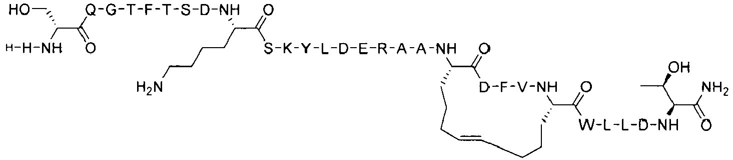 |
| | | HsQGTFTSDKSKYLDERAA^{∗}S_{5H}DFV^{∗}S_{5H}WLLDT-NH₂ |
| 18 | TP 557 | 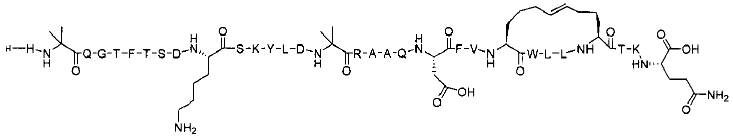 |
| | | HUQGTFTSDKSKYLDURAAQDFV*S_{5H}WLL*S_{5H}TKγE-NH₂ |
| 19 | TP 558 | 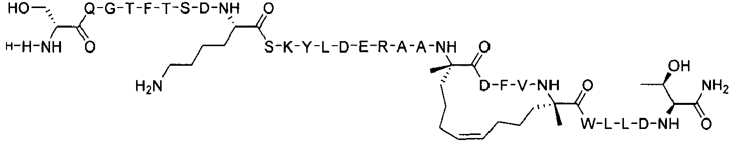 |
| | | HsQGTFTSDKSKYLDERAA*S₅ₘₑDFV*S₅ₘₑWLLDT-NH₂ |
| | | Table legend: U= alpha-aminoisobutyric acid (Aib); γE= γ-glutamic acid; γE γE = γ-glutamic acid-γ-glutamic acid; s=D-Serine; C16 = -CO-(CH₂)₁₄-CH₃; *X = amino acids cyclized to form a double bond containing hydrocarbon ring; S₅ₘₑ = (S)-2-amino-2-methylhept-6-enoic acid; S7me = (S)-2-amino-2-methylnon-8-enoic acid; S5H = (S)-2-aminohept-6-enoic acid; R6me = (R)-2-amino-2-methyloct-7-enoic acid; and NH₂ = C-terminal amide |

The present invention further includes compositions comprising or consisting of one or more of the peptides shown in Table 1 and a pharmaceutically acceptable carrier.

"a-amino acid" or simply "amino acid" refers to a molecule containing both an amino group and a carboxyl group bound to a carbon, which is designated the α-carbon, attached to a side chain (R group) and a hydrogen atom and may be represented by the formula shown for (R) and (S) α-amino acids

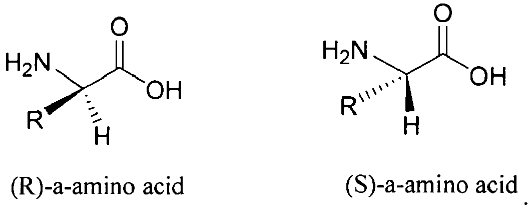

In general, L-amino acids have an (S) configuration except for cysteine, which has an (R) configuration, and glycine, which is achiral. Suitable α-amino acids for the all-D configuration peptides disclosed herein include only the D-isomers of the naturally-occurring amino acids and analogs thereof, as well as non-naturally occurring amino acids prepared by organic synthesis or other metabolic routes except for α,α-disubstituted amino acids, which may be L, D, or achiral. Unless the context specifically indicates otherwise, the term amino acid, as used herein, is intended to include amino acid analogs. As used herein, D amino acids are denoted by the superscript "D" (e.g., ^{D}Leu) and L amino acids by "L" (e.g., L-Leu) or no L identifier (e.g., Leu).

"α,α-disubstituted amino acid" refers to a molecule or moiety containing both an amino group and a carboxyl group bound to the α-carbon that is attached to two natural or non-natural amino acid side chains, or combination thereof. Exemplary α,α-disubstituted amino are shown below. These α,α-disubstituted amino acids comprise a side chain with a terminal olefinic reactive group.

The aforementioned amino acids used for introducing the staple by ring closing metathesis have the following structures: 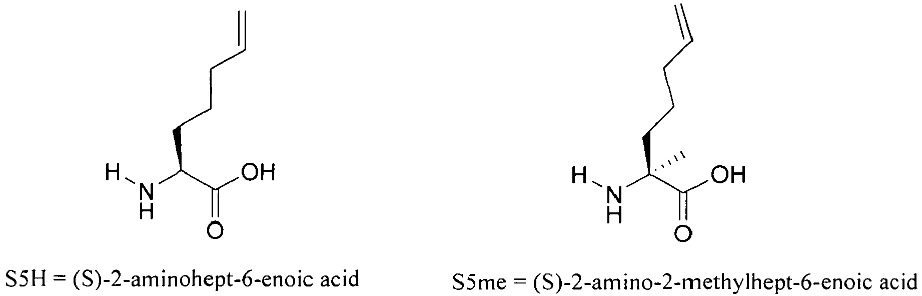 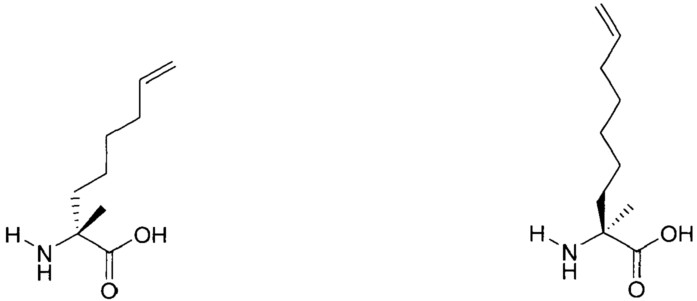 R6me = (R)-2-amino-2-methyloct-7-enoic acid S7me = (S)-2-amino-2-methylnon-8-enoic acid

The co-agonist peptides of the present invention may be conjugated to an α,ω-dicarboxylic acid comprising an aliphatic chain of 14 to 20 methylene groups (fatty diacid) wherein one end of the molecule is the proximal end and the other end is the distal end and wherein the proximal end and the distal end both have a carboxyl (COOH) group. The fatty diacid may be represented by the structure HO₂C(CH₂)ₙCO₂H, wherein n is 11, 12, 13, 14, 15, 16, 17, or 18 to provide fatty diacids Tetradecanedioic acid, Hexadecanedioic acid, Heptadecanedioic acid, Octadecanedioic acid, Nonadecanedioic acid, and Eicosanedioic acid, respectively. The aforementioned fatty diacids have the following structures

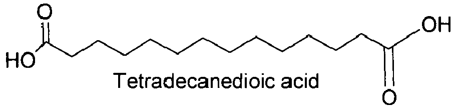

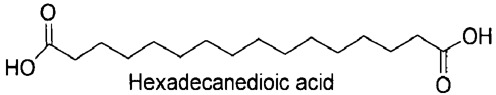

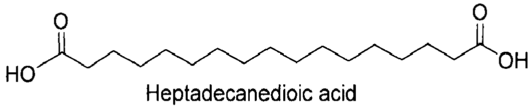

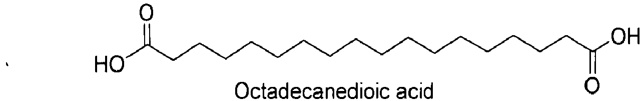

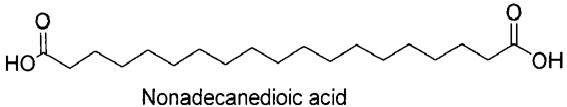

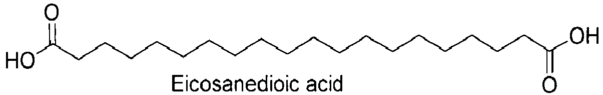

As a component of the co-agonist peptide, the acid functionality at the proximal end of the fatty diacid is conjugated to the amino group of a linker in a C(O)-NH linkage and the acid functionality at the distal end of the fatty diacid is a free carboxyl group (COOH). The COOH group at the distal end helps confer a longer half-life to the co-agonist peptide by its ability to non-covalently bind to serum albumin, a known carrier for fatty acids in serum. The COOH group enhances duration of action as it provides a better non-covalent interaction with serum albumin than peptides that have been acylated using a fatty acid, which bind serum albumin less efficiently and form a less stable non-covalent interaction with the serum albumin.

The co-agonist peptides of the present invention may also be conjugated to a carboxylic acid comprising an aliphatic chain of 14 to 20 methylene groups (fatty acid) wherein one end of the molecule is the proximal end and the other end is the distal end and wherein the proximal end or the distal end has a carboxyl (COOH) group. The fatty acid may be represented by the structure HO₂C(CH₂)ₙCH₃, wherein n is 11, 12, 13, 14, 15, 16, 17, or 18 to provide fatty acids Tetradecanoic acid, Hexadecanoic acid, Heptadecanoic acid, Octadecanoic acid, Nonadecanoic acid, and Eicosanoic acid, respectively. The fatty acid may have one of the following structures

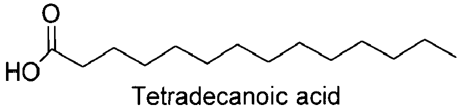

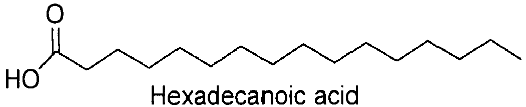

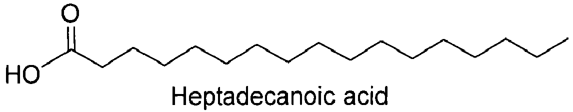

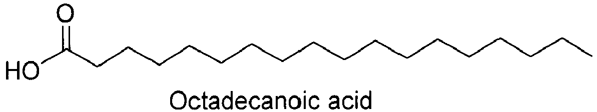

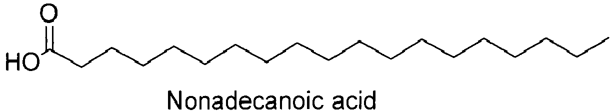

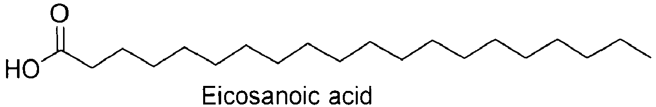

In particular aspects, the GCG/GLP-1 receptor co-agonist peptide is further conjugated to a fatty acid at position 10 of the peptide. The fatty acid may be represented by the structure HO₂C(CH₂)ₙ wherein n is 11, 12, 13, 14, 15, 16, 17, 18 or 19.

The structure of K(γEγE-fatty acid) wherein the linker is γEγE and the fatty acid component comprises C14, C16, C17, C18, C19, or C20 is represented by 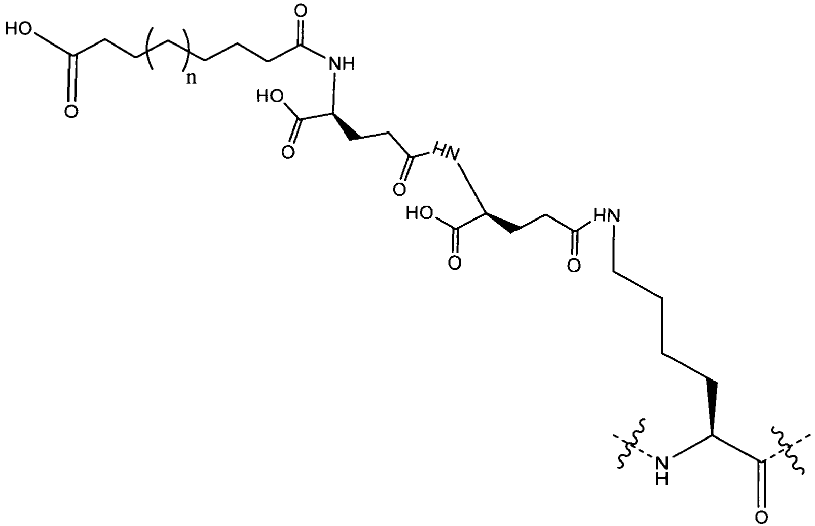 wherein n is 7, 9, 10, 11, 12, 13, or 14, respectively, and the wavy lines represent the bonds between adjacent amino acids in the co-agonist peptide sequence.

In the peptides shown herein, the structure of K(γEγE-C16) may be represented by 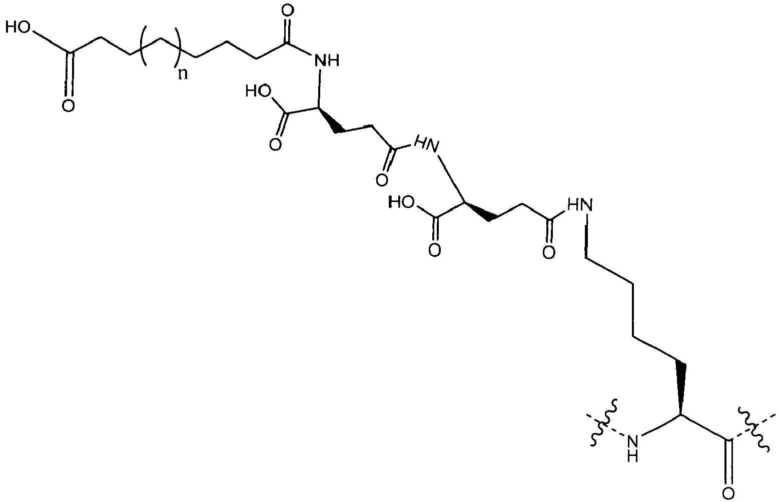 wherein n is 9 and the wavy lines represent the bonds between adjacent amino acids in the peptide sequence.

In the peptides shown herein, the structure of K(γE-C16) may be represented by 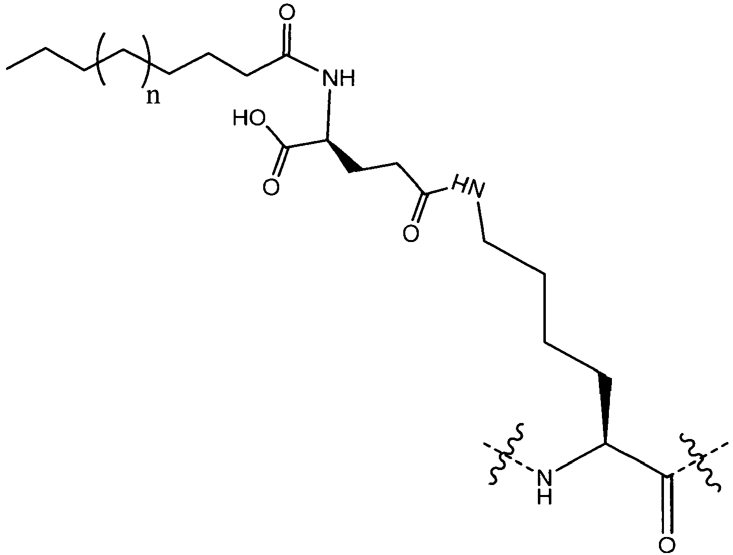 wherein n is 9 and the wavy lines represent the bonds between adjacent amino acids in the peptide sequence.

The structure of KγE at position 30 in the co-agonist peptide is represented by 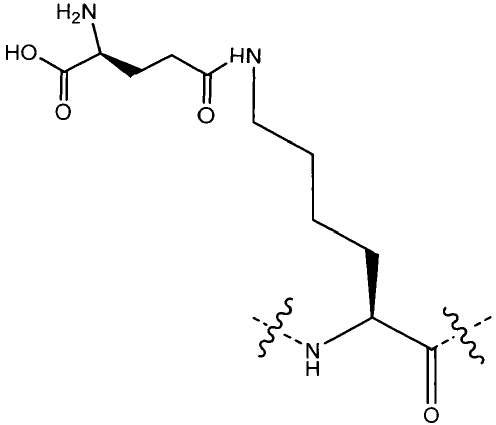 wherein the wavy lines represent the bonds between adjacent amino acids in the co-agonist peptide sequence.

The stapled GCG/GLP-1 receptor co-agonist peptides of the present invention have measurable activity at the glucagon receptor and/or the GLP-1 receptor.

The co-agonist peptides disclosed herein may have anywhere from at least about 1% (including at least about 1.5%, 2%, 5%, 7%, 10%, 20%, 30%, 40%, 50%, 60%, 75%, 100%, 125%, 150%, 175%) to about 200% or higher activity at the GLP-1 receptor relative to native GLP-1 and anywhere from at least about 1% (including about 1.5%, 2%, 5%, 7%, 10%, 20%, 30%, 40%, 50%, 60%, 75%, 100%, 125%, 150%, 175%, 200%, 250%, 300%, 350%, 400%, 450%) to about 500% or higher activity at the glucagon receptor relative to native glucagon. In some embodiments, the co-agonist peptides described herein exhibit no more than about 100%, 1000%, 10,000%, 100,000%, or 1,000,000% of the activity of native glucagon at the glucagon receptor. In some embodiments, the co-agonist peptides described herein exhibit no more than about 100%, 1000%, 10,000%, 100,000%, or 1,000,000% of the activity of native GLP-1 at the GLP-1 receptor. In exemplary embodiments, a co-agonist peptide may exhibit at least 10% of the activity of native glucagon at the glucagon receptor and at least 50% of the activity of native GLP-1 at the GLP-1 receptor, or at least 40% of the activity of native glucagon at the glucagon receptor and at least 40% of the activity of native GLP-1 at the GLP-1 receptor, or at least 60% of the activity of native glucagon at the glucagon receptor and at least 60% of the activity of native GLP-1 at the GLP-1 receptor.

The present invention further provides a method for treating a patient for metabolic disease comprising administering the patient an effective amount of the peptide of any one of the aforementioned peptides to treat the metabolic disease in the patient.

The present invention further provides method for treating a patient for metabolic disease comprising administering the patient an effective amount of the composition of the aforementioned compositions to treat the metabolic disease in the patient.

In particular aspects, the metabolic disease is diabetes, non-alcoholic fatty liver disease (NAFLD), non-alcoholic steatohepatitis (NASH), or obesity.

In particular aspects, the diabetes is Type I diabetes, Type II diabetes, or gestational diabetes.

In particular aspects, the patient has more than one metabolic disease, for example, diabetes and NASH, NAFLD, or obesity; obesity and NASH or NAFLD; diabetes, NASH, and obesity; diabetes, NAFLD, and obesity; or diabetes and obesity.

The present invention further provides for the use of any one of the aforementioned peptides for manufacture of a medicament for the treatment of metabolic disease.

The present invention further provides for the use of any one of the aforementioned compositions for manufacture of a medicament for the treatment of metabolic disease.

In particular aspects, the metabolic disease is diabetes, non-alcoholic fatty liver disease (NAFLD), non-alcoholic steatohepatitis (NASH), or obesity.

In particular aspects, the diabetes is Type I diabetes, Type II diabetes, or gestational diabetes.

In particular aspects, the medicament is for treatment of more than one metabolic disease, for example, diabetes and NASH, NAFLD, or obesity; obesity and NASH or NAFLD; diabetes, NASH, and obesity; diabetes, NAFLD, and obesity; or diabetes and obesity.

In particular aspects of the compounds disclosed herein the C-terminal protecting group may be an amide or ester. For example, the carboxylic acid of the C-terminal amino acid is replaced with a charge-neutral group, such as an amide or ester.

Further provided is method for treating a metabolic disease in a patient or individual comprising: administering to the patient or individual an effective amount of any one of the aforementioned compositions comprising a co-agonist peptide and administering to the patient or individual an effective amount of a composition comprising an insulin or insulin analog to treat the metabolic disease in the patient or individual.

In particular aspects, the composition comprising the co-agonist peptide is administered at a time prior to the time the composition comprising the insulin or insulin analog is administered. In another aspect, the composition comprising the insulin or insulin analog is administered at a time prior to the time the composition comprising the co-agonist peptide is administered. In a further still aspect, the composition comprising the co-agonist peptide is administered at the same time as the composition comprising the insulin or insulin analog is administered.

In particular aspects, the insulin analog is insulin detemir, insulin glargine, insulin levemir, insulin glulisine, or insulin lispro.

In particular aspects, the metabolic disease is diabetes, non-alcoholic fatty liver disease (NAFLD), non-alcoholic steatohepatitis (NASH), or obesity.

In particular aspects, the diabetes is Type I diabetes, Type II diabetes, or gestational diabetes.

In particular aspects, the patient has more than one metabolic disease, for example, diabetes and NASH, NAFLD, or obesity; obesity and NASH or NAFLD; diabetes, NASH, and obesity; diabetes, NAFLD, and obesity; or diabetes and obesity.

The present invention further provides a composition comprising any one of the aforementioned peptides; an insulin or insulin analog; and, a pharmaceutically acceptable carrier.

The present invention further provides for the use of a composition comprising any one of the aforementioned peptides; an insulin or insulin analog; and, a pharmaceutically acceptable carrier for the treatment of a metabolic disease.

In particular aspects, the metabolic disease is diabetes, non-alcoholic fatty liver disease (NAFLD), non-alcoholic steatohepatitis (NASH), or obesity. In further aspects, the diabetes is Type I diabetes, Type II diabetes, or gestational diabetes.

The present invention further provides for the use of a composition comprising any one of the aforementioned peptides; an insulin or insulin analog; and, a pharmaceutically acceptable carrier for the manufacture of a medicament for the treatment of a metabolic disease.

In particular aspects, the metabolic disease is diabetes, non-alcoholic fatty liver disease (NAFLD), non-alcoholic steatohepatitis (NASH), or obesity. In further aspects, the diabetes is Type I diabetes, Type II diabetes, or gestational diabetes.

### Definitions

The term "about" as used herein means greater or lesser than the value or range of values stated by 10 percent, but is not intended to designate any value or range of values to only this broader definition. Each value or range of values preceded by the term "about" is also intended to encompass the embodiment of the stated absolute value or range of values.

The term "individual" is meant to include humans and companion or domesticated animals such as dogs, cats, horses, and the like. Therefore, the compositions comprising a compound or one or more co-agonist peptides as disclosed herein are also useful for treating or preventing obesity and obesity-related disorders in cats and dogs. As such, the term "mammal" includes humans and companion animals such as cats and dogs.

As used herein, the term "pharmaceutically acceptable carrier" includes any carrier suitable for administering to an individual, for example any of the standard pharmaceutical carriers, including but not limited to a phosphate buffered saline solution, water, emulsions such as an oil/water or water/oil emulsion, and various types of wetting agents. The term also encompasses any of the agents approved by a regulatory agency of the U.S. Federal government or listed in the U.S. Pharmacopeia for use in animals, including humans. In general, "pharmaceutically acceptable carrier" means a non-toxic material that does not interfere with the effectiveness of the biological activity of the active ingredient(s), approved by a regulatory agency of the Federal or a state government or listed in the U.S. Pharmacopoeia or other generally recognized pharmacopoeia for use in animals and, more particularly, in humans. The term "carrier" refers to a diluent, adjuvant, excipient, or vehicle with which the therapeutic is administered and includes, but is not limited to such sterile liquids as water and oils. The characteristics of the carrier will depend on the route of administration.

As used herein the term "pharmaceutically acceptable salt" refers to salts of compounds that retain the biological activity of the parent compound, and which are not biologically or otherwise undesirable. Many of the compounds disclosed herein are capable of forming acid and/or base salts by virtue of the presence of amino and/or carboxyl groups or groups similar thereto. Pharmaceutically acceptable base addition salts can be prepared from inorganic and organic bases. Salts derived from inorganic bases, include by way of example only, sodium, potassium, lithium, ammonium, calcium and magnesium salts. Salts derived from organic bases include, but are not limited to, salts of primary, secondary and tertiary amines.

Pharmaceutically acceptable salts may be prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc, and the like. Particularly preferred are the ammonium, calcium, magnesium, potassium, and sodium salts. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethyl-morpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like. The term "pharmaceutically acceptable salt" further includes all acceptable salts such as acetate, lactobionate, benzenesulfonate, laurate, benzoate, malate, bicarbonate, maleate, bisulfate, mandelate, bitartrate, mesylate, borate, methylbromide, bromide, methylnitrate, calcium edetate, methylsulfate, camsylate, mucate, carbonate, napsylate, chloride, nitrate, clavulanate, N-methylglucamine, citrate, ammonium salt, dihydrochloride, oleate, edetate, oxalate, edisylate, pamoate (embonate), estolate, palmitate, esylate, pantothenate, fumarate, phosphate/diphosphate, gluceptate, polygalacturonate, gluconate, salicylate, glutamate, stearate, glycollylarsanilate, sulfate, hexylresorcinate, subacetate, hydrabamine, succinate, hydrobromide, tannate, hydrochloride, tartrate, hydroxynaphthoate, teoclate, iodide, tosylate, isothionate, triethiodide, trifluoro acetate (TFA salt), lactate, panoate, valerate, and the like which can be used as a dosage form for modifying the solubility or hydrolysis characteristics or can be used in sustained release or pro-drug formulations. It will be understood that, as used herein, references to the co-agonist peptides disclosed herein are meant to also include embodiments that comprise a co-agonist peptide or peptides and one or more of the pharmaceutically acceptable salts.

As used herein, the term "treating" includes prophylaxis of the specific disorder or condition, or alleviation of the symptoms associated with a specific disorder or condition and/or preventing or eliminating said symptoms. For example, as used herein the term "treating diabetes" will refer in general to altering glucose blood levels in the direction of normal levels and may include increasing or decreasing blood glucose levels depending on a given situation.

As used herein an "effective" amount or a "therapeutically effective amount" means the total amount of each active component of the pharmaceutical composition or method that is sufficient to show a meaningful patient benefit, i.e., treatment, healing, prevention or amelioration of the relevant medical condition, or an increase in rate of treatment, healing, prevention or amelioration of such conditions. It refers to the amount of a co-agonist peptide or peptides that is nontoxic but sufficient to provide the desired effect. For example one desired effect would be the prevention or treatment of hyperglycemia, e.g., as measured by a change in blood glucose level closer to normal, or treatment of obesity by inducing weight loss and/or preventing weight gain, e.g., as measured by reduction in body weight, or preventing or reducing an increase in body weight, or normalizing body fat distribution. The amount that is "effective" will vary from subject to subject, depending on the age and general condition of the individual, mode of administration, and the like. Thus, it is not always possible to specify an exact "effective amount." However, an appropriate "effective" amount in any individual case may be determined by one of ordinary skill in the art using routine experimentation. When applied to an individual active ingredient, administered alone, the term refers to that ingredient alone. When applied to a combination, the term refers to combined amounts of the active ingredients that result in the therapeutic effect, whether administered in combination, serially, or simultaneously.

The term, "parenteral" means not through the alimentary canal but by some other route, e.g., subcutaneous, intramuscular, intraspinal, or intravenous.

As used herein, the term "peptide" encompasses a chain of 3 or more amino acids and typically less than 100 amino acids, wherein the amino acids are naturally occurring or coded or non-naturally occurring or non-coded amino acids. Non-naturally occurring amino acids refer to amino acids that do not naturally occur in vivo but which, nevertheless, can be incorporated into the peptide structures described herein. "Non-coded" as used herein refers to an amino acid that is not an L-isomer of any of the following 20 amino acids: Ala, Cys, Asp, Glu, Phe, Gly, His, He, Lys, Leu, Met, Asn, Pro, Gln, Arg, Ser, Thr, Val, Trp, Tyr. "Coded" as used herein refers to an amino acid that is an L-isomer of any of the following 20 amino acids: Ala, Cys, Asp, Glu, Phe, Gly, His, He, Lys, Leu, Met, Asn, Pro, Gin, Arg, Ser, Thr, Val, Trp, Tyr. In some embodiments, the peptides and variant peptides described herein are about the same length as SEQ ID NO: 1 (which is 29 amino acids in length), e.g. 25-35 amino acids in length. Exemplary lengths include 29, 30, 31, 32, 33, 34, 35, 36, 37, 38, 39, 40, 41, 42, 43, 44, 45, 46, 47, 48, 49, or 50 amino acids in length.

Typically, polypeptides and proteins have a polymer length that is greater than that of "peptides."

Similarly, a reference herein to "position 28" would mean the corresponding position 29 for a glucagon analog in which one amino acid has been added before the N-terminus of SEQ ID NO: 1. As used herein an "amino acid modification" refers to (i) a substitution or replacement of an amino acid of SEQ ID NO: 1 with a different amino acid (naturally- occurring or coded or non-coded or non-naturally-occurring amino acid), (ii) an addition of an amino acid (naturally-occurring or coded or non-coded or non-naturally-occurring amino acid), to SEQ ID NO: 1 or (iii) a deletion of one or more amino acids of SEQ ID NO: 1.

Amino acid "modification" refers to an insertion, deletion or substitution of one amino acid with another. In some embodiments, the amino acid substitution or replacement is a conservative amino acid substitution, e.g., a conservative substitution of the amino acid at one or more of positions 2, 5, 7, 10, 11, 12, 13, 14, 16, 17, 18, 19, 20, 21, 24, 27, 28 or 29. As used herein, the term "conservative amino acid substitution" is the replacement of one amino acid with another amino acid having similar properties, e.g., size, charge, hydrophobicity, hydrophilicity, and/or aromaticity, and includes exchanges within one of the following five groups:
I. Small aliphatic, nonpolar or slightly polar residues:
   Ala, Ser, Thr, Pro, Gly;
II. Polar, negatively- charged residues and their amides and esters:
   Asp, Asn, Glu, Gin, cysteic acid and homocysteic acid;
III. Polar, positively- charged residues:
   His, Arg, Lys; Ornithine (Orn)
IV. Large, aliphatic, nonpolar residues:
   Met, Leu, He, Val, Cys, Norleucine (Nle), homocysteine
V. Large, aromatic residues:
   Phe, Tyr, Trp, acetyl phenylalanine

In some embodiments, the amino acid substitution is not a conservative amino acid substitution, e.g., is a non-conservative amino acid substitution.

As used herein the term "charged amino acid" or "charged residue" refers to an amino acid that comprises a side chain that is negatively- charged (i.e., de-protonated) or positively-charged (i.e., protonated) in aqueous solution at physiological pH. For example negatively-charged amino acids include aspartic acid, glutamic acid, cysteic acid, homocysteic acid, and homoglutamic acid, whereas positively-charged amino acids include arginine, lysine and histidine. Charged amino acids include the charged amino acids among the 20 coded amino acids, as well as atypical or non-naturally occurring or non- coded amino acids.

As used herein the term "acidic amino acid" refers to an amino acid that comprises a second acidic moiety (other than the carboxylic acid of the amino acid), including for example, a carboxylic acid or sulfonic acid group.

As used herein, the term "acylated amino acid" refers to an amino acid comprising an acyl group which is non-native to a naturally-occurring amino acid, regardless of the means by which it is produced (e.g. acylation prior to incorporating the amino acid into a peptide, or acylation after incorporation into a peptide).

As used herein the term "alkylated amino acid" refers to an amino acid comprising an alkyl group which is non-native to a naturally-occurring amino acid, regardless of the means by which it is produced. Accordingly, the acylated amino acids and alkylated amino acids of the present disclosures are non-coded amino acids.

In particular aspects of the compounds disclosed herein the C-terminal protecting group may be an amide or ester. For example, the carboxylic acid of the C-terminal amino acid is replaced with a charge-neutral group, such as an amide or ester.

As used herein, the term "selectivity" of a molecule for a first receptor relative to a second receptor refers to the following ratio: EC₅₀ of the molecule at the second receptor divided by the EC₅₀ of the molecule at the first receptor. For example, a molecule that has an EC50 of 1 nM at a first receptor and an EC₅₀ of 100 nM at a second receptor has 100-fold selectivity for the first receptor relative to the second receptor.

As used herein the term "native glucagon" refers to a peptide consisting of the sequence of SEQ ID NO: 1 and the term "native GLP-1" is a generic term that designates GLP- 1(7-36) amide, GLP-1 (7-37) acid or a mixture of those two compounds.

As used herein, "glucagon potency" or "potency compared to native glucagon" of a molecule refers to the inverse ratio of the EC₅₀ of the molecule at the glucagon receptor divided by the EC₅₀ of native glucagon at glucagon receptor.

As used herein, "GLP-1 potency" or "potency compared to native GLP-1" of a molecule refers to the inverse ratio of the EC₅₀ of the molecule at GLP-1 receptor divided by the EC₅₀ of native GLP-1 at GLP-1 receptor.

### Pharmaceutical Compositions

Further provided are pharmaceutical compositions comprising a therapeutically effective amount of one or more of the co-agonist peptides disclosed herein for the treatment of a metabolic disorder in an individual. Such disorders include, but are not limited to, obesity, metabolic syndrome or syndrome X, type II diabetes, complications of diabetes such as retinopathy, hypertension, dyslipidemias, cardiovascular disease, gallstones, osteoarthritis, and certain forms of cancers. The obesity-related disorders herein are associated with, caused by, or result from obesity.

"Obesity" is a condition in which there is an excess of body fat. The operational definition of obesity is based on the Body Mass Index (BMI), calculated as body weight per height in meters squared (kg/m²). "Obesity" refers to a condition whereby an otherwise healthy subject has a Body Mass Index (BMI) greater than or equal to 30 kg/m², or a condition whereby a subject with at least one co-morbidity has a BMI greater than or equal to 27 kg/m². An "obese subject" is an otherwise healthy subject with a Body Mass Index (BMI) greater than or equal to 30 kg/m² or a subject with at least one co-morbidity with a BMI greater than or equal to 27 kg/m². A "subject at risk for obesity" is an otherwise healthy subject with a BMI of 25 kg/m² to less than 30 kg/m² or a subject with at least one co-morbidity with a BMI of 25 kg/m² to less than 27 kg/m².

The increased risks associated with obesity occur at a lower Body Mass Index (BMI) in Asians. In Asian countries, including Japan, "obesity" refers to a condition whereby a subject with at least one obesity-induced or obesity-related co-morbidity that requires weight reduction or that would be improved by weight reduction, has a BMI greater than or equal to 25 kg/m². In Asian countries, including Japan, an "obese subject" refers to a subject with at least one obesity-induced or obesity-related co-morbidity that requires weight reduction or that would be improved by weight reduction, with a BMI greater than or equal to 25 kg/m². In Asian countries, a "subject at risk of obesity" is a subject with a BMI of greater than 23 kg/m² to less than 25 kg/m².

As used herein, the term "obesity" is meant to encompass all of the above definitions of obesity.

Obesity-induced or obesity-related co-morbidities include, but are not limited to, diabetes, non-insulin dependent diabetes mellitus - type 2, impaired glucose tolerance, impaired fasting glucose, insulin resistance syndrome, dyslipidemia, hypertension, hyperuricacidemia, gout, coronary artery disease, myocardial infarction, angina pectoris, sleep apnea syndrome, Pickwickian syndrome, non-alcoholic fatty liver disease (NAFLD), non-alcoholic steatohepatitis (NASH), fatty liver; cerebral infarction, cerebral thrombosis, transient ischemic attack, orthopedic disorders, arthritis deformans, lumbodynia, emmeniopathy, and infertility. In particular, co-morbidities include: hypertension, hyperlipidemia, dyslipidemia, glucose intolerance, cardiovascular disease, sleep apnea, diabetes mellitus, and other obesity-related conditions.

"Treatment" (of obesity and obesity-related disorders) refers to the administration of the compounds of the present invention to reduce or maintain the body weight of an obese subject. One outcome of treatment may be reducing the body weight of an obese subject relative to that subject's body weight immediately before the administration of the compounds of the present invention. Another outcome of treatment may be preventing body weight regain of body weight previously lost as a result of diet, exercise, or pharmacotherapy. Another outcome of treatment may be decreasing the occurrence of and/or the severity of obesity-related diseases. The treatment may suitably result in a reduction in food or calorie intake by the subject, including a reduction in total food intake, or a reduction of intake of specific components of the diet such as carbohydrates or fats; and/or the inhibition of nutrient absorption; and/or the inhibition of the reduction of metabolic rate; and in weight reduction in patients in need thereof. The treatment may also result in an alteration of metabolic rate, such as an increase in metabolic rate, rather than or in addition to an inhibition of the reduction of metabolic rate; and/or in minimization of the metabolic resistance that normally results from weight loss.

"Prevention" (of obesity and obesity-related disorders) refers to the administration of the compounds of the present invention to reduce or maintain the body weight of a subject at risk of obesity. One outcome of prevention may be reducing the body weight of a subject at risk of obesity relative to that subject's body weight immediately before the administration of the compounds of the present invention. Another outcome of prevention may be preventing body weight regain of body weight previously lost as a result of diet, exercise, or pharmacotherapy. Another outcome of prevention may be preventing obesity from occurring if the treatment is administered prior to the onset of obesity in a subject at risk of obesity. Another outcome of prevention may be decreasing the occurrence and/or severity of obesity-related disorders if the treatment is administered prior to the onset of obesity in a subject at risk of obesity. Moreover, if treatment is commenced in already obese subjects, such treatment may prevent the occurrence, progression or severity of obesity-related disorders, such as, but not limited to, arteriosclerosis, Type II diabetes, polycystic ovarian disease, cardiovascular diseases, osteoarthritis, dermatological disorders, hypertension, insulin resistance, hypercholesterolemia, hypertriglyceridemia, and cholelithiasis.

The obesity-related disorders herein are associated with, caused by, or result from obesity. Examples of obesity-related disorders include overeating and bulimia, hypertension, diabetes, elevated plasma insulin concentrations and insulin resistance, dyslipidemias, hyperlipidemia, endometrial, breast, prostate and colon cancer, osteoarthritis, obstructive sleep apnea, cholelithiasis, gallstones, heart disease, abnormal heart rhythms and arrythmias, myocardial infarction, congestive heart failure, coronary heart disease, sudden death, stroke, polycystic ovarian disease, craniopharyngioma, the Prader-Willi Syndrome, Frohlich's syndrome, GH-deficient subjects, normal variant short stature, Turner's syndrome, and other pathological conditions showing reduced metabolic activity or a decrease in resting energy expenditure as a percentage of total fat-free mass, e.g, children with acute lymphoblastic leukemia. Further examples of obesity-related disorders are metabolic syndrome, also known as syndrome X, insulin resistance syndrome, sexual and reproductive dysfunction, such as infertility, hypogonadism in males and hirsutism in females, gastrointestinal motility disorders, such as obesity-related gastro-esophageal reflux, respiratory disorders, such as obesity-hypoventilation syndrome (Pickwickian syndrome), cardiovascular disorders, inflammation, such as systemic inflammation of the vasculature, arteriosclerosis, hypercholesterolemia, hyperuricaemia, lower back pain, gallbladder disease, gout, and kidney cancer. The compounds of the present invention are also useful for reducing the risk of secondary outcomes of obesity, such as reducing the risk of left ventricular hypertrophy.

The term "diabetes," as used herein, includes both insulin-dependent diabetes mellitus (IDDM, also known as type I diabetes) and non-insulin-dependent diabetes mellitus (NIDDM, also known as Type II diabetes). Type I diabetes, or insulin-dependent diabetes, is the result of an absolute deficiency of insulin, the hormone which regulates glucose utilization. Type II diabetes, or insulin-independent diabetes (i.e., non-insulin-dependent diabetes mellitus), often occurs in the face of normal, or even elevated levels of insulin and appears to be the result of the inability of tissues to respond appropriately to insulin. Most of the Type II diabetics are also obese. The compounds of the present invention are useful for treating both Type I and Type II diabetes. The compounds are especially effective for treating Type II diabetes. The compounds of the present invention are also useful for treating and/or preventing gestational diabetes mellitus.

The co-agonist peptides disclosed herein are insulinotropic and can be administered to patients with a disturbed glucose metabolism such as insulin resistance but no overt diabetes, as well as patients who for any reason cannot receive nutrition through the alimentary canal. Such patients include surgery patients, comatose patients, patients in shock, patients with gastrointestinal disease, patients with digestive hormone disease, and the like. In particular, obese patients, atherosclerotic patients, vascular disease patients, patients with gestational diabetes, patients with liver disease such as liver cirrhosis, patients with acromegaly, patients with glucorticoid excess such as cortisol treatment or Cushings disease, patients with activated counterregulatory hormones such as would occur after trauma, accidents and surgery and the like, patients with hypertriglyceridemia and patients with chronic pancreatitis can be readily and suitably nourished according to the invention without subjecting the patient to hypo- or hyperglycemia. In particular, the administration to such a patient aims to provide a therapy to as rapidly as possible deliver the nutritional and caloric requirements to the patient while maintaining his plasma glucose below the so-called renal threshold of about 160 to 180 milligrams per deciliter of glucose in the blood. Although normal patients not having glucose levels just below the renal threshold can also be treated according to the invention as described above, patients with disturbed glucose metabolism such as hyperglycemic patients whose plasma glucose level is just above the renal threshold also find the therapy suitable for their condition. In particular, such patients who have a degree of hyperglycemia below the renal threshold at intermittent intervals can receive a combination treatment of nutrients plus insulinotropic peptides according to any of the following regimens. Normal patients not suffering from such hyperglycemia can also be treated using the peptide analogs disclosed herein.

The co-agonist peptides disclosed herein may be used in a pharmaceutical composition when combined with a pharmaceutically acceptable carrier. Such compositions comprise a therapeutically-effective amount of one or more of the co-agonist peptides disclosed herein and a pharmaceutically acceptable carrier. Such a composition may also be comprised of (in addition to the co-agonist peptides disclosed herein and a carrier) diluents, fillers, salts, buffers, stabilizers, solubilizers, and other materials well known in the art. Compositions comprising the co-agonist peptides disclosed herein can be administered, if desired, in the form of salts provided the salts are pharmaceutically acceptable. Salts may be prepared using standard procedures known to those skilled in the art of synthetic organic chemistry. The co-agonist peptides disclosed herein may be in multimers (for example, heterodimers or homodimers) or complexes with itself or other peptides. As a result, pharmaceutical compositions of the invention may comprise one or more co-agonist peptides disclosed herein in such multimeric or complexed form.

The pharmacological composition can comprise one or more co-agonist peptides disclosed herein; one or more co-agonist peptides disclosed herein and one or more other agents for treating a metabolic disorder; or the pharmacological composition comprising the one or more co-agonist peptides disclosed herein can be used concurrently with a pharmacological composition comprising an agent for treating a metabolic disorder. Such disorders include, but are not limited to, obesity, metabolic syndrome or syndrome X, type II diabetes, complications of diabetes, hypertension, dyslipidemias, cardiovascular disease, gallstones, osteoarthritis, and certain forms of cancers.

When the pharmacological composition comprises another agent for treating a metabolic disorder or the treatment includes a second pharmacological composition comprising an agent for treating a metabolic disorder, the agent includes, but are not limited to, cannabinoid (CB1) receptor antagonists, glucagon like peptide 1 (GLP-1) receptor agonists, glucagon receptor antagonists, lipase inhibitors, leptin, tetrahydrolipstatin, 2-4-dinitrophenol, acarbose, sibutramine, phentamine, fat absorption blockers, simvastatin, mevastatin, ezetimibe, atorvastatin, sitagliptin, metformin, orlistat, Qnexa, topiramate, naltrexone, bupriopion, phentermine, losartan, losartan with hydrochlorothiazide, and the like.

Examples of other active ingredients that may be administered separately or in the same pharmaceutical composition in combination with a co-agonist peptide as described herein include, but are not limited to:
(1) dipeptidyl peptidase-IV (DPP-4) inhibitors (e.g., sitagliptin, alogliptin, linagliptin, vildagliptin, saxagliptin and omarigliptin);
(2) insulin sensitizers, including (i) PPARγ agonists, such as the glitazones (e.g. pioglitazone, AMG 131, MBX2044, mitoglitazone, lobeglitazone, IDR-105, rosiglitazone, and balaglitazone), and other PPAR ligands, including (1) PPARα/γ dual agonists (e.g., ZYH2, ZYH1, GFT505, chiglitazar, muraglitazar, aleglitazar, sodelglitazar, and naveglitazar); (2) PPARα agonists such as fenofibric acid derivatives (e.g., gemfibrozil, clofibrate, ciprofibrate, fenofibrate, bezafibrate), (3) selective PPARγ modulators (SPPARyM's), (e.g., such as those disclosed in WO 02/060388, WO 02/08188, WO 2004/019869, WO 2004/020409, WO 2004/020408, and WO 2004/066963); and (4) PPARγ partial agonists; (ii) biguanides, such as metformin and its pharmaceutically acceptable salts, in particular, metformin hydrochloride, and extended-release formulations thereof, such as Glumetza™, Fortamet™, and GlucophageXR™; and (iii) protein tyrosine phosphatase-1B (PTP-1B) inhibitors (e.g., ISIS-113715 and TTP814);
(3) insulin or insulin analogs (e.g., insulin detemir, insulin glulisine, insulin degludec, insulin glargine, insulin lispro and inhalable formulations of each);
(4) leptin and leptin derivatives and agonists;
(5) amylin and amylin analogs (e.g., pramlintide);
(6) sulfonylurea and non-sulfonylurea insulin secretagogues (e.g., tolbutamide, glyburide, glipizide, glimepiride, mitiglinide, meglitinides, nateglinide and repaglinide);
(7) α-glucosidase inhibitors (e.g., acarbose, voglibose and miglitol);
(8) glucagon receptor antagonists (e.g., such as those disclosed in WO 98/04528, WO 99/01423, WO 00/39088, and WO 00/69810);
(9) incretin mimetics, such as GLP-1, GLP-1 analogs, derivatives, and mimetics; and GLP-1 receptor agonists (e.g., dulaglutide, semaglutide, albiglutide, exenatide, liraglutide, lixisenatide, taspoglutide, CJC-1131, and BIM-51077, including intranasal, transdermal, and once-weekly formulations thereof);
(10) LDL cholesterol lowering agents such as (i) HMG-CoA reductase inhibitors (e.g., simvastatin, lovastatin, pravastatin, crivastatin, fluvastatin, atorvastatin, pitavastatin and rosuvastatin), (ii) bile acid sequestering agents (e.g., colestilan, colestimide, colesevalam hydrochloride, colestipol, cholestyramine, and dialkylaminoalkyl derivatives of a cross-linked dextran), (iii) inhibitors of cholesterol absorption, (e.g., ezetimibe), and (iv) acyl CoA:cholesterol acyltransferase inhibitors, (e.g., avasimibe);
(11) HDL-raising drugs;
(12) antiobesity compounds;
(13) agents intended for use in inflammatory conditions, such as aspirin, non-steroidal anti-inflammatory drugs or NSAIDs, glucocorticoids, and selective cyclooxygenase-2 or COX-2 inhibitors;
(14) antihypertensive agents, such as ACE inhibitors (e.g.,lisinopril, enalapril, ramipril, captopril, quinapril, and tandolapril), A-II receptor blockers (e.g., losartan, candesartan, irbesartan, olmesartan medoxomil, valsartan, telmisartan, and eprosartan), renin inhibitors (e.g., aliskiren), beta blockers, and calcium channel blockers;
(15) glucokinase activators (GKAs) (e.g., AZD6370);
(16) inhibitors of 11β-hydroxysteroid dehydrogenase type 1, (e.g., such as those disclosed in U.S. Patent No. 6,730,690, and LY-2523199);
(17) CETP inhibitors (e.g., anacetrapib, and torcetrapib);
(18) inhibitors of fructose 1,6-bisphosphatase, (e.g., such as those disclosed in U.S. Patent Nos. 6,054,587; 6,110,903; 6,284,748; 6,399,782; and 6,489,476);
(19) inhibitors of acetyl CoA carboxylase-1 or 2 (ACC1 or ACC2);
(20) AMP-activated Protein Kinase (AMPK) activators;
(21) other agonists of the G-protein-coupled receptors: (i) GPR-109, (ii) GPR-119 (e.g., MBX2982 and PSN821), and (iii) GPR-40 (e.g., TAK875, 5-[4-[[(1R)-4-[6-(3-hydroxy-3-methylbutoxy)-2-methylpyridine-3-yl]-2,3-dihydro-1H-indene-1-yl]oxy]phenyl]isothiazole-3-ol 1-oxide,5-(4-((3-(2,6-dimethyl-4-(3-(methylsulfonyl)propoxy)phenyl)phenyl)methoxy)phenyl)iso, 5-(4-((3-(2-methyl-6-(3-hydroxypropoxy)pyridine-3-yl)-2-methylphenyl)methoxy)phenyl)isothiazole-3-ol 1-oxide, and 5-[4-[[3-[4-(3-aminopropoxy)-2,6-dimethylphenyl]phenyl]methoxy]phenyl]isothiazole-3-ol 1-oxide);
(22) SSTR3 antagonists (e.g., such as those disclosed in WO 2009/001836);
(23) neuromedin U receptor agonists (e.g., such as those disclosed in WO 2009/042053, including, but not limited to, neuromedin S (NMS));
(24) SCD inhibitors;
(25) GPR-105 antagonists (e.g., such as those disclosed in WO 2009/000087);
(26) SGLT inhibitors (e.g., ASP1941, SGLT-3, empagliflozin, dapagliflozin, canagliflozin, BI-10773, PF-04971729, remogloflozin, TS-071, tofogliflozin, ertugliflozin, ipragliflozin, and LX-4211);
(27) inhibitors of acyl coenzyme A:diacylglycerol acyltransferase 1 and 2 (DGAT-1 and DGAT-2);
(28) inhibitors of fatty acid synthase;
(29) inhibitors of acyl coenzyme A:monoacylglycerol acyltransferase 1 and 2 (MGAT-1 and MGAT-2);
(30) agonists of the TGR5 receptor (also known as GPBAR1, BG37, GPCR19, GPR131, and M-BAR);
(31) ileal bile acid transporter inhibitors;
(32) PACAP, PACAP mimetics, and PACAP receptor 3 agonists;
(33) PPAR agonists;
(34) protein tyrosine phosphatase-1B (PTP-1B) inhibitors;
(35) IL-1b antibodies, (e.g., XOMA052 and canakinumab); and
(36) bromocriptine mesylate and rapid-release formulations thereof.

Of particular interest are metformin hydrochloride, pioglitazone, rosiglitazone, simvastatin, atorvastatin, or a sulfonylurea.

Antiobesity compounds that can be combined with compounds as disclosed herein include topiramate; zonisamide; naltrexone; phentermine; bupropion; the combination of bupropion and naltrexone; the combination of bupropion and zonisamide; the combination of topiramate and phentermine; fenfluramine; dexfenfluramine; sibutramine; lipase inhibitors, such as orlistat and cetilistat; melanocortin receptor agonists, in particular, melanocortin-4 receptor agonists; CCK-1 agonists; melanin-concentrating hormone (MCH) receptor antagonists; neuropeptide Y₁ or Y₅ antagonists (such as MK-0557); CB1 receptor inverse agonists and antagonists (such as rimonabant and taranabant); β₃ adrenergic receptor agonists; ghrelin antagonists; bombesin receptor agonists (such as bombesin receptor subtype-3 agonists); and 5-hydroxytryptamine-2c (5-HT2c) agonists, such as lorcaserin. For a review of anti-obesity compounds that can be combined with compounds of the present invention, see Chaki et al., "Recent advances in feeding suppressing agents: potential therapeutic strategy for the treatment of obesity," Expert Opin. Ther. Patents, 11: 1677-1692 (2001); Spanswick and Lee, "Emerging antiobesity drugs," Expert Opin. Emerging Drugs, 8: 217-237 (2003); Fernandez-Lopez, et al., "Pharmacological Approaches for the Treatment of Obesity," Drugs, 62: 915-944 (2002); and Gadde, et al., "Combination pharmaceutical therapies for obesity," Exp. Opin. Pharmacother., 10: 921-925 (2009).

In another aspect of the invention, a pharmaceutical composition is disclosed which comprises one or more of the following agents:
(a) a compound as disclosed herein, e.g. a one or co-agonists as disclosed herein wherein each co-agonist may independently be a peptide comprising the amino acid sequence of SEQ ID NO:20;
(b) one or more compounds selected from the group consisting of:
   (1) dipeptidyl peptidase-IV (DPP-4) inhibitors;
   (2) insulin sensitizers, including (i) PPARγ agonists, such as the glitazones (e.g. AMG 131, MBX2044, mitoglitazone, lobeglitazone, IDR-105, pioglitazone, rosiglitazone, and balaglitazone) and other PPAR ligands, including (1) PPARα/γ dual agonists, such as ZYH1, YYH2, chiglitazar, GFT505, muraglitazar, aleglitazar, sodelglitazar, and naveglitazar, (2) PPARα agonists, such as fenofibric acid derivatives (e.g., gemfibrozil, clofibrate, ciprofibrate, fenofibrate and bezafibrate), (3) selective PPARγ modulators (SPPARyM's), and (4) PPARγ partial agonists; (ii) biguanides, such as metformin and its pharmaceutically acceptable salts, in particular, metformin hydrochloride, and extended-release formulations thereof, such as Glumetza®, Fortamet®, and GlucophageXR®; (iii) protein tyrosine phosphatase-1B (PTP-1B) inhibitors, such as ISI-113715, and TTP814;
   (3) sulfonylurea and non-sulfonylurea insulin secretagogues, (e.g., tolbutamide, glyburide, glipizide, glimepiride, mitiglinide, and meglitinides, such as nateglinide and repaglinide);
   (4) α-glucosidase inhibitors (e.g., acarbose, voglibose and miglitol);
   (5) glucagon receptor antagonists;
   (6) LDL cholesterol lowering agents such as (i) HMG-CoA reductase inhibitors (e.g., lovastatin, simvastatin, pravastatin, cerivastatin, fluvastatin, atorvastatin, pitavastatin, and rosuvastatin), (ii) bile acid sequestering agents (e.g., colestilan, cholestyramine, colestimide, colesevelam hydrochloride, colestipol, and dialkylaminoalkyl derivatives of a cross-linked dextran), (iii) inhibitors of cholesterol absorption, (e.g., ezetimibe), and (iv) acyl CoA:cholesterol acyltransferase inhibitors (e.g., avasimibe);
   (7) HDL-raising drugs; and nicotinic acid receptor agonists;
   (8) antiobesity compounds;
   (9) agents intended for use in inflammatory conditions, such as aspirin, non-steroidal anti-inflammatory drugs (NSAIDs), glucocorticoids, and selective cyclooxygenase-2 (COX-2) inhibitors;
   (10) antihypertensive agents, such as ACE inhibitors (e.g., enalapril, lisinopril, ramipril, captopril, quinapril, and tandolapril), A-II receptor blockers (e.g., losartan, candesartan, irbesartan, olmesartan medoxomil, valsartan, telmisartan, and eprosartan), renin inhibitors (e.g., aliskiren), beta blockers (e.g., calcium channel blockers);
   (11) glucokinase activators (GKAs) (e.g., AZD6370);
   (12) inhibitors of 11β-hydroxysteroid dehydrogenase type 1 (e.g., such as those disclosed in U.S. Patent No. 6,730,690; WO 03/104207; and WO 04/058741);
   (13) inhibitors of cholesteryl ester transfer protein (CETP), (e.g., torcetrapib and MK-0859);
   (14) inhibitors of fructose 1,6-bisphosphatase (e.g., such as those disclosed in U.S. Patent Nos. 6,054,587; 6,110,903; 6,284,748; 6,399,782; and 6,489,476);
   (15) inhibitors of acetyl CoA carboxylase-1 or 2 (ACC1 or ACC2);
   (16) AMP-activated Protein Kinase (AMPK) activators;
   (17) agonists of the G-protein-coupled receptors: (i) GPR-109, (ii) GPR-119 (e.g., MBX2982, and PSN821), and (iii) GPR-40 (e.g., TAK875, 5-[4-[[(1R)-4-[6-(3-hydroxy-3-methylbutoxy)-2-methylpyridine-3-yl]-2,3-dihydro-1H-indene-1-yl]oxy]phenyl]isothiazole-3-ol 1-oxide, 5-(4-((3-(2,6-dimethyl-4-(3-(methylsulfonyl)propoxy)phenyl)phenyl)methoxy)-phenyl)iso, 5-(4-((3-(2-methyl-6-(3-hydroxypropoxy)pyridine-3-yl)-2-methylphenyl)methoxy)-phenyl)isothiazole-3-ol 1-oxide, and 5-[4-[[3-[4-(3-aminopropoxy)-2,6-dimethylphenyl]phenyl]-methoxy]phenyl]isothiazole-3-ol 1-oxide);
   (18) SSTR3 antagonists (e.g., such as those disclosed in WO 2009/011836);
   (19) neuromedin U receptor agonists (e.g., such as those disclosed in WO2009/042053, including, but not limited to, neuromedin S (NMS));
   (20) inhibitors of stearoyl-coenzyme A delta-9 desaturase (SCD);
   (21) GPR-105 antagonists (e.g., such as those disclosed in WO 2009/000087);
   (22) inhibitors of glucose uptake, such as sodium-glucose transporter (SGLT) inhibitors and its various isoforms, such as SGLT-1; SGLT-2 (e.g., ASP1941, TS071, BI10773, tofogliflozin, LX4211, canagliflozin, dapagliflozin ertugliflozin, ipragliflozin, and remogliflozin; and SGLT-3);
   (23) inhibitors of acyl coenzyme A:diacylglycerol acyltransferase 1 and 2 (DGAT-1 and DGAT-2);
   (24) inhibitors of fatty acid synthase;
   (25) inhibitors of acyl coenzyme A:monoacylglycerol acyltransferase 1 and 2 (MGAT-1 and MGAT-2);
   (26) agonists of the TGR5 receptor (also known as GPBAR1, BG37, GPCR19, GPR131, and M-BAR);
   (28) bromocriptine mesylate and rapid-release formulations thereof, and
   (29) IL-lb antibodies (e.g., XOMA052, and canakinumab); and
(c) a pharmaceutically acceptable carrier.

When a co-agonist peptide of the present invention is used contemporaneously with one or more other drugs, peptides, or proteins, a pharmaceutical composition containing such other drugs, peptides, or proteins in addition to the co-agonist peptide of the present invention may be provided. Accordingly, the pharmaceutical compositions of the present invention include those that also contain one or more other active ingredients, in addition to a co-agonist peptide of the present invention.

Methods of administrating the pharmacological compositions comprising the one or more co-agonist peptides disclosed herein to an individual include, but are not limited to, intradermal, intramuscular, intraperitoneal, intravenous, subcutaneous, intranasal, epidural, and oral routes. The compositions can be administered by any convenient route, for example by infusion or bolus injection, by absorption through epithelial or mucocutaneous linings (for example, oral mucosa, rectal and intestinal mucosa, and the like), ocular, and the like and can be administered together with other biologically-active agents. Administration can be systemic or local. In addition, it may be advantageous to administer the composition into the central nervous system by any suitable route, including intraventricular and intrathecal injection. Intraventricular injection may be facilitated by an intraventricular catheter attached to a reservoir (for example, an Ommaya reservoir). Pulmonary administration may also be employed by use of an inhaler or nebulizer, and formulation with an aerosolizing agent. It may also be desirable to administer the one or more co-agonist peptides disclosed herein locally to the area in need of treatment; this may be achieved by, for example, and not by way of limitation, local infusion during surgery, topical application, by injection, by means of a catheter, by means of a suppository, or by means of an implant.

Various delivery systems are known and can be used to administer the co-agonist peptides disclosed herein including, but not limited to, encapsulation in liposomes, microparticles, microcapsules; minicells; polymers; capsules; tablets; and the like. In one embodiment, the co-agonist peptides disclosed herein may be delivered in a vesicle, in particular a liposome. In a liposome, the co-agonist peptides disclosed herein are combined, in addition to other pharmaceutically acceptable carriers, with amphipathic agents such as lipids which exist in aggregated form as micelles, insoluble monolayers, liquid crystals, or lamellar layers in aqueous solution. Suitable lipids for liposomal formulation include, without limitation, monoglycerides, diglycerides, sulfatides, lysolecithin, phospholipids, saponin, bile acids, and the like. Preparation of such liposomal formulations is within the level of skill in the art, as disclosed, for example, in U.S. Patent No. 4,837,028 and U.S. Patent No. 4,737,323. In yet another embodiment, the co-agonist peptides disclosed herein can be delivered in a controlled release system including, but not limited to: a delivery pump (*See,* for example, Saudek, et al., New Engl. J. Med. 321: 574 (1989) and a semi-permeable polymeric material (*See,* for example, Howard, et al., J. Neurosurg. 71: 105 (1989)). Additionally, the controlled release system can be placed in proximity of the therapeutic target, thus requiring only a fraction of the systemic dose. *See,* for example, Goodson, In: Medical Applications of Controlled Release, 1984. (CRC Press, Bocca Raton, Fla.).

The amount of the compositions comprising one or more of the co-agonist peptides disclosed herein which will be effective in the treatment of a particular disorder or condition will depend on the nature of the disorder or condition, and may be determined by standard clinical techniques by those of average skill within the art. In addition, *in vitro* assays may optionally be employed to help identify optimal dosage ranges. The precise dose to be employed in the formulation will also depend on the route of administration, and the overall seriousness of the disease or disorder, and should be decided according to the judgment of the practitioner and each patient's circumstances. Ultimately, the attending physician will decide the amount of the composition with which to treat each individual patient. Initially, the attending physician will administer low doses of the composition and observe the patient's response. Larger doses of the composition may be administered until the optimal therapeutic effect is obtained for the patient, and at that point the dosage is not increased further.

In general, the daily dose range lie within the range of from about 0.001 mg to about 100 mg per kg body weight of a mammal, preferably 0.01 mg to about 50 mg per kg, and most preferably 0.1 to 10 mg per kg, in single or divided doses. On the other hand, it may be necessary to use dosages outside these limits in some cases. However, suitable dosage ranges for intravenous administration of the compositions comprising the one or more co-agonist peptides disclosed herein are generally about 5-500 micrograms (µg) of active compound per kilogram (Kg) body weight. Suitable dosage ranges for intranasal administration are generally about 0.01 pg/kg body weight to 1 mg/kg body weight.

Effective doses may be extrapolated from dose-response curves derived from in vitro or animal model test systems. Suppositories generally contain active ingredient in the range of 0.5% to 10% by weight; oral formulations preferably contain 10% to 95% active ingredient. Ultimately the attending physician will decide on the appropriate duration of therapy using compositions comprising the one or more co-agonist peptides disclosed herein of the present invention. Dosage will also vary according to the age, weight and response of the individual patient.

Further provided is a pharmaceutical pack or kit, comprising one or more containers filled with one or more of the ingredients of the pharmaceutical compositions and co-agonist peptides disclosed herein. Optionally associated with such container(s) may be a notice in the form prescribed by a governmental agency regulating the manufacture, use or sale of pharmaceuticals or biological products, which notice reflects approval by the agency of manufacture, use or sale for human administration.

The present invention further provides for the use of any one of the aforementioned peptides for manufacture of a medicament for the treatment of obesity metabolic disease.

The present invention further provides for the use of any one of the aforementioned compositions for manufacture of a medicament for the treatment of obesity metabolic disease.

In particular aspects, the metabolic disease is diabetes, non-alcoholic fatty liver disease (NAFLD), non-alcoholic steatohepatitis (NASH), or obesity.

In particular aspects, the diabetes is Type I diabetes, Type II diabetes, or gestational diabetes.

In particular aspects, the medicament is for treatment of more than one metabolic disease, for example, diabetes and NASH, NAFLD, or obesity; obesity and NASH or NAFLD; diabetes, NASH, and obesity; diabetes, NAFLD, and obesity; or diabetes and obesity.

Further provided is method for treating a metabolic disease in a patient or individual comprising: administering to the patient or individual an effective amount of any one of the aforementioned compositions comprising a co-agonist peptide and administering to the patient or individual an effective amount of a composition comprising an insulin or insulin analog to treat the metabolic disease in the patient or individual.

In particular aspects, the composition comprising the co-agonist peptide is administered at a time prior to the time the composition comprising the insulin or insulin analog is administered. In another aspect, the composition comprising the insulin or insulin analog is administered at a time prior to the time the composition comprising the co-agonist peptide is administered. In a further still aspect, the composition comprising the co-agonist peptide is administered at the same time as the composition comprising the insulin or insulin analog is administered.

In particular aspects, the insulin analog is insulin detemir, insulin glargine, insulin levemir, insulin glulisine, or insulin lispro.

In particular aspects, the metabolic disease is diabetes, non-alcoholic fatty liver disease (NAFLD), non-alcoholic steatohepatitis (NASH), or obesity.

In particular aspects, the diabetes is Type I diabetes, Type II diabetes, or gestational diabetes.

In particular aspects, the patient has more than one metabolic disease, for example, diabetes and NASH, NAFLD, or obesity; obesity and NASH or NAFLD; diabetes, NASH, and obesity; diabetes, NAFLD, and obesity; or diabetes and obesity.

The present invention further provides a composition comprising any one of the aforementioned peptides; an insulin or insulin analog; and, a pharmaceutically acceptable carrier.

The present invention further provides for the use of a composition comprising any one of the aforementioned peptides; an insulin or insulin analog; and, a pharmaceutically acceptable carrier for the treatment of a metabolic disease.

In particular aspects, the metabolic disease is diabetes, non-alcoholic fatty liver disease (NAFLD), non-alcoholic steatohepatitis (NASH), or obesity. In further aspects, the diabetes is Type I diabetes, Type II diabetes, or gestational diabetes.

The present invention further provides for the use of a composition comprising any one of the aforementioned peptides; an insulin or insulin analog; and, a pharmaceutically acceptable carrier for the manufacture of a medicament for the treatment of a metabolic disease.

In particular aspects, the metabolic disease is diabetes, non-alcoholic fatty liver disease (NAFLD), non-alcoholic steatohepatitis (NASH), or obesity. In further aspects, the diabetes is Type I diabetes, Type II diabetes, or gestational diabetes.
List of Abbreviations Aq or aq. is aqueous; CH₂Cl₂ is dichloromethane; DCM is dichloromethane; Dde is 1-(4,4-dimethyl-2,6-dioxocyclohex-1-ylidene)ethyl; DIC is N,N'-diisopropylcarbodiimide; DIEA is diisopropylethylamine; DMF is *N,N*-dimethylformamide; DMSO is dimethylsulfoxide; Fmoc is Fluorenylmethyloxycarbonyl; Fmoc -Cl is Fluorenylmethyloxycarbonyl chloride; g is gram(s); h or hr or hrs is hour(s); Grubbs Catalyst® 2nd Generation is benzylidene[1,3-bis(2,4,6-trimethylphenyl)-2-imidazolidinylidene]dichloro(tricyclohexylphosphine)ruthenium; HATU is O-(7-azabenzotriazol-1-yl)-N,N,N',N'-tetramethyluronium hexafluorophosphate; HOAt is hydroxybenzoazatriazole; HOBt is hydroxybenzotriazole; L is liter; LC-MS is liquid chromatography-mass spectroscopy; mL is milliliter; min or mins is minute(s); Mol is mole(s); mmol is mmole(s); mg is milligram(s); MS is mass spectroscopy; N is normal; NMP is 1-methyl-2-pyrrolidinone; PG is protecting group; rt or r.t. or RT is room temperature; *t*-Bu is is *tert*-butyl; *t*-BuOH is *tert*-butyl alcohol; TFA is trifluoroacetic acid; and UPLC is ultra performance liquid chromatography.

### Methods of Synthesis of the Compounds of the Present Invention:

The following reaction schemes and Examples illustrate methods which may be employed for the synthesis of the peptides of the present invention, using appropriate materials. These reaction schemes and Examples are provided for the purpose of illustration only and are not to be construed as limitations on the disclosed invention. Those skilled in the art will readily understand that known variations of protecting groups, as well as of the conditions and processes of the following preparative procedures, can be used to prepare these peptides. Starting materials are either commercially available or made by known procedures in the literature or as illustrated. All temperatures are degrees Celsius unless otherwise noted.

### EXAMPLE 1

### GENERAL SYNTHETIC PROCEDURE FOR PEPTIDES OF SEQ ID Nos. 6-19 (Table 1)

The peptides in Table 1 were synthesized by solid phase Fmoc/tBu strategy on a Rink-amide PEG-PS resin, Champion (Biosearch Technologies (150 µmοl, loading 0.28 mmol/g) on a Symphony Protein Technologies Inc. synthesizer.

All of the amino acids were dissolved at a 0.3 M concentration in a solution of 0.3M HOBt (Hydroxybenzotriazole) in DMF. The amino acids were activated with equimolar amounts of HATU (O-(7-azabenzotriazol-1-yl)-N,N,N',N'-tetramethyluronium hexafluorophosphate) and a 2-fold molar excess of DIEA (diisopropylethylamine) solution 2M in NMP. The acylation reactions were performed for 1 hour with 5-fold excess of activated amino acid over the resin free amino groups.

Double acylation reactions of 45 minutes were performed from His¹ to Thr⁷, from Asp¹⁵ to Aib¹⁶ (aminoisobutyric acid) and from Phe²² to Val²³. The N-terminal residue was incorporated as Boc-His(Trt)-OH. For seq ID 7, 8, 10, 12, 13, 14, 15, 16, the Lys at position 10 was protected by the orthogonal Dde [1-(4,4-dimethyl-2,6-dioxocyclohex-1-ylidene)ethyl] protecting group.

Sequences ID 6-19 were assembled as peptide precursors on the resin having two olefin-bearing amino acids from the following list: S₅ₘₑ, S₇ₘₑ, S_{5H}, and R₆ₘₑ.

The olefin bearing amino acids, such as R6me, S7me, S5me and S5H are combined at suitable positions within the sequence, either at i, i+4 (for S5me with S5me or S5H and S5H) or at i, i+7 (R6me & S7me) to form an all hydrocarbon linkage by applying the chemical strategy of ring closing metathesis. One R₆ₘₑ amino acid and one S₇ₘₑ amino acid cyclize to form an all hydrocarbon ring, with formation of the linkage (E or Z)-tridec-6-ene between their alpha carbons as shown for structures of SEQ ID Nos. 6, 7, 8, 9, 10, 11, and 13. Two S₅ₘₑ amino acids cyclize to form an all hydrocarbon ring, with formation of the linkage (E or Z)-dec-5-ene between their alpha carbons as shown for structures of SEQ ID Nos. 12 , 14, and 19. Two S_{5H} amino acids cyclize to form an all hydrocarbon ring, with formation of the linkage (E or Z)-dec-5-ene between their alpha carbons as shown for structures of SEQ ID Nos. 15, 16, 17, and 18.

All of the olefin amino acids were incorporated by manual coupling, activated with HOAt (Hydroxybenzoazatriazole) and DIC (N,N'-diisopropylcarbodiimide). For sequences ID 6-19, the ring closing metathesis between the olefin side chains was performed by incubation of the corresponding peptide resins at the end of the assembly with 20 mol of Grubbs Catalyst® 2nd Generation in dichloroethane at reflux for 1 hour, for two cycles. After the metathesis, the Dde protecting group of Lys¹⁰ was removed by treatment with a solution of 2% hydrazine in DMF and for peptides with sequence ID 7, 8, 10, 12, 13, 14, 15, 16, side chain derivatization was accomplished by manual coupling of Fmoc-Glu-OtBu residues and palmitic acid activated with DIC and HOAt.

The dry peptide-resins were treated for 2 hours at room temperature with 88% TFA, 5% phenol, 2% triisopropylsilane and 5% water to afford protecting groups deprotection and cleavage from resin. The solution was filtered to remove the resin and the crude peptide solution was precipitated in cold methyl *tert*-butyl ether. The peptide pellet was resuspended, washed and centrifuged in cold methyl *tert*-butyl ether for 2 times. The peptide pellet was dried under vacuum and then resuspended in H₂O, 20% acetonitrile, and lyophilized.

The crude peptides with sequence ID: 6-19 were purified by reverse-phase HPLC using as eluents (A) 0.1% TFA in water and (B) 0.1% TFA in acetonitrile.

All the final peptides were characterized on an Acquity UPLC Waters Chromatograph, with BEH300 C4 Acquity Waters 2.1 x 100 mm, 1.7µm, at 45°C, using H₂O, 0.1% TFA (A) and CH₃CN, 0.1% TFA (B) as solvents and by electrospray mass spectrometry on a Acquity SQ Detector.

### EXAMPLE 2

### PEPTIDE SEQ ID NO. 16 (TP551)

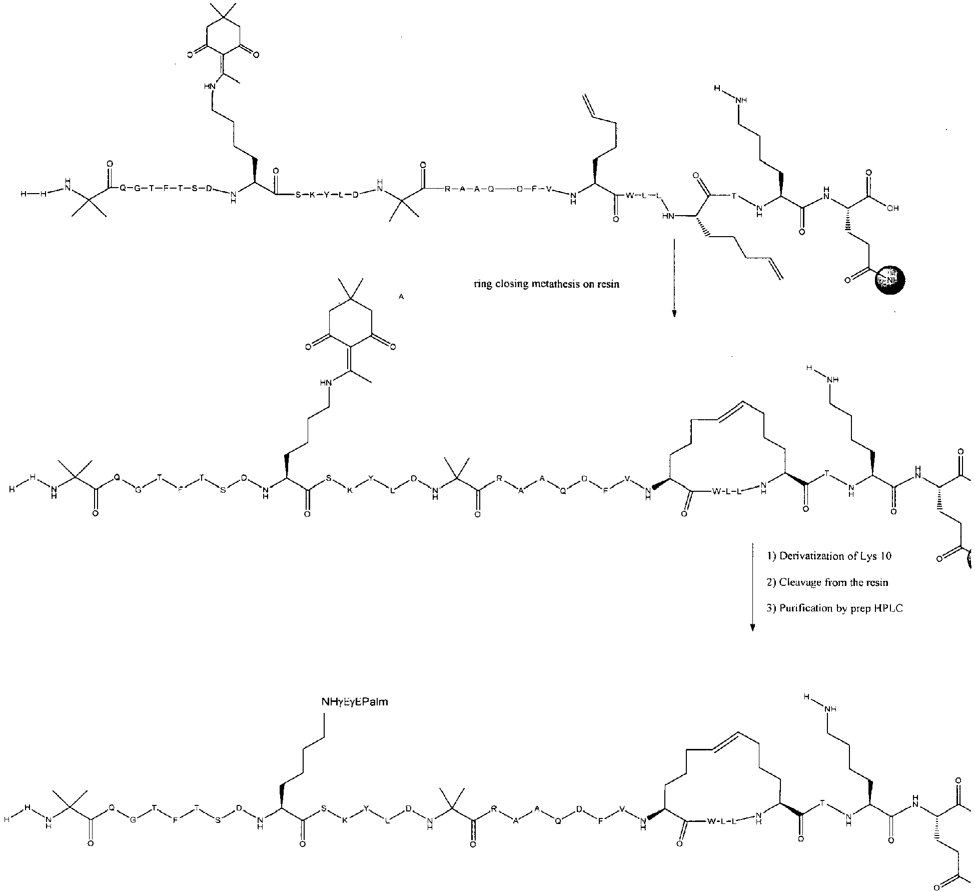

The peptide of SEQ ID NO. 16
HUQGTFTSDK(γEγEC16) SKYLDURAAQDFV*S_{5H}WLL*S_{5H}TKγE-NH₂
was synthesized according to the method of Example 1 starting with the appropriate starting materials and reagents.

Double acylation reactions of 45 minutes were performed from His¹ to Thr⁷, from Asp¹⁵ to Aib¹⁶ (aminoisobutyric acid) and from Phe²² to Val²³. The N-terminal residue was incorporated as Boc-His(Trt)-OH. Lysine at position 10 was protected by the orthogonal Dde [1-(4,4-dimethyl-2,6-dioxocyclohex-1-ylidene)ethyl] protecting group.

Sequences ID 16, was assembled as peptide precursors on the resin having two olefin-bearing amino acids as S_{5H}.

The two olefin bearing amino acids, S5H were combined at positions i, i+4, namely 24 and 28 within the sequence, to form an all hydrocarbon linkage by applying the chemical strategy of ring closing metathesis. Two S_{5H} amino acids cyclize to form an all hydrocarbon ring, with formation of the linkage (E or Z)-dec-5-ene between their alpha carbons as shown for structures of SEQ ID Nos. 16.

The olefin amino acids were incorporated by manual coupling, activated with HOAt (Hydroxybenzoazatriazole) and DIC (N,N'-diisopropylcarbodiimide). The ring closing metathesis between the olefin side chains was performed by incubation of the corresponding peptide resin at the end of the assembly with 20 mol of Grubbs Catalyst® 2nd Generation in dichloroethane at reflux for 1 hour, for two cycles. After the metathesis, the Dde protecting group of Lys¹⁰ was removed by treatment with a solution of 2% hydrazine in DMF and side chain derivatization was accomplished by manual coupling of Fmoc-Glu-OtBu residues and palmitic acid activated with DIC and HOAt.

The dry peptide-resin was treated for 2 hours at room temperature with 88% TFA, 5% phenol, 2% triisopropylsilane and 5% water to afford protecting groups deprotection and cleavage from resin. The solution was filtered to remove the resin and the crude peptide solution was precipitated in cold methyl *tert-*butyl ether. The peptide pellet was resuspended, washed and centrifuged in cold methyl *tert*-butyl ether for 2 times. The peptide pellet was dried under vacuum and then resuspended in H₂O, 20% acetonitrile, and lyophilized.

The crude peptide (200 mg in 3 ml of DMSO) was purified by reverse-phase HPLC using preparative Waters Deltapak C4 (40 x 200 mm, 15 µm, 300Ǻ) and using as eluents (A) 0.1% TFA in water and (B) 0.1% TFA in acetonitrile The following gradient of eluent B was used: 35%B to 30%B over 5 min, 30%B to 50%B over 20min-80%B, flow rate 80 mL/min, wavelength 214 nm.

The final peptide was characterized on an Acquity UPLC Waters Chromatograph, with BEH300 C4 Acquity Waters 2.1 x 100 mm, 1.7µm, at 45°C, using H₂O, 0.1% TFA (A) and CH₃CN, 0.1% TFA (B) as solvents and by electrospray mass spectrometry on a Acquity SQ Detector. The peptide were characterized by electrospray mass spectrometry on a Acquity SQ Detector (MW found: 4073.71 Da; MW expected: 4074.2).

### EXAMPLE 3

Activity of the peptides at the Glucagon receptor (GCGR) and GLP-1 receptor (GLP1R) was measured in a cAMP activity assay.

Peptides were dissolved in 100% DMSO and serially diluted to generate 10 point titrations. The peptide solutions were then transferred into 384-well assay plates (150 nL/well). Assay ready frozen cells expressing human GLP1R or human GCGR were suspended in growth media consisting of DMEM medium (GIBCO), 10% FBS (GIBCO), 1x NEAA(GIBCO), 1x P/S (GIBCO), 10ug/ml Blasticidin (GIBCO) and 200 µg/mL Hygromycin (GIBCO). Cells were then diluted in assay buffer consisting of PBS (GIBCO), 7.5% BSA (Perkin Elmer), 100 µM RO 20-1724 (Sigma), with or without 20% human serum (MP Biomedical). The cell suspensions (15 µL) were then added to the assay plates containing the peptide solutions (30,000 cells/well for human GCGR; 10,000 cells/well for human GLP1R). The cells were incubated for 1 hour at room temperature in the dark. Production of cAMP was determined using HitHunter™ cAMPXS kits (DiscoverX) following manufacturer protocol. The plates were incubated for overnight at room temperature in the dark. Luminescence was measured using an EnVision Multilabel plate reader (Perkin Elmer). Native GLP-1 and Glucagon (Bachem) are used as control peptides. EC₅₀ values were calculated using uses a 4 parameter logistic fit based on the Levenberg-Marquardt algorithm.

The peptides of the present invention have EC₅₀ values at each of the glucagon and GLP-1 receptors that is less than 5 nM. The peptides in SEQ ID Nos. 6-19 have the specific glucagon receptor EC₅₀ values, and GLP-1 receptor EC₅₀ values shown in Table 2.

| Table 2 | | | | |
|---|---|---|---|---|
| SEQ ID NO: | Peptide | GCGR EC50 human | GLP1 R EC50 human | hGCG R/hGL P1R (hTone) |
| 6 | HsQGTFTSDKS^{∗}R₆ₘₑYLDERA^{∗}*S₇ₘₑQDFVQWLLDT-NH₂ | 20 | 5.3 | NA |
| 7 | HsQGTFTSDK(γEγEC16)S^{∗}R₆ₘₑYLDERA^{∗}S₇ₘₑQDFVQWLLDT-NH₂ | 5 | 0.77 | NA |
| 8 | HsQGTFTSDK(γEγEC16)SKYLDE^{∗}R₆ₘₑAAQDFV^{∗}S₇ₘₑWLLDT-NH₂ | 5 | 2.28 | NA |
| 9 | HsQGTFTSDKSKYLDE^{∗}R₆ₘₑAAQDFV^{∗}S₇ₘₑWLLDT-NH₂ | 5 | 5 | NA |
| 10 | HsQGTFTSDK(γEγEC16)SKYLDERAA^{∗}R₆ₘₑDFVQWL^{∗}S₇ₘₑDT-NH₂ | 8.64 | 0.45 | 19.02 |
| 11 | HsQGTFTSDKSKYLDERAA^{∗}R₆ₘₑDFVQWL^{∗}S₇ₘₑDT-NH₂ | 5 | 2.74 | 1.82 |
| 12 | HsQGTFTSDKL(γEγEC16)SKYLDERAA^{∗}S₅ₘₑDFV^{∗}S₅ₘₑWLLDT-NH₂ | 1.2 | 1.05 | 1.14 |
| 13 | HUQGTFTSDK(γEγEC16)SKYLDURAAQ*R6meFVQWLM*S7meTKγE -NH2 | 5 | 0.26 | 19.11 |
| 14 | HUQGTFTSDK(γEγEC16)SKYLDURAAQDFV*S5meWLM*S5meTKγE -NH2 | 4.56 | 0.13 | 35.14 |
| 15 | HsQGTFTSDK(γEγEC16)SKYLDERAA^{∗}S_{5H}DFV^{∗}S_{5H}WLLDT-NH₂ | 0.67 | 1.5 | 0.44 |
| 16 | HUQGTFTSDK(γEγEC16)SKYLDURAAQDFV*S5HWLL*S5HTKγE- NH2 | 0.48 | 0.18 | 2.67 |
| 17 | HsQGTFTSDKSKYLDERAA^{∗}S_{5H}DFV^{∗}S_{5H}WLLDT-NH₂ | 3.38 | 2.38 | 1.42 |
| 18 | HUQGTFTSDKSKYLDURAAQDFV^{∗}S_{5H}WLL^{∗}S_{5H}TKγE-NH₂ | 4.25 | 0.13 | 33.1 |
| 19 | HsQGTFTSDKSKYLDERAA^{∗}S₅ₘₑDFV^{∗}S₅ₘₑWLLDT-NH₂ | 2.15 | 2.79 | 0.77 |
| Table legend: U= alpha-aminoisobutyric acid; γE= γ-glutamic acid; γE γE = γ-glutamic acid- γ-glutamic acid; s=D-serine; C16 = -CO-(CH₂)₁₄-CH₃; *X = amino acids cyclized to form a double bond containing ring; S5me = (S)-2-amino-2-methylhept-6-enoic acid; S7me = (S)-2-amino-2-methylnon-8-enoic acid; S5H = (S)-2-aminohept-6-enoic acid; R6me = (R)-2-amino-2-methyloct-7-enoic acid; and NH₂ = C-terminal amide | | | | |

### EXAMPLE 4

### Thioflavin T Assay

The physical stability of the stapled peptides may be tested in a Thioflavin T Assay (Schlein, Morten; The AAPS Journal, Vol. 19, No. 2, March 2017)) to determine the amount of time before fibril formation commences.

## Claims

1. A peptide comprising the amino acid sequence of native human glucagon
HSQGTFTSDYSKYLDSRRAQDFVQWLMNT (SEQ ID NO:1)
wherein
1) L-Serine at X² is replaced with α-aminoisobutyric acid, or D-Serine;
2) Tyrosine at X¹⁰ is replaced with is Lysine, Lysine conjugated to a fatty acid, or Lysine conjugated to a fatty diacid;
3) L-Serine at X¹⁶ is replaced with α-aminoisobutyric acid, or Glutamic acid; and
4) X³⁰ is absent, or Lysine conjugated by a gamma-glutamic acid spacer; and up to eight additional amino acid substitutions selected from:
1) Lysine at X¹² is optionally replaced with (R)-2-amino-2-methyloct-7-enoic acid;
2) Arginine at X¹⁷ is optionally replaced with (R)-2-amino-2-methyloct-7-enoic acid;
3) Alanine at X¹⁹ is optionally replaced with (S)-2-amino-2-methylnon-8-enoic acid;
4) Glutamine at X²⁰ is optionally replaced with (R)-2-amino-2-methyloct-7-enoic acid, (S)-2-amino-2-methylhept-6-enoic acid, or (S)-2-aminohept-6-enoic acid;
5) Aspartic acid at X²¹ is optionally replaced with (R)-2-amino-2-methyloct-7-enoic acid;
6) Glutamine at X²⁴ is optionally replaced with (S)-2-amino-2-methylnon-8-enoic acid, (S)-2-amino-2-methylhept-6-enoic acid, or (S)-2-aminohept-6-enoic acid;
7) Methionine at X²⁷ is optionally replaced with Leucine, or (S)-2-amino-2-methylnon-8-enoic acid; and
8) Asparagine at X²⁸ is optionally replaced with Aspartic acid, (S)-2-amino-2-methylnon-8-enoic acid, (S)-2-amino-2-methylhept-6-enoic acid, or (S)-2-aminohept-6-enoic acid;
provided that the peptide contains at least two amino acids selected from: (R)-2-amino-2-methyloct-7-enoic acid, (S)-2-amino-2-methylnon-8-enoic acid, (S)-2-amino-2-methylhept-6-enoic acid, and (S)-2-aminohept-6-enoic acid, which cyclize to form a double bond containing ring;
or a pharmaceutically acceptable salt thereof.

2. The peptide of Claim 1 wherein the two amino acids acids which cyclize to form
a double bond containing ring are selected from:
1) one (R)-2-amino-2-methyloct-7-enoic acid and one (S)-2-amino-2-methylnon-8-enoic acid;
2) two (S)-2-amino-2-methylhept-6-enoic acids; and
3) two (S)-2-aminohept-6-enoic acids;
or a pharmaceutically acceptable salt thereof.

3. The peptide of Claim 1 comprising the formula
HX²QGTFTSDX¹⁰SX¹²YLDX¹⁶X¹⁷AX¹⁹X²⁰X²¹FVX²⁴WLX²⁷X²⁸TX³⁰-NH₂ (SEQ ID NO:20)
wherein
X² is α-aminoisobutyric acid, or D-Serine;
X¹⁰ is Lysine, Lysine conjugated to a fatty acid, or Lysine conjugated to a fatty diacid;
X¹² is (R)-2-amino-2-methyloct-7-enoic acid, or Lysine;
X¹⁶ is α-aminoisobutyric acid (Aib), or Glutamic acid;
X¹⁷ is (R)-2-amino-2-methyloct-7-enoic acid, or Arginine;
X¹⁹ is (S)-2-amino-2-methylnon-8-enoic acid, or Alanine;
X²⁰ is Glutamine, (R)-2-amino-2-methyloct-7-enoic acid, (S)-2-amino-2-methylhept-6-enoic acid, or (S)-2-aminohept-6-enoic acid;
X²¹ is Aspartic acid or (R)-2-amino-2-methyloct-7-enoic acid;
X²⁴ is Glutamine, (S)-2-amino-2-methylnon-8-enoic acid, (S)-2-amino-2-methylhept-6-enoic acid, or (S)-2-aminohept-6-enoic acid;
X²⁷ is Leucine, (S)-2-amino-2-methylnon-8-enoic acid, or Methionine;
X²⁸ is Aspartic acid, (S)-2-amino-2-methylnon-8-enoic acid, (S)-2-amino-2-methylhept-6-enoic acid, or (S)-2-aminohept-6-enoic acid;
X³⁰ is absent, or Lysine conjugated by a gamma-glutamic acid spacer;
provided that the peptide contains at least two amino acids selected from (R)-2-amino-2-methyloct-7-enoic acid, (S)-2-amino-2-methylnon-8-enoic acid, (S)-2-amino-2-methylhept-6-enoic acid, and (S)-2-aminohept-6-enoic acid, which cyclize to form a double bond containing ring; and
wherein the peptide optionally includes a protecting group that, if present, is joined to the C-terminal carboxy group of the peptide;
or a pharmaceutically acceptable salt thereof.

4. The peptide of Claim 3, wherein the two amino acids acids which cyclize to form
a double bond containing ring are selected from:
1) one (R)-2-amino-2-methyloct-7-enoic acid and one (S)-2-amino-2-methylnon-8-enoic acid;
2) two (S)-2-amino-2-methylhept-6-enoic acids; and
3) two (S)-2-aminohept-6-enoic acids;
or a pharmaceutically acceptable salt thereof.

5. The peptide of Claim 3, wherein the fatty diacid comprises a C14, C15, C16, C17, C18, C19, or C20 fatty diacid, and the fatty acid at position 10 comprises a C14, C16, C17, C18, C19, or C20 fatty acid; or a pharmaceutically acceptable salt thereof.

6. The peptide of Claim 3, wherein X¹⁰ is Lysine, or Lysine conjugated to a fatty acid; or a pharmaceutically acceptable salt thereof.

7. The peptide of Claim 3, wherein X¹⁰ is Lysine, or Lysine conjugated to a fatty acid via a gamma-glutamic acid - gamma-glutamic acid linker; or a pharmaceutically acceptable salt thereof.

8. The peptide of Claim 3, wherein X¹⁰ is Lysine or Lysine conjugated to a C16 fatty acid via a gamma-glutamic acid - gamma-glutamic acid linker; or a pharmaceutically acceptable salt thereof.

9. The peptide of Claim 1 comprising the formula
HX²QGTFTSDX¹⁰SX¹²YLDX¹⁶X¹⁷AX¹⁹X²⁰X²¹FVX²⁴WLX²⁷X²⁸TX³⁰-NH₂ (SEQ ID NO:20)
wherein
X² is α-aminoisobutyric acid, or D-Serine;
X¹⁹ is Lysine, or Lysine conjugated to a fatty acid;
X¹² is (R)-2-amino-2-methyloct-7-enoic acid, or Lysine;
X¹⁶ is α-aminoisobutyric acid, or Glutamic acid;
X¹⁷ is (R)-2-amino-2-methyloct-7-enoic acid, or Arginine;
X¹⁹ is (S)-2-amino-2-methylnon-8-enoic acid, or Alanine;
X²⁰ is Glutamine, (R)-2-amino-2-methyloct-7-enoic acid, (S)-2-amino-2-methylhept-6-enoic acid, or (S)-2-aminohept-6-enoic acid;
X²¹ is Aspartic acid or (R)-2-amino-2-methyloct-7-enoic acid;
X²⁴ is Glutamine, (S)-2-amino-2-methylnon-8-enoic acid, (S)-2-amino-2-methylhept-6-enoic acid, or (S)-2-aminohept-6-enoic acid;
X²⁷ is Leucine, (S)-2-amino-2-methylnon-8-enoic acid, or Methionine;
X²⁸ is Aspartic acid, (S)-2-amino-2-methylnon-8-enoic acid, (S)-2-amino-2-methylhept-6-enoic acid, or (S)-2-aminohept-6-enoic acid ;
X³⁰ is absent, or Lysine conjugated by a gamma-glutamic acid spacer;
provided that the peptide contains at least two amino acids selected from (R)-2-amino-2-methyloct-7-enoic acid, (S)-2-amino-2-methylnon-8-enoic acid, (S)-2-amino-2-methylhept-6-enoic acid, and (S)-2-aminohept-6-enoic acid, which cyclize to form a double bond containing ring; and
wherein the peptide optionally includes a protecting group that, if present, is joined to the C-terminal carboxy group of the peptide;
or a pharmaceutically acceptable salt thereof.

10. The peptide of Claim 9 wherein the two amino acids acids which cyclize to form
a double bond containing ring are selected from:
1) one (R)-2-amino-2-methyloct-7-enoic acid and one (S)-2-amino-2-methylnon-8-enoic acid;
2) two (S)-2-amino-2-methylhept-6-enoic acids; and
3) two (S)-2-aminohept-6-enoic acids;
or a pharmaceutically acceptable salt thereof.

11. The peptide of Claim 9, wherein the fatty acid at position 10 comprises a C14, C16, C17, C18, C19, or C20 fatty acid; or a pharmaceutically acceptable salt thereof.

12. The peptide of Claim 9, wherein X¹⁰ is Lysine, or Lysine conjugated to a fatty acid via a gamma-glutamic acid - gamma-glutamic acid linker; or a pharmaceutically acceptable salt thereof.

13. The peptide of Claim 9, wherein X¹⁰ is Lysine or Lysine conjugated to a C16 fatty acid via a gamma-glutamic acid - gamma-glutamic acid linker; or a pharmaceutically acceptable salt thereof.

14. The peptide of Claim 1 comprising the formula
HX²QGTFTSDX¹⁰SX¹²YLDX¹⁶x¹⁷AX¹⁹X²⁰X²¹FVX²⁴WLX²⁷X²⁸TX³⁰-NH₂ (SEQ ID NO:20)
wherein
X² is α-aminoisobutyric acid, or D-Serine;
X¹⁰ is Lysine, or Lysine conjugated to a fatty acid via a gamma-glutamic acid - gamma-glutamic acid linker;
X¹² is (R)-2-amino-2-methyloct-7-enoic acid, or Lysine;
X¹⁶ is α-aminoisobutyric acid (Aib), or Glutamic acid;
X¹⁷ is (R)-2-amino-2-methyloct-7-enoic acid, or Arginine;
X¹⁹ is (S)-2-amino-2-methylnon-8-enoic acid, or Alanine;
X²⁰ is Glutamine, (R)-2-amino-2-methyloct-7-enoic acid, (S)-2-amino-2-methylhept-6-enoic acid, or (S)-2-aminohept-6-enoic acid;
X²¹ is Aspartic acid or (R)-2-amino-2-methyloct-7-enoic acid;
X²⁴ is Glutamine, (S)-2-amino-2-methylnon-8-enoic acid, (S)-2-amino-2-methylhept-6-enoic acid, or (S)-2-aminohept-6-enoic acid;
X²⁷ is Leucine, (S)-2-amino-2-methylnon-8-enoic acid, or Methionine;
X²⁸ is Aspartic acid, (S)-2-amino-2-methylnon-8-enoic acid, (S)-2-amino-2-methylhept-6-enoic acid, or (S)-2-aminohept-6-enoic acid;
X³⁰ is absent, or Lysine conjugated by a gamma-glutamic acid spacer;
provided that the peptide contains at least two amino acids selected from (R)-2-amino-2-methyloct-7-enoic acid, (S)-2-amino-2-methylnon-8-enoic acid, (S)-2-amino-2-methylhept-6-enoic acid, and (S)-2-aminohept-6-enoic acid, which cyclize to form a double bond containing ring; and
wherein the peptide optionally includes a protecting group that, if present, is joined to the C-terminal carboxy group of the peptide;
or a pharmaceutically acceptable salt thereof.

15. The peptide of Claim 14 wherein the two amino acids acids which cyclize to
form a double bond containing ring are selected from:
1) one (R)-2-amino-2-methyloct-7-enoic acid and one (S)-2-amino-2-methylnon-8-enoic acid;
2) two (S)-2-amino-2-methylhept-6-enoic acids; and
3) two (S)-2-aminohept-6-enoic acids;
or a pharmaceutically acceptable salt thereof.

16. The peptide of Claim 14, wherein the fatty acid at position 10 comprises a C14, C16, C17, C18, C19, or C20 fatty acid; or a pharmaceutically acceptable salt thereof.

17. The peptide of Claim 14, wherein X¹⁰ is Lysine or Lysine conjugated to a C16 fatty acid via a gamma-glutamic acid - gamma-glutamic acid linker; or a pharmaceutically acceptable salt thereof.

18. The peptide of Claim 1, wherein the peptide has the amino acid sequence of SEQ ID NO: 6, 7, 8, 9, 10, 11, 12, 13, 14, 15, 16, 17, 18, or 19; or a pharmaceutically acceptable salt thereof.

19. A composition comprising a peptide of Claim 1, or a pharmaceutically acceptable salt thereof, and a pharmaceutically acceptable carrier.

20. A method for treating a patient for a metabolic disease or disorder comprising administering the patient an effective amount of any one or more of the peptides of Claim 1, or a pharmaceutically acceptable salt thereof, to treat the metabolic disease or disorder in the patient.

21. The method of Claim 20, wherein the metabolic disease or disorder comprises diabetes, non-alcoholic fatty liver disease (NAFLD), non-alcoholic steatohepatitis (NASH), or obesity.

22. The method of Claim 20, wherein the diabetes comprises Type I diabetes, Type II diabetes, or gestational diabetes.

23. A method for treating a patient for a metabolic disease or disorder comprising administering the patient an effective amount of the composition of Claim 19 to treat the metabolic disease or disorder in the patient.

24. The method of Claim 23, wherein the metabolic disease or disorder comprises diabetes, non-alcoholic fatty liver disease (NAFLD), non-alcoholic steatohepatitis (NASH), or obesity.

25. Use of a peptide of Claim 1, or a pharmaceutically acceptable salt thereof, for the manufacture of a medicament for the treatment of a metabolic disease or disorder.

26. The use of Claim 25, wherein the metabolic disease or disorder comprises diabetes, non-alcoholic fatty liver disease (NAFLD), non-alcoholic steatohepatitis (NASH), or obesity.

27. The use of Claim 26, wherein the diabetes comprises Type I diabetes, Type II diabetes, or gestational diabetes.

28. A method for treating a metabolic disease or disorder in a patient or individual comprising: administering to the patient or individual an effective amount of a peptide of Claim 1, or a pharmaceutically acceptable salt thereof, and administering to the patient or individual an effective amount of a composition comprising an insulin or insulin analog to treat the metabolic disease or disorder in the patient or individual.

29. The method of Claim 28, wherein the insulin analog comprises insulin detemir, insulin glargine, insulin levemir, insulin glulisine, insulin degludec, or insulin lispro.

30. The method of Claim 28, wherein the metabolic disease or disorder comprises diabetes, non-alcoholic fatty liver disease (NAFLD), non-alcoholic steatohepatitis (NASH), or obesity.

31. The method of Claim 30, wherein the diabetes comprises Type I diabetes, Type II diabetes, or gestational diabetes.
